# EUROPÄISCHE PATENTSCHRIFT

(11) **EP 0 663 395 B1**
(45) Veröffentlichungstag und Bekanntmachung des Hinweises auf die Patenterteilung: **16.05.2001**
(21) Anmeldenummer: 94119149.6
(22) Anmeldetag: 05.12.1994
(51) Int. Cl.: C07D 231/20, C07D 405/04, C07D 405/12, A61K 31/415

(54) **3-(Phenylalkylaminoalkyloxy)-5-phenylpyrazol-Derivate, Verfahren und Zwischenprodukte zu ihrer Herstellung und ihre Verwendung als herzfrequenzsenkendes Mittel**
3-(phenylalkylaminoalkyloxy)-5-phenylpyrazole derivatives, process and intermediates for their preparation and their use as cardiac rhythm reduction agent
Dérivés de 3-(phénylalkylaminoalkyloxy)-5-phénylpyrazole, procédé et intermédiaires pour leur préparation et leur utilisation comme agent de réduction du rythme cardiaque

(30) Priorität: 08.12.1993 DE 4341749
(43) Veröffentlichungstag der Anmeldung: 19.07.1995
(73) Patentinhaber: Solvay Pharmaceuticals GmbH, 30173 Hannover (DE)
(72) Erfinder: Kehrbach, Wolfgang, Dr., D-30173 Hannover (DE); Mlinaric, Michael, Dr., D-30163 Hannover (DE); Ziegler, Dieter, Dr., D-30996 Hemmingen (DE); Brückner, Reinhard, Dr., D-30559 Hannover (DE); Bielenberg, Willi, Dr., D-31061 Alfeld/Leine (DE)
(74) Vertreter: Lauer, Dieter, Dr.

(56) Entgegenhaltungen:
- EP-A- 0 007 019
- EP-A- 0 037 344
- EP-A- 0 076 756
- EP-A- 0 170 861
- EP-A- 0 491 263
- US-A- 3 920 691

## Beschreibung

Die vorliegende Erfindung betrifft neue Phenylalkylaminoalkyl-Verbindungen, welche am Alkylrest einen 5-Phenylpyrazolyl-3-oxy-Substituenten tragen, und deren Salze sowie diese Verbindungen enthaltende pharmazeutische Zubereitungen und Verfahren und Zwischenprodukte zur Herstellung dieser Verbindungen.

Aus der europäischen Patentanmeldung Nr. 70 19 sind 3-Hydroxycarbonylmethoxy-5-phenylpyrazol-Verbindungen und deren Ester und Amide mit blutlipidsenkenden Eigenschaften bekannt. Aus der europäischen Patentanmeldung Nr. 170 861 sind 3-(Aminoalkylaminocarbonylmethoxy)-5-phenylpyrazol-Verbindungen mit antiarrhythmischen Wirkungen, insbesondere die Reizschwelle zur Auslösung von Rhythmusstörungen am Herzen erhöhenden Wirkungen, bekannt.

In der europäischen Patentanmeldung Nr. 37 344 werden 1-Amino-3-alkylaminoalkoxy-5-phenylpyrazol-Verbindungen beschrieben, welche neben antiarrhythmischen Eigenschaften und gewissen Wirkungen auf das ZNS auch herzfrequenzsenkende Eigenschaften aufweisen. Aus der europäischen Patentanmeldung Nr. 76 756 sind 3-Alkylaminoalkoxy-5-phenylpyrazol-Verbindungen mit vorwiegend Klasse-I-antiarrhythmischen Wirkungen bekannt. In der europäischen Patentanmeldung Nr. 491 263 werden phenylalkylaminoalkylsubstituierte Terpenverbindungen mit u. a. ulkushemmenden und die gastrointestinale Schleimhaut schützenden Wirkungen beschrieben. Schließlich sind aus dem amerikanischen Patent US 3,920,691 bereits 3-(3-Amino-2-hydroxypropoxy )-1,5-disubstituierte Pyrazole mit β-adrenerge Rezeptoren blockierender Wirkung bekannt.

Der vorliegenden Erfindung liegt die Aufgabe zugrunde, neue 5-Phenylpyrazol-Verbindungen mit wertvollen pharmakologischen Eigenschaften zu entwickeln. Ferner liegt der Erfindung die Aufgabe zugrunde, neue, insbesondere zur Herzfrequenzsenkung einsetzbare pharmazeutische Wirkstoffe zu entwickeln.

Es wurde nun gefunden, daß die erfindungsgemäßen neuen in 3-Stellung einen Phenylalkylaminoalkyloxy-Rest tragenden 5-Phenylpyrazol-Verbindungen wertvolle herzwirksame pharmakologische Eigenschaften besitzen und ausgeprägte die Herzfrequenz senkende Wirkungen mit einem günstigen Wirkungsprofil aufweisen. Aufgrund ihres Wirkungprofiles eignen sich die erfindungsgemäßen Substanzen als herzfrequenzsenkend wirksame Wirkstoffe zur Behandlung von ischämischen Zuständen.

Die Erfindung betrifft daher neue Verbindungen der allgemeinen Formel I
(siehe Formel I) worin
- n: eine ganze Zahl von 1 bis 5 bedeutet,
- A: für eine Gruppe der allgemeinen Formel a
(siehe Formel a)
worin R¹ Wasserstoff oder eine niedere Alkylgruppe bedeutet und Q eine (CH₂)ₘ-Gruppe, worin m 2 bis 8 bedeutet und welche gegebenenfalls in α-Stellung zu dem Sauerstoffatom durch 1 bis 2 niedere Alkylgruppen substituiert sein kann, steht oder A für eine Grupppe der allgemeinen Formel b
(siehe Formel b)
worin p 4 bis 6 bedeutet und B eine (CH₂)ᵣ-Gruppe, worin r 1 bis 3 bedeutet, darstellt, steht,
- R²: Wasserstoff, Halogen, niederes Alkyl, niederes Alkoxy, Hydroxy, Trifluormethyl oder Nitro oder, falls R³, R⁶ und R⁷ nicht Hydroxy sind, auch niederes Alkanoyloxy bedeutet und
- R³: Wasserstoff, Halogen, niederes Alkyl, niederes Alkoxy, Hydroxy oder, falls R², R⁶ und R⁷ nicht Hydroxy sind, auch niederes Alkanoyloxy bedeutet oder
- R² und R³: an zwei benachbarte Kohlenstoffatome gebunden sind und gemeinsam eine Alkylendioxygruppe mit 1 bis 2 Kohlenstoffatomen darstellen,
- R⁴: Wasserstoff oder niederes Alkyl bedeutet,
- R⁵: in 1- oder 2-Stellung angeordnet ist und Wasserstoff, niederes Alkyl oder eine Phenylniederalkylgruppe bedeutet,
- R⁶: Wasserstoff, niederes Alkyl, niederes Alkoxy, Hydroxy, Halogen, oder Nitro oder, falls R², R³ und R⁷ nicht Hydroxy sind, auch niederes Alkanoyloxy bedeutet und
- R⁷: Wasserstoff, niederes Alkyl, niederes Alkoxy, Hydroxy oder Halogen oder, falls R², R³ und R⁶ nicht Hydroxy sind, auch niederes Alkanoyloxy bedeutet oder
- R⁶ und R⁷: an zwei benachbarte Kohlenstoffatome gebunden sind und gemeinsam eine Alkylendioxygruppe mit 1 bis 2 Kohlenstoffatomen bilden,
sowie deren Säureadditionssalze.

Sofern in den Verbindungen der Formel I die Substituenten R², R³, R⁶ und R⁷ der Phenylringe niedere Alkylgruppen darstellen oder enthalten, können diese gerade oder verzweigt sein und insbesondere 1 bis 4, vorzugsweise 1 bis 2 Kohlenstoffatome enthalten und stellen insbesondere Methyl, Methoxy oder Acetoxy dar. Sofern die Substituenten Halogen darstellen, kommen insbesondere Fluor, Chlor oder Brom, vorzugsweise Fluor oder Chlor, in Frage.

Sofern die Gruppe A eine Gruppe der Formel a) darstellt, bedeutet R¹ vorzugsweise niederes Alkyl, insbesondere Methyl, oder Wasserstoff, und Q steht für eine vorzugsweise unsubstituierte Alkylenkette (CH₂)ₘ worin m 2 bis 8, vorzugsweise 2 bis 6 insbesondere 2, 3 oder 4 bedeutet. Falls A für eine Gruppe der Formel b) steht, enthält diese vorzugsweise einen 6-gliedrigen Ring, d. h. p ist vorzugsweise 5 und B steht vorzugsweise für eine unsubstituierte Alkylenkette mit 1 bis 3 Kohlenstoffatomen, insbesondere die Methylengruppe.

Die Substituenten R² und R³ stellen vorzugsweise niedere Alkoxygruppen, insbesondere Methoxy, Hydroxy oder Wasserstoff dar. Die Substituenten R⁶ und R⁷ stellen vorzugsweise niederes Alkoxy, insbesondere Methoxy, Hydroxy oder Wasserstoff dar oder R⁶ gegebenenfalls auch Nitro.

R⁴ bedeutet vorzugsweise Wasserstoff. Falls R⁴ für niederes Alkyl steht ist dieses vorzugsweise Methyl. Eine niedere Alkylgruppe oder Phenylniederalkylgruppe R⁵ ist vorzugsweise an dem dem Sauerstoffatom benachbarten Stickstoffatom des Pyrazolrings angeordnet. R⁵ stellt vorzugsweise Wasserstoff oder auch eine Phenylniederalkylgruppe dar. Falls R⁵ für niederes Alkyl steht, bedeutet dieses vorzugsweise Methyl oder Äthyl. Falls R⁵ für eine Phenylniederalkylgruppe steht, kann deren Alkylenkette 1 bis 3 Kohlenstoffe enthalten, und die Gruppe stellt vorzugsweise Benzyl dar.

Erfindungsgemäß werden die neuen Verbindungen der Formel I und deren Säureadditionssalze erhalten, indem man in an sich bekannter Weise
a) zur Herstellung von Verbindungen der allgemeinen Formel Ia
   (siehe Formel Ia)
   worin R⁴, R⁵, A und n obige Bedeutung besitzen und R^{2'}, R^{3'}, R^{6'} und R^{7'} die für R², R³, R⁶ und R⁷ angegebene Bedeutung mit Ausnahme von niederem Alkanoyloxy besitzen, Verbindungen der allgemeinen Formel II
   (siehe Formel II)
   worin R⁴, R⁵, R^{6'} und R^{7'} obige Bedeutung besitzen, wobei jedoch allfällige freie Hydroxygruppen mit einer Hydroxy-Schutzgruppe versehen sind, mit Verbindungen der allgemeinen Formel III
   (siehe Formel III)
   worin R^{2'}, R^{3'}, n und A obige Bedeutung besitzen, wobei jedoch allfällige freie Hydroxygruppen mit einer Hydroxy-Schutzgruppe versehen sind, und X für eine abspaltbare Fluchtgruppe steht, umsetzt und anschließend allfällige Hydroxy-Schutzgruppen wieder abspaltet, oder
b) zur Herstellung von Verbindungen der Formel Ia Verbindungen der Formel II mit Triphenylphosphin in Gegenwart von Azodicarbonsäureestern der allgemeinen Formel XVIII
   (siehe Formel XVIII)
   worin R¹⁰ und R¹¹ je niederes Alkyl bedeuten, umsetzt und das Reaktionsprodukt mit Verbindungen der allgemeinen Formel IV
   (siehe IV)
   worin R^{2'}, R^{3'}, n und A obige Bedeutung besitzen, wobei jedoch eine allfällige freie NH-Gruppe in dem Rest A mit einer Aminoschutzgruppe und allfällige freie Hydroxygruppen mit Hydroxy-Schutzgruppen versehen sind, umsetzt und anschließend allfällige Amino- oder Hydroxy-Schutzgruppen wieder abspaltet, oder
c) zur Herstellung von Verbindungen der allgemeinen Formel Ib
   (siehe Formel Ib)
   worin R¹, R^{2'}, R^{3'}, R⁴, R^{6'}, R^{7'}, Q und n obige Bedeutung besitzen und R^{5'} die für R5 angegebene Bedeutung besitzt, wobei jedoch, falls die Kettenlänge m des Restes Q 3 ist, R^{5'} eine an dem dem Sauerstoffatom benachbarten Stickstoffatom des Pyrazolringes angeordnete niedere Alkylgruppe oder Phenylniederalkylgruppe darstellt, Verbindungen der allgemeinen Formel V
   (siehe Formel V)
   worin R¹, R^{2'}, R^{3'} und n obige Bedeutung besitzen, wobei jedoch allfällige freie Hydroxygruppen mit einer Hydroxy-Schutzgruppe versehen sind, mit Verbindungen der allgemeinen Formel VI
   (siehe Formel VI)
   worin R⁴, R^{5'}, R^{6'}, R^{7'} und Q obige Bedeutung besitzen, wobei jedoch allfällige freie Hydroxygruppen mit einer Hydroxy-Schutzgruppe versehen sind, und Y eine aminolytisch abspaltbare Fluchtgruppe bedeutet, umsetzt und anschließend allfällige Hydroxy-Schutzgruppen wieder abspaltet, oder
d) zur Herstellung von Verbindungen der Formel Ia Verbindungen der allgemeinen Formel VII
   (siehe Formel VII)
   worin R^{2'}, R^{3'}, n und Y obige Bedeutung besitzen, wobei jedoch allfällige freie Hydroxygruppen mit einer Hydroxy-Schutzgruppe versehen sind, mit Verbindungen der allgemeinen Formel VIII
   (siehe Fomel VIII)
   worin R⁴, R⁵, R^{6'}, R^{7'} und A obige Bedeutung besitzen, wobei jedoch allfällige freie Hydroxygruppen mit einer Hydroxy-Schutzgruppe versehen sind, umsetzt und anschließend allfällige Hydroxy-Schutzgruppen wieder abspaltet, oder
e) zur Herstellung von Verbindungen der allgemeinen Formel Ic
   (siehe Formel Ic)
   worin R¹, R^{3'} R⁴, R⁵, R^{7'} und n obige Bedeutung besitzen, R^{2''} und R^{6''} die für R² und R⁶ angegebene Bedeutung mit Ausnahme von Nitro und niederem Alkanoyloxy besitzen, und Q' eine (CH₂)ₘ,-Gruppe, worin m' 1 bis 7 bedeutet und welche gegebenenfalls in α-Stellung zu dem Sauerstoffatom durch 1 bis 2 niedere Alkylgruppen substituiert ist, darstellt, Verbindungen der allgemeinen Formel IX
   (siehe Formel IX)
   worin R^{2''}, R^{3'}, R⁴, R⁵, R^{6''}, R^{7'}, n und Q' obige Bedeutung besitzen, wobei jedoch allfällige freie Hydroxygruppen mit einer Hydroxy-Schutzgruppe versehen sind, und R¹' niederes Alkyl oder eine Aminoschutzgruppe bedeutet, reduziert und anschließend allfällige Amino- und/oder Hydroxy-Schutzgruppen wieder abspaltet, oder
f) zur Herstellung von Verbindungen der allgemeinen Formel Id
   (siehe Formel Id)
   worin R⁴, n und A obige Bedeutung besitzen, R^{5'''} die für R⁵ angegebene Bedeutung besitzt, wobei jedoch R^{5'''} nicht Benzyl bedeutet, falls der Rest A eine NH-Gruppe enthält, und R^{2'''}, R^{3''}, R^{6'''} und R^{7''} die für R², R³, R⁶ und R⁷ angegebene Bedeutung mit Ausnahme von Hydroxy besitzen, wobei jedoch mindestens einer der Substituenten R^{2'''}, R^{3''}, R^{6'''} und R^{7''} niederes Alkanoyloxy bedeutet, in Verbindungen der allgemeinen Formel X
   (siehe Formel X)
   worin n und R⁴ obige Bedeutung besitzen, R^{2IV}, R^{3'''}, R^{6IV} und R^{7'''} die für R², R³, R⁶ und R⁷ angegebene Bedeutung mit Ausnahme von niederem Alkanoyloxy besitzen, wobei jedoch mindestens einer der Substituenten R^{2IV}, R^{3'''}, R^{6IV} und R^{7'''} Hydroxy bedeutet, R^{5IV} niederes Alkyl oder eine Phenylniederalkylgruppe bedeutet und A' die für A angegebene Bedeutung besitzt, wobei jedoch eine allfällige in dem Rest A' enthaltene NH-Gruppe durch eine Benzylschutzgruppe geschützt ist, die freien Hydroxygruppen R^{2IV}, R^{3'''}, R^{6'''} und/oder R^{7'''} acyliert und anschließend eine allfällige Benzylschutzgruppe wieder abspaltet, und
gewünschtenfalls in erhaltenen Verbindungen der Formel I, worin R², R³, R6 und/oder R⁷ Methoxy bedeuten, aus diesen Gruppen eine Hydroxygruppe freisetzt und/oder gewünschtenfalls aus erhaltenen Verbindungen der Formel I, worin R⁵ Benzyl bedeutet, die Benzylgruppe abspaltet, und gewünschtenfalls freie Verbindungen der Formel I in ihre Säureadditionssalze überführt oder die Säureadditonssalze in die freien Verbindungen der Formel I überführt.

Die Umsetzung von Verbindungen der Formel II mit Verbindungen der Formel III gemäß Verfahrensvariante a) kann auf an sich bekannte Weise unter zur Bildung von Pyrazol-ether-Verbindungen durch Alkylierung von Pyrazololen üblichen Bedingungen erfolgen. Als Fluchtgruppe X in den Verbindungen der Formel III kommen bevorzugt Halogene wie Chlor, Brom oder Jod oder auch organische Sulfonsäure-Reste in Frage, beispielsweise Reste von Niederalkansulfonsäuren wie z.B. Methansulfonsäure oder von aromatischen Sulfonsäuren wie Benzolsulfonsäure oder durch niederes Alkyl oder Halogen substituierten Benzolsulfonsäuren, z.B. Toluolsulfonsäuren oder Bromsulfonsäuren. Im allgemeinen werden in etwa äquivalente Mengen von Verbindungen der Formeln II und III eingesetzt. Falls R⁵ eine niedere Alkyl- oder Phenylniederalkylgruppe bedeutet kann auch ein Überschuß an Verbindungen der Formel III eingesetzt werden, falls jedoch R⁵ Wasserstoff bedeutet, ist es zweckmäßig einen Überschuß an Verbindungen der Formel II einzusetzen oder die freie NH-Funktion des Pyrazolringes durch eine anschließend wieder abspaltbare Amino-Schutzgruppe zu schützen, um Nebenreaktionen zu vermeiden. Die Umsetzung wird zweckmäßig in einem unter den Reaktionsbedingungen inerten organischen Lösungsmittel unter basischen Bedingungen durchgeführt. Als inerte organische Lösungsmittel eignen sich Dimethylformamid, Tetramethylharnstoff, Aceton oder je nach Art der als säurebindendes Mittel zugesetzten Base auch Ether, insbesondere cyclische Ether wie Tetrahydrofuran, niedere Alkohole, oder aromatische Kohlenwasserstoffe wie Benzol oder Toluol oder Gemische aus den vorgenannten Lösungsmitteln. Zweckmäßigerweise wird die Reaktion in Gegenwart einer mindestens äquivalenten Menge einer säurebindenden Base durchgeführt. Als Basen eignen sich z. B. anorganische oder organische Alkalimetallverbindungen. Beispiele von geeigneten Basen sind Alkalimetallcarbonate oder auch -hydroxide, Alkalimetallhydride wie Natriumhydrid oder niedere Alkalimetallalkoholate wie z. B. Kaliumtert butylat oder Natriummethylat oder Alkalimetallamide wie z. B. Lithiumamid oder Lithiumdiisopropylamid. Gewünschtenfalls können zur Beschleunigung der Reaktion katalytische Mengen eines Jodidsalzes, z. B. eines Alkalimetall- oder Ammoniumiodids wie Kaliumjodid oder tert. Butylammoniumjodid, zugesetzt werden. Die Reaktion kann bei Temperaturen zwischen Raumtemperatur und Siedetemperatur des Reaktionsgemisches durchgeführt werden. Zweckmäßig wird die Umsetzung z. B. unter Verwendung einer anorganischen Base wie Kaliumcarbonat oder Natriumhydrid in einem inerten Lösungsmittel wie Dimethylformamid bevorzugt bei Temperaturen zwischen 80 und 120° C durchgeführt. Gewünschtenfalls kann die Umsetzung auch in einem Zweiphasensystem in Gegenwart eines Phasentransferkatalysators, beispielsweise Benzyltriniederalkylammoniumchlorid durchgeführt werden. Eine allfällige Hydroxygruppe in der Gruppe A kann während der Reaktion durch eine anschließend leicht wieder abspaltbare Hydroxyschutzgruppe geschützt werden. Die Herstellung von Verbindungen der Formel Ia gemäß Verfahrensvariante b) kann unter den für Mitsunobu-Reaktionen üblichen Bedingungen erfolgen. Beispielsweise kann die Reaktion in einem inerten wasserfreien aprotischen organischen Lösungsmittel insbesondere einem cyclischen Äther wie Tetrahydrofuran oder Dioxan und/oder einem aromatischen Kohlenwasserstoff wie Benzol oder Toluol durchgeführt werden.

Die Umsetzungen von Verbindungen der Formel V mit Verbindungen der Formel VI und von Verbindungen der Formel VII mit Verbindungen der Formel VIII gemäß den Verfahrensvarianten c) und, d) können nach an sich zur Alkylierung von Aminen üblichen Methoden ausgeführt werden. Als aminolytisch abspaltbare Reste Y in den Verbindungen der Formeln VI und VII eignen sich Halogene wie Chlor, Brom oder Jod, vorzugsweise Brom oder Chlor, oder auch ein organischer Sulfonsäurerest, beispielsweise der Rest einer Niederalkansulfonsäure wie z. B. Methansulfonsäure oder von aromatischen Sulfonsäuren wie Benzolsulfonsäure oder durch niederes Alkyl oder durch Halogen substituierten Benzolsulfonsäuren, z. B. Toluolsulfonsäuren oder Brombenzolsulfonsäuren. Die Umsetzung kann in einem unter den Reaktionsbedingungen inerten organischen Lösungsmittel unter basischen Bedingungen durchgeführt werden. Es können die vorstehend für die Verfahrensvariante a) angegebenen Lösungsmittel, Basen und Reaktionsbedingungen eingesetzt werden. Eine allfällige Hydroxygruppe in der Kette Q oder der Gruppe A wird zweckmäßigerweise wie für die Verfahrensvariante a) angegeben während der Reaktion durch eine leicht wieder abspaltbare Hydroxy-Schutzgruppe geschützt. Zweckmäßig wird die Reaktion bei Temperaturen zwischen Raumtemperatur und Siedetemperatur des Reaktionsgemisches ausgeführt und kann je nach Reaktionsbedingungen 1/4 bis 6 Stunden benötigen. Zweckmäßig wird die Umsetzung z. B. in einem inerten Lösungsmittel wie Dimethylformamid in Gegenwart einer anorganischen Base wie Kaliumcarbonat bei Temperaturen zwischen 80 und 120 °C durchgeführt. Die Verfahrensvarianten c) und d) eignen sich insbesondere für die Herstellung solcher Verbindungen der Formel I, worin R⁵ eine an das dem Sauerstoffatom benachbarte Stickstoffatom gebundene niedere Alkyl- oder Phenylniederalkylgruppe bedeutet.

Die Reduktion von Verbindungen der Formel IX gemäß Verfahrensvariante e) kann nach an sich zur Reduktion von Amiden üblichen Methoden erfolgen. Als Reduktionsmittel eignen sich zur Amidreduktion befähigte komplexe Metallhydride, insbesondere Aluminiumhydride wie Lithiumalumniumhydrid. Die Reaktion findet in einem unter den Reaktionsbedingungen inerten ausreichend wasserfreien Lösungsmittel statt. Als Lösungsmittel eignen sich beispielsweise cyclische Ether wie Tetrahydrofuran oder Dioxan oder offenkettige Ether wie Ethylenglycoldimethylether oder Diethylenglycoldimethylether, gegebenenfalls im Gemisch mit aromatischen Kohlenwasserstoffen wie Benzol oder Toluol. Die Reaktion kann je nach Art des verwendeten Reduktionsmittels bei erhöhter Temperatur, beispielsweise Siedepunkt des Reaktionsgemisches durchgeführt werden. Als günstig erweist sich beispielsweise die Reaktion mit Lithiumaluminiumhydrid bei Siedetemperatur des Reaktionsgemisches. Die Reaktionszeit kann zwischen 1 und 10 Stunden betragen.

Die Acylierung von Verbindungen der Formel X gemäß Verfahrensvariante (f) kann nach an sich zur Phenolesterbildung üblichen Methoden durch Umsetzen von Verbindungen der Formel XII mit einem reaktiven Derivat einer entsprechenden niederen Carbonsäure, beispielsweise einem niederen Carbonsäurehalogenid oder -anhydrid, erfolgen.

Falls die Gruppe A' in den Verbindungen der Formel X eine durch Benzyl geschützte NH-Funktion enthält, kann diese Benzylschutzgruppe anschließend nach an sich für Benzylaminspaltungen bekannten Methoden wieder abgespalten werden. Hierbei wird eine allfällige Benzylgruppe R⁵ ebenfalls mit abgespalten. Vorteilhaft ist beispielsweise eine reduktive Spaltung. Diese kann unter Verwendung von Ameisensäure als Reduktionsmittel in Gegenwart eines Palladium-Katalysators durchgeführt werden oder hydrogenolytisch unter Verwendung von Wasserstoff als Reduktionsmittel in Gegenwart eines Palladium-Katalysators erfolgen.

Falls die Reste R^{2'}, R^{3'}, R6' und R^{7'} freie Hydroxysubstituenten darstellen, müssen diese während der vorstehend be-schriebenen Umsetzungen gemäß Verfahrensvarianten a) - e) sowie auch bei der Herstellung der Ausgangsstoffe auf an sich bekannte Weise durch leicht wieder abspaltbare Schutzgruppen geschützt werden. Geeignete nach der Reaktion leicht wieder abspaltbare Schutzgruppen für Hydroxylgruppen sind z.B. bekannt aus E. McOmie "Protective Groups in Organic Chemistry" Plenum Press 1971. Beispielsweise eignen sich zum Schutz einer Hydroxylgruppe Ester, z.B. Acetate, leicht abspaltbare Carbonate wie Benzylcarbonate und leicht abspaltbare Äther wie Tetrahydropyranyläther oder Benzyläther. Es müssen jeweils Schutzgruppen gewählt werden, die während der durchgeführten Reaktionen nicht angegriffen werden und die anschließend unter Bedingungen abspaltbar sind, unter denen die gebildeten Produkte nicht angegriffen werden. Bevorzugt können Ätherschutzgruppen, wie Benzyläther verwendet werden. So werden bei der Umsetzung von Ausgangsverbindungen der Formel II mit Ausgangsverbindungen der Formel III, worin mindestens einer der Reste R^{2'}, R^{3'}, R^{6'} und R7 eine durch Benzyletherbildung geschützte Hydroxygruppe darstellt, zunächst Verbindungen der allgemeinen Formel XXVIII erhalten
(siehe Formel XXVIII)
worin R⁴, R⁵, A und n obige Bedeutung besitzen und die Reste R^{2V}, R^{3IV}, R^{6V} und R^{7IV} die für R^{2'}, R^{3'}, R^{6'} und R^{7'} angegebene Bedeutung besitzen oder Benzyloxy bedeuten, wobei jedoch mindestens einer der Reste R^{2V}, R^{3IV}, R^{6V} und R^{7IV} Benzyloxy ist. Die Verbindungen der Formel XXVIII sind neu und stellen wertvolle Zwischenprodukte zur Herstellung von pharmakologisch wirksamen Verbindungen, beispielsweise Verbindungen der Formel I, dar und besitzen auch selbst herzwirksame den Eigenschaften der Verbindungen der Formel I ähnliche pharmakologische Eigenschaften.

Falls der Rest R⁵ Wasserstoff darstellt und/oder der Rest A eine freie NH-Gruppe enthält, können die freien NH-Gruppen gewünschtenfalls auf an sich bekannte Weise durch leicht wieder abspaltbare Schutzgruppen geschützt werden. Dies kann zum Beispiel zweckmäßig sein während der Umsetzung der Verbindungen der Formel V mit den Verbindungen der Formel VI, falls die Kettenlänge m des Restes Q 3 ist. Geeignete nach der Reaktion leicht wieder abspaltbare Schutzgruppen für NH-Gruppen sind z.B. bekannt aus E. McOmie "Protective Groups in Organic Chemistry" Plenum Press 1971. Beispielsweise eignen sich zum Schutz einer NH-Funktion im Pyrazolring die Benzyl- und die Tritylgruppe oder die Tetrahydropyranylgruppe. Es müssen jeweils Schutzgruppen gewählt werden, die während der durchgeführten Reaktionen nicht angegriffen werden und die anschließend unter Bedingungen abspaltbar sind, unter denen die gebildeten Produkte nicht angegriffen werden. Bevorzugt kann die Benzylgruppe als Schutzgruppe verwendet werden.

In Verbindungen der Formel I, worin die Substituenten R², R³, R⁶ und/oder R⁷ Methoxy bedeuten, kann aus diesen Gruppen gewünschtenfalls nachträglich die Hydroxygruppe freigesetzt werden. Methoxygruppen können mit zur Spaltung von Methoxyarylethern geeigneten Methoden auf an sich bekannte Weise zu Hydroxygruppen gespalten werden. Beispielsweise kann die Etherspaltung durch Behandeln mit Jodwasserstoff oder Bromwasserstoff in einem unter den Reaktionsbedingungen inerten Lösungsmittel, z. B. Acetanhydrid oder Essigsäure, oder mit Jodtrimethylsilan oder Bortribromid in einem halogenierten Kohlenwasserstoff wie Dichlormethan erfolgen. Allfällige niedere Alkanoyloxygruppen werden unter den Bedingungen der Etherspaltung ebenfalls gespalten.

Aus Verbindungen der Formel I, worin R⁵ eine Benzylgruppe darstellt, kann diese Benzylgruppe nachträglich nach an sich für Benzylaminspaltungen bekannten Methoden abgespalten werden, um entsprechende Verbindungen der Formel I, worin R⁵ Wasserstoff bedeutet, zu erhalten. Die Abspaltung der Benzylgruppe kann durch reduktive oder hydrogenolytische Spaltung, beispielsweise bei unter Verfahrensvariante g) zur Abspaltung einer Benzylschutzgruppe angegebenen Bedingungen, erfolgen.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit anorganischen Säuren, z. B. Halogenwasserstoffsäuren, insbesondere Chlorwasserstoffsäure, Schwefelsäure oder Phosphorsäuren, oder mit organischen Säuren, beispielsweise niederen aliphatischen Mono- oder Dicarbonsäuren wie Maleinsäure, Fumarsäure, Milchsäure, Weinsäure oder Essigsäure, oder Sulfonsäuren, beispielsweise Niederalkansulfonsäuren, wie Methansulfonsäure oder gegebenenfalls im Benzolring durch Halogen oder niederes Alkyl substituierte Benzolsulfonsäuren wie p-Toluolsulfonsäure, oder Cyclohexylaminsulfonsäure.

Falls in den Verbindungen der Formel I A die Gruppe b) darstellt enthalten die Verbindungen ein Chiralitätszentrum und können in zwei optisch aktiven Formen oder als Racemat vorliegen. Die vorliegende Erfindung umfaßt sowohl die racemischen Gemische wie auch die optischen Isomeren dieser Verbindungen der Formel I. Die optisch aktiven Verbindungen können aus den racemischen Gemischen in an sich bekannter Weise durch übliche Trennverfahren erhalten werden, z. B. durch chromatographische Trennung an chiralen Trennmaterialien oder fraktionierte Kristallisation geeigneter Salze mit optisch aktiven Säuren. Enantiomerenreine Verbindungen können auch durch Synthese aus entsprechenden enantiomerenreinen Ausgangsverbindungen hergestellt werden. So können beispielsweise sterisch reine Isomere der Ausgangsverbindungen der Formeln III, IV oder VIII nach den vorstehend beschriebenen Verfahrensvarianten a), b) und d) in sterisch reine Verbindungen der Formel I überführt werden.

Die Ausgangsverbindungen der Formel II sind bekannt oder können nach an sich bekannten Verfahren hergestellt werden. Es ist bekannt, daß die Verbindungen der Formel II in mehreren tautomeren Formen vorliegen und daß neben der durch die Formel II wiedergegebenen Enolform der 5-Phenylpyrazol-3-ole auch die entsprechende Ketoform der 5-Phenylpyrazol-3-one existiert. Im allgemeinen liegen Gemische aus den verschiedenen tautomeren Formen vor, deren Zusammensetzung je nach Art der Substituenten variieren kann. Beide Formen beziehungsweise deren Gemische können zur erfindungsgemäßen Herstellung von Verbindungen der Formel I verwendet werden. Mit Verbindungen der Formel II sind daher in der vorliegenden Anmeldung alle tautomeren Formen dieser Verbindungen gemeint.

Verbindungen der Formel II können nach an sich zur Herstellung von 5-Phenylpyrazolin-3-onen bekannten Verfahren erhalten werden, z. B. durch cyclisierende Kondensation von gegebenenfalls substituierten Hydrazinen der allgemeinen Formel XIX
(siehe Formel XIX)
worin R⁵ obige Bedeutung besitzt, mit Benzoylessigsäureestern der allgemeinen Formel XX
(siehe Formel XX)
worin R^{2'}, R^{3'} und R⁴ obige Bedeutung besitzen, oder mit Phenylpropiolsäureestern der allgemeinen Formel XXI
(siehe Formel XXI)
worin R^{2'} und R^{3'} obige Bedeutung besitzen. Die Umsetzung erfolgt in einem unter den Reaktionsbedingungen inerten organischen Lösungsmittel. Falls R⁵ niederes Alkyl oder eine Phenylniederalkylgruppe bedeutet, entstehen bei der Umsetzung Gemische von isomeren Verbindungen worin R⁵ in 1- oder 2-Stellung angeordnet ist. Das Verhältnis von 1- zu 2-substituierten Verbindungen kann je nach Art der Ausgangsstoffe und des verwendeten Lösungsmittels variieren. Isomeren-Gemisch aus 1- und 2-substituierten Verbindungen können auf an sich bekannte Weise durch fraktionierte Kristallisation oder durch Chromatographie getrennt werden.

In Verbindungen der Formel II, worin R⁵ Wasserstoff bedeutet, kann gewünschtenfalls auf an sich bekannte Weise eine Benzylgruppe R⁵ nachträglich eingeführt werden, indem man die Verbindungen mit einem Benzylhalogenid, vorzugsweise Benzylbromid, in einem unter den Reaktionsbedingungen inerten organischen Lösungsmittel, beispielsweise einem aromatischen Kohlenwasserstoff wie Toluol, in Gegenwart einer Base, beispielsweise Pyridin oder vorzugsweise Collidin, umsetzt. Bei der Benzylierung können Isomerengemische aus in 1-Stellung und in 2-Stellung des Pyrazolringes benzylierten Verbindungen auftreten. Diese können auf an sich bekannte Weise, z. B. durch fraktionierte Kristallisation oder durch Chromatographie getrennt werden.

Verbindungen der Formel III sind bekannt oder können nach an sich bekannten Methoden oder analog zu an sich bekannten Methoden hergestellt werden. Beispielsweise können Verbindungen der allgemeinen Formel IIIa
(siehe Formel IIIa)
worin R¹, R^{2'}, R^{3'}, n, Q und X obige Bedeutung besitzen, erhalten werden, indem man Amine der Formel V mit Verbindungen der allgemeinen Formel XXX
(siehe Formel XXX)
worin Q obige Bedeutung besitzt, Y obige Bedeutung besitzt und vorzugsweise Halogen, insbesondere Chlor oder Brom bedeutet und T für eine Fluchtgruppe X oder für Hydroxy steht, umsetzt, und anschließend die Gruppe T gewünschtenfalls auf an sich bekannte Weise in eine andere Fluchtgruppe X umwandelt. Die Umsetzung kann nach an sich zur Aminoalkylierung üblichen Methoden, beispielsweise bei den vorstehend für die Umsetzung von Verbindungen der Formel V mit Verbindungen der Formel VI angegebenen Bedingungen erfolgen. Zur Vermeidung von Nebenreaktionen ist es zweckmäßig, einen Überschuß an Verbindungen der Formel XXX einzusetzen. Sofern in den Verbindungen der Formel XXX der Rest T eine Fluchtgruppe X darstellt, ist es vorteilhaft wenn die beiden in der Verbindung der Formel XXX enthaltenen Fluchtgruppen unterschiedliche Reaktivität aufweisen, um eine gleichzeitige Reaktion beider Fluchtgruppen mit Verbindungen der Formel V zu vermeiden. Sofern die Amine der Formel V mit Halogenalkoholen der Formel XXX umgesetzt wurden, kann in dem Reaktionsprodukt anschließend die Hydroxygruppe auf an sich bekannte Weise gegen einen Rest X ausgetauscht werden. So können die Hydroxyverbindungen beispielsweise zur Einführung eines Halogenrestes X mit Thionylchlorid oder mit Phosphorhalogeniden auf an sich bekannte Weise umgesetzt werden. Sulfonsäurereste X können auf an sich bekannte Weise eingeführt werden, indem man die Hydroxyverbindungen mit einem entsprechenden Sulfonsäurehalogenid acyliert.

Verbindungen der allgemeinen Formel IIIb
(siehe Formel IIIb)
worin R^{2'}, R^{3'}, n, p, B und X obige Bedeutung besitzen, können beispielsweise erhalten werden, indem man Verbindungen der allgemeinen Formel VII mit Verbindungen der allgemeinen Formel XXII
(siehe Formel XXII)
worin p und B obige Bedeutung besitzen, umsetzt und in den erhaltenen Verbindungen der allgemeinen Formel XXIII
(siehe Formel XXIII)
worin R^{2'}, R^{3'}, n, p und B obige Bedeutung besitzen, die Hydroxygruppe auf an sich bekannte Weise gegen einen Rest X austauscht. Die Umsetzung der Verbindungen der Formel VII mit den Aminen der Formel XXII kann nach an sich zur Aminoalkylierung üblichen Methoden, beispielsweise unter den für die Verfahrensvarianten c) und d) angegebenen Bedingungen erfolgen.

Verbindungen der Formel IV können erhalten werden, indem man Verbindungen der Formel VII unter zur Aminoalkylierung üblichen Bedingungen mit Aminoalkoholen der allgemeinen Formel XXIV
(siehe Formel XXIV)
worin A obige Bedeutung besitzt, umsetzt.

Verbindungen der Formel V sind bekannt oder können nach an sich bekannten Methoden oder analog zu an sich bekannten Methoden hergestellt werden. Beispielsweise können Verbindungen der Formel V ausgehend von entsprechenden Säuren der allgemeinen Formel XIV
(siehe Formel XIV)
worin R^{2'}, R^{3'} und n obige Bedeutung besitzen, erhalten werden. So können reaktionsfähige Säurederivate der Säuren der Formel XIV mit Aminen der allgemeinen Formel XIII
(siehe Formel XIII)
worin R¹ obige Bedeutung besitzt, zu entsprechenden Amiden umgesetzt und diese anschließend zu Verbindungen der Formel V reduziert werden. Die Reduktion kann beispielsweise unter den zur Reduktion der Verbindungen der Formel IX gemäß Verfahrensvariante e) angegebenen Bedingungen erfolgen.

Säuren der Formel XIV sind bekannt oder können auf an sich bekannte Weise erhalten werden.

Verbindungen der Formel VI können auf an sich bekannte Weise erhalten werden, indem man entsprechende Verbindungen der Formel II mit Verbindungen der allgemeinen Formel XXIX
(siehe Formel XXIX)
worin X und Q obige Bedeutung besitzen und T' für eine aminolytisch abspaltbare Gruppe Y oder für Hydroxy steht, umsetzt und anschließend die Gruppe T' gewünschtenfalls in eine aminolytisch abspaltbare Gruppe Y überführt. Die Umsetzung kann unter den zur Umsetzung von Verbindungen der Formel II mit Verbindungen der Formel III gemäß Verfahrensvariante a) angegebenen Bedingungen erfolgen. Zur Vermeidung von Nebenreaktionen ist es zweckmäßig einen Überschuß an Verbindungen der Formel XXIX einzusetzen. Sofern in den Verbindungen der Formel XXIX der Rest T' eine Fluchtgruppe Y darstellt, ist es vorteilhaft, wenn die beiden in der Verbindung der Formel XXIX enthaltenen Fluchtgruppen unterschiedliche Reaktivität aufweisen. Sofern Verbindungen der Formel XXIX eingesetzt werden, worin T' Hydroxy bedeutet, kann die Hydroxygruppe in den erhaltenen Verbindungen anschließend auf an sich bekannte Weise in eine Gruppe Y überführt werden. Dies kann beispielsweise in der vorstehend bei der Herstellung der Verbindungen der Formel III beschriebenen Weise erfolgen.

Verbindungen der Formel VIII können erhalten werden, indem man Verbindungen der Formel II mit Verbindungen der allgemeinen Formel XXV
(siehe Formel XXV)
worin A und X obige Bedeutung besitzen und D eine solvolytisch oder hydrogenolytisch abspaltbare Schutzgruppe bedeutet, umsetzt und aus den erhaltenen Reaktionsprodukten der allgemeinen Formel XXVI
(siehe Formel XXVI)
worin R⁴, R⁵, R^{6'}, R^{7'}, A und D obige Bedeutung besitzen, die Schutzgruppe wieder abspaltet. Die Umsetzung von Verbindungen der Formel II mit den Verbindungen der Formel XXV kann auf an sich bekannte Weise, beispielsweise unter den für die Umsetzung der Verbindungen der Formel II mit Verbindungen der Formel III gemäß Verfahrensvariante a) angegebenen Bedingungen erfolgen. Als Schutzgruppe D eignen sich beispielsweise hydrogenolytisch abspaltbare Reste wie Benzyl, welches durch katalytische Hydrierung in Gegenwart eines Palladium-Katalysators abgespalten werden kann.

Verbindungen der Formel XXV sind bekannt oder können auf an sich bekannte Weise, beispielsweise analog der Herstellung der Verbindungen der Formel III, erhalten werden. So können Verbindungen der allgemeinen Formel XXVa
(siehe Formel XXVa)
worin R¹, Q, D und X obige Bedeutung besitzen, durch Einführung einer Aminoschutzgruppe D in Amine der Formel XIII und anschließende Umsetzung der geschützten Amine mit Verbindungen der Formel X erhalten werden, und Verbindungen der allgemeinen Formel XXVb
(siehe Formel XXVb)
worin D, p, B und X obige Bedeutung besitzen, können durch Einführung einer Aminoschutzgruppe D in Aminoalkohole der Formel XXII und anschließende Überführung der Hydroxygruppe in einen Rest X erhalten werden. Die Ausgangsverbindungen der Formel IX sind neue Verbindungen, welche wertvolle Zwischenprodukte zur Herstellung von pharmakologisch wirksamen Verbindungen, beispielsweise den Verbindungen der Formel IC darstellen.

Verbindungen der Formel IX können nach an sich zur Amidbildung üblichen Methoden erhalten werden, indem man Amine der Formel V mit Säuren der allgemeinen Formel XV
(siehe Formel XV)
worin R⁴, R⁵, R^{6''}, R^{7'} und Q' obige Bedeutung besitzen, oder deren reaktionsfähigen Säurederivaten unter zur Aminoacylierung üblichen Bedingungen umsetzt. So können die Amine der Formel V mit reaktionsfähigen Derivaten der Säuren der Formel XV zweckmäßig in einem unter den Reaktionsbedingungen inerten Lösungsmittel und in Gegenwart eines säurebindenden Reagenzes umgesetzt werden. Als reaktionsfähige Derivate der Säuren der Formel XV kommen insbesondere Säurehalogenide, gegebenenfalls gemischte Säureanhydride und Ester in Frage. Als Lösungsmittel eignen sich halogenierte Kohlenwasserstoffe wie Dichlormethan, Chloroform, Dichlorethan oder Tetrachlorkohlenstoff, aromatische Kohlenwasserstoffe wie Benzol, Toluol oder Chlorbenzol, cyclische Ether wie Tetrahydrofuran oder Dioxan, Dimethylformamid oder Gemische dieser Lösungsmittel. Als säurebindende Reagenzien eignen sich organische oder anorganische Basen. Beispiele geeigneter organischer Basen sind tertiäre organische Amine, insbesondere tertiäre niedere Alkylamine wie Triethylamin, Tripropylamine oder N-Niederalkylpiperidine. Geeignete anorganische Basen sind insbesondere Alkalimetallcarbonate oder -bicarbonate. Falls als Acylierungsmittel die Säure selbst oder auch ein Ester eingesetzt werden, kann die Umsetzung der Verbindungen der Formel V mit den Säuren der Formel XV zweckmäßigerweise in Gegenwart eines dehydratisierenden Reagenzes, beispielsweise eines aus der Peptidchemie zur Amidbildung geeignet bekannten Kopplungsreagenzes, durchgeführt werden. Als Beispiele derartiger Reagenzien, welche die Acylierung auch dadurch fördern, daß sie mit der Säure in situ unter Bildung eines reaktionsfähigen Säurederivates reagieren, seien insbesondere genannt Alkyl-, vorzugsweise Cycloalkylcarbodiimide wie Dicyclohexylcarbodiimid, Carbonylimidazol oder N-Niederalkyl-2-halogenpyridiniumsalze, insbesondere Halogenide oder Tosylate, vorzugsweise N-Methyl-2-chlorpyridiniumjodid (= Mukayama Reagenz). Die Umsetzung in Gegenwart eines derartigen Kopplungsreagenzes kann zweckmäßigerweise bei Temperaturen von -30 °C bis +50 °C unter neutralen Reaktionsbedingungen in Lösungsmitteln wie halogenierten Kohlenwasserstoffen und/oder aromatischen Kohlenwasserstoffen durchgeführt werden. Wenn als Ausgangsverbindungen Derivate von Säuren der Formel XV, worin R⁵ Wasserstoff ist, eingesetzt werden, können diese unter den vorgenannten Reaktionsbedingungen für die Amidbildung zunächst, insbesondere falls Q' eine Methylengruppe darstellt, teilweise in entsprechende cyclische Verbindungen der allgemeinen Formel XVa
(siehe Formel XVa)
worin R⁴, R^{6''}, R^{7'} und Q' obige Bedeutung besitzen, umgewandelt werden, welche dann mit den Aminen der Formel V weiter reagieren.

Die Säuren der Formel XV können nach an sich bekannten Methoden erhalten werden, indem man entsprechende Verbindungen der Formel II mit Halogencarbonsäureestern oder -nitrilen der allgemeinen Formeln XVIa und XVIb
(siehe Formeln XVIa und XVIb)
worin Q' obige Bedeutung besitzt, Hal für Halogen, insbesondere Chlor oder Brom, steht und R12 niederes Alkyl bedeutet, umsetzt und die erhaltenen Ester oder Nitrile anschließend zu den Säuren der Formel XV hydrolysiert. Die Umsetzung von Verbindungen der Formel II mit den Verbindungen der Formeln XVIa oder XVIb kann nach an sich zur Alkylierung am Sauerstoffatom in 3-Stellung von 5-Phenylpyrazolin-3-onen bekannten Verfahren unter basischen Bedingungen, beispielsweise unter den für die Verfahrensvariante a) angegebenen Bedingungen, durchgeführt werden. Zweckmäßig wird die Reaktion in Dimethylformamid in Gegenwart von Kaliumcarbonat durchgeführt. Die anschließende Hydrolyse zu den Säuren der Formel XV erfolgt vorzugsweise unter alkalischen Bedingungen. Die Säuren der Formel XV können auf an sich bekannte Weise in ihre reaktiven Säurederivate überführt werden. Hierbei können sie teilweise zu entsprechenden cyclischen Verbindungen der Formel XVa kondensiert werden. Da cyclische Verbindungen der Formel XVa in gleicher Weise wie die reaktiven Derivate der Säuren der Formel XV mit den Aminen der Formel V weiter umgesetzt werden können, erübrigt sich die Auftrennung etwaiger Gemische aus Derivaten der Säuren der Formel XV und Verbindungen der Formel XVa.

Verbindungen der Formel XI können auch erhalten werden, indem man Verbindungen der allgemeinen Formel XXVII
(siehe Formel XXVII)
worin R¹, R^{2''}, R^{3'}, n, Q' und X obige Bedeutung besitzen, mit entsprechenden Verbindungen der Formel II umsetzt. Die Umsetzung kann auf an sich bekannte Weise, beispielsweise bei den in Verfahrensvariante a) zur Umsetzung von Verbindungen der Formel II mit Verbindungen der Formel III angegebenen Bedingungen erfolgen.

Verbindungen der Formel XXVII können erhalten werden, indem man Amine der allgemeinen Formel Va
(siehe Formel Va)
worin R^{1'}, R^{2'}, R^{3'} und n obige Bedeutung besitzen, mit reaktionsfähigen Derivaten der Säuren der allgemeinen Formel XVII
(siehe Formel XVII)
worin Q' und X obige Bedeutung besitzen, nach zur Aminoacylierung üblichen Methoden umsetzt. Die Umsetzung kann beispielweise unter den für die Umsetzung von Aminverbindungen der Formel V mit raktionsfähigen Derivaten der Säuren der Formel XV angegebenen Bedingungen erfolgen. Als reaktionsfähige Derivate der Säuren der Formel XVII werden vorzugsweise deren Halogenide eingesetzt.

Verbindungen der Formel X stellen mit Ausnahme derjenigen Verbindungen, worin A' eine durch eine Benzylgruppe geschützte NH-Gruppe enthält, entsprechende Verbindungen der Formel I dar.

Die übrigen Verbindungen der allgemeinen Formel Xa
(siehe Formel Xa)
worin R^{2IV}, R^{3'''}, R⁴, R^{5IV}, R^{6IV}, R^{7'''} und n obige Bedeutung besitzen, R¹² Benzyl bedeutet und Q'' eine (CH₂)ₘ-Gruppe, worin m 2 bis 8 bedeutet, und welche gegebenenfalls in α-Stellung zu dem Sauerstoffatom durch 1 bis 2 niedere Alkylgruppen substituiert sein kann, darstellt, können erhalten werden, indem man entsprechende Verbindungen der allgemeinen Formel IIIc
(siehe Formel IIIc)
worin R^{2IV}, R^{3'''}, R¹², n, Q'' und X obige Bedeutung besitzen, unter den vorstehend für die Verfahrensvariante a) angegebenen Bedingungen umsetzt. Verbindungen der Formel IIIc können nach den zur Herstellung von Verbindungen der Formel IIIa beschriebenen Methoden erhalten werden. Beispielsweise können entsprechende Säuren der Formel XIV mit Benzylamin zu Benzylamiden umgesetzt werden, welche anschließend zu Aminen reduziert werden können, die dann mit entsprechenden Verbindungen der Formel XXX zu Verbindungen der Formel IIIc umgesetzt werden können.

Die Verbindungen der Formel I und ihre pharmakologische akzeptablen Säureadditionssalze zeichnen sich durch interessante pharmakologische Eigenschaften aus. Insbesondere zeigen die Verbindungen herzkreislaufwirksame Eigenschaften und zeichnen sich durch ausgeprägte herzfrequenzsenkende Wirkungen aus. Dabei besitzen sie ein zur Behandlung ischämischer Zustände wie z. B. bei koronarer Herzkrankheit günstiges Wirkungsprofil, da in der Substanzgruppe auch zytoprotektive Wirkungen vorhanden sind.

Die herzwirksamen Eigenschaften der Verbindungen lassen sich in pharmakologischen Standardtestmethoden in vitro und in vivo nachweisen.

Beschreibung der pharmakologischen Untersuchungsmethoden:

### 1. Bestimmung der minimalen toxischen Dosis

Männlichen Mäusen von 20 bis 25 g Gewicht werden p.o. Maximaldosen von 300 mg/kg der Testsubstanz verabreicht. Die Tiere werden 3 Stunden lang sorgfältig auf Toxizitätssymptome beobachtet. Über einen Zeitraum von 72 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird in der nachfolgenden Tabelle A als minimale toxische Dosis angegeben. Die in Tabelle A angegebenen Beispielnummern beziehen sich auf die nachstehenden Herstellungsbeispiele.

**Tabelle A**

| Testsubstanz Beispiel Nr. | Minimale toxische Dosis mg/kg Maus p.o. |
|---|---|
| 1 | > 300 |
| 7 | > 300 |
| 8 | > 300 |
| 9 | > 300 |
| 15 | > 300 |
| 17 | > 300 |
| 18 | > 300 |
| 21 | > 300 |
| 26 | > 300 |
| 44 | 300 |
| 46 | > 300 |
| 47 | > 300 |

### 2. In-vitro-Nachweis der herzfrequenzsenkenden Wirkung.

Der direkte Einfluß der Wirksubstanzen auf die Herzfrequenz (FRQ) wurde an spontan schlagenden, isolierten rechten Vorhöfen von männlichen Pirbright-white Meerschweinchen der Gewichtsklasse 250-300 g geprüft. In der nachfolgenden Tabelle B ist als FRQ 75 diejenige Konzentration in µmol/l angegeben, bei der es 20 Minuten nach der Substanzgabe zu einer Abnahme der Frequenz auf 75 % des Ausgangswertes kommt. Die für die Testsubstanzen in der Tabelle B angegebenen Beispielnummern beziehen sich auf die nachstehenden Herstellungsbeispiele.

**Tabelle B**

| Testsubstanz Beispiel Nr. | Herzfrequenz senkende Wirkung Effektive Konzentration in µmol/l zur Erreichung von FRQ 75 % |
|---|---|
| 1 | 1,6 |
| 3 | 1,7 |
| 6 | 6,1 |
| 7 | 2,6 |
| 8 | 2,5 |
| 9 | 5,7 |
| 12 | 3,5 |
| 13 | 0,9 |
| 15 | 4,3 |
| 17 | 4,0 |
| 18 | 1,3 |
| 21 | 3,4 |
| 26 | 5,6 |
| 27 | 1,9 |
| 32 | 9,1 |
| 44 | 1,6 |
| 46 | 7,0 |
| 47 | 6,4 |
| 51 | 3,0 |
| 52 | 3,5 |
| 53 | 4,9 |

### 3. In vitro Nachweis der zytoprotektiven Wirkung

Die zytoprotektive Wirkung wurde an isolierten in einer Nährlösung gehaltenen elektrisch gereizten linken Vorhöfen von männlichen Pirbright-White Meerschweinchen der Gewichtsklasse 250 bis 300 g geprüft. Durch Begasung der Nährlösung mit Stickstoff für 60 Minuten wurde eine zeitweilige Hypoxie herbeigeführt. Infolge der Hypoxie kam es zu einer Kontraktur der Vorhöfe. Als Meßparameter diente das Integral der Kontraktur über den Zeitraum der Hypoxie. In der nachfolgenden Tabelle C ist die effektive Konzentration in µmol/l angegeben, bei der das Kontrakturintegral auf 50 % des Kontrollwertes reduziert wurde.

**Tabelle C**

| Testsubstanz Beispiel Nr. | Zytoprotektive Wirkung gegenüber der durch Hypoxie induzierten Vorhof-Kontraktur. Effektive Konzentration in µmol/l |
|---|---|
| 21 | 3,0 |
| 26 | 2,8 |
| 44 | 0,27 |

In in vitro-Versuchen an isolierten Herzmuskelzellen zeigten die Verbindungen cytoprotektive Wirkungen gegenüber cytotoxischen Substanzen wie Veratridin oder einer Kombination aus Oligomycin und Desoxyglucose.

Aufgrund ihrer vorstehend beschriebenen Wirkungen sind die Verbindungen der Formel I als herzwirksame Arzneimittel für größere Säugetiere, insbesondere Menschen, geeignet zur Behandlung von ischämischen Zuständen wie z. B. bei koronaren Herzkrankheiten und deren Folgeerscheinungen wie z. B. Herzinsuffizienz. Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoffe enthalten als orale Präparate. Im allgemeinen eignen sich jedoch für Applikationen an größeren Säugetieren, insbesondere Menschen, Arzneiformen mit einem Wirkstoffgehalt von 1 bis 200 mg pro Einzeldosis.

Als Heilmittel können die Verbindungen der Formel I mit üblichen pharmazeutischen Hilfsstoffen in galenischen Zubereitungen, wie z.B. Tabletten, Kapseln, Suppositorien oder Lösungen, enthalten sein. Diese galenischen Zubereitungen können nach an sich bekannten Methoden hergestellt werden unter Verwendung üblicher fester oder flüssiger Trägerstoffe wie z. B. Milchzucker, Stärke oder Talkum oder flüssigen Paraffinen und/oder unter Verwendung von üblichen pharmazeutischen Hilfstoffen, beispielsweise Tablettensprengmitteln, Lösungsvermittlern oder Konservierungsmitteln.

Die nachfolgenden Beispiele sollen die Erfindung näher erläutern. Die Strukturen der neuen Verbindungen wurden durch spektroskopische Untersuchungen, insbesondere durch Analyse der NMR-, Massen-, IR-/oder UV-Spektren gesichert.

### Beispiel 1:

### 3-{3-[N-(2-(3,4-Dimethoxyphenyl)-ethyl)-N-methylamino]-propyloxy}-5-(3,4-dimethoxyphenyl)-pyrazol

A) Zu 100 ml 21 %iger Natriumethylatlösung in Ethanol (= Lösung I) wurden unter Eiskühlung tropfenweise 32 ml Acetessigsäureethylester zugesetzt. Zu der Reaktionslösung wurden nach 20 Minuten 75 ml einer Lösung von 50 g 3,4-Dimethoxybenzoylchlorid in 150 ml Tetrahydrofuran (= Lösung II) zugetropft. Danach wurden dem Reaktionsgemisch abwechselnd, jeweils im Abstand von 20 Minuten weitere Mengen der Lösungen I und II zugetropft, bis das Reaktionsgemisch insgesamt 190 ml der Lösung I und 176 ml der Lösung II enthielt. Nach 16 Stunden wurden die ausgefallenen Salze abfiltriert, mit Tetrahydrofuran gewaschen und dann in Wasser aufgeschwemmt. Das wäßrige Gemisch wurde unter Eiskühlung auf pH 1 angesäuert und mit Methyl-tert.-Butylether extrahiert. Der Extrakt wurde zur Trockene eingedampft und es wurden 45 g 2-Acetyl-2-(3,4-dimethoxybenzoyl)-essigsäureethylester als gelbes Öl erhalten.
B) 45 g des unter A) erhaltenen Produktes wurden in 197 ml wäßrigem Ethanol unter Zusatz von 0,72 g Natriumacetat am Rückfluß gekocht. Anschließend wurde das Reaktionsgemisch dreimal unter Zusatz von Ethanol zur Trockene eingedampft. Der Rückstand wurde in Dichlormethan gelöst und mit Natriumsulfat getrocknet. Anschließend wurde die Lösung zur Trockene eingedampft, und es wurden 33 g 3,4-Dimethoxybenzoylessigsäureethylester als gelbes Öl erhalten.
C) 33 g des vorstehend erhaltenen Produktes wurden in 250 ml Ethanol gegeben und dem Reaktionsgemisch wurden unter Eiskühlung tropfenweise 10 ml Hydrazinhydrat zugesetzt. Nach 16-stündigem Stehenlassen bei Raumtemperatur wurde das kristallisierte Reaktionsprodukt abfiltriert, mit Isopropanol gewaschen und getrocknet. Es wurden 24 g 5-(3,4-Dimethoxyphenyl)-pyrazolin-3-on erhalten.
D) 29 ml 1-Brom-3-chlorpropan wurden in 300 ml Dimethylformamid gelöst und zu der Lösung wurden 24 g Kaliumcarbonat zugefügt. Zu dem Reaktionsgemisch wurde eine Lösung von 18 g 2-(3,4-Dimethoxyphenyl)-ethyl-N-methyl-amin in 50 ml Dimethylformamid unter Rühren innerhalb von 2 Stunden zugetropft. Anschließend wurde das Reaktionsgemisch noch eine Stunde gerührt. Dann wurden die gebildeten Salze abfiltriert. Das Filtrat wurde bei einer Badtemperatur von max. 50 °C eingeengt und der Rückstand wurde in 200 ml 0,5 m Zitronensäurelösung gelöst und die Lösung wurde zur Entfernung von unumgesetztem Bromchlorpropan mit tert.-Butylmethylether extrahiert. Die wäßrige Phase wurde sodann durch Zugabe von Natriumhydrogencarbonat leicht alkalisch eingestellt und mehrmals mit Essigsäureethylester extrahiert. Anschließend wurden die vereinigten Essigsäureethylesterextrakte mit Magnesiumsulfat getrocknet und eingeengt. Als Rückstand erhielt man 21 g 3-[N-(2-(3,4-Dimethoxyphenyl)ethyl)-N-methylamino]-propylchlorid.
E) 17 g 5-(3,4-Dimethoxyphenyl)-pyrazolin-3-on wurden in 200 ml Dimethylformamid gelöst. Der Lösung wurden 11 g Kaliumcarbonat zugesetzt und das Reaktionsgemisch wurde 0,5 Stunden bei 100 °C unter Stickstoffatmosphäre erhitzt. Dann wurde eine Lösung von 21 g 3-[N-(2-(3,4-Di-methoxyphenyl)-ethyl)-N-methylamino]-propylchlorid in 150 ml Dimethylformamid zugetropft. Nach 2 Stunden wurden die gebildeten Salze abfiltriert, das Filtrat bis zur Trockne eingeengt und der Rückstand in Essigsäureethylester gelöst. Die Lösung wurde anschließend zur Entfernung von nicht umgesetztem 5-(3,4-Dimethoxyphenyl)-pyrazolin-3-on mit 7 %-iger, wäßriger Natriumhydroxidlösung gewaschen, mit Natriumsulfat getrocknet und zur Trockne eingeengt. Als Rückstand verblieben 35 g öliges Rohprodukt. Dieses wurde durch Chromatographie über feinkörniges Kieselgel unter leicht erhöhtem Druck (Flash-Chromatographie) unter Verwendung von tert.-Butylmethylether/ Methanol 10:1 als Elutionsmittel gereinigt. Es wurden 22,3 g der Titelverbindung als Öl erhalten. IR-Spektrum der Base (als Film) : 1513 cm⁻¹, 1260 cm⁻¹. 1236 cm⁻¹.

Zur Salzbildung wurden 2,6 g der vorstehend erhaltenen öligen Titelverbindung in Isopropanol gelöst, dieser Lösung 3,7 ml einer isopropanolischen 2n-Salzsäurelösung zugegeben und das Reaktionsgemisch unter vermindertem Druck bis zur Trockne eingeengt. Das als Rückstand verbliebenen Salz wurde aus Isopropanol umkristallisiert und bei 80 °C im Hochvakuum getrocknet. Es wurden 2,1 g 3-{3-[N-(2-(3,4-Dimethoxyphenyl)-ethyl)-N-methylamino]-propyloxy)-5-(3,4-dimethoxyphenyl)-pyrazol-hydrochlorid mit einem Schmelzpunkt von 189 bis 192 °C erhalten.

### Beispiel 2:

### 3-{3-[N-(2-(3,4-Dimethoxyphenyl)-ethyl)-N-methylamino]-propyloxy}-5-(3,4-dimethoxyphenyl)-pyrazol

1,31 g Triphenylphosphin wurden in 15 ml Tetrahydrofuran gelöst. Zu der Lösung wurden 1,6 ml einer Lösung von 0,8 ml Azodicarbonsäureethylester in 0,8 ml Toluol bei einer Temperatur von 5 °C zugetropft. Nach 5 Minuten wurde eine Lösung von 1,1 g 5-(3,4-Dimethoxyphenyl)-pyrazolin-3-on in 10 ml Dimethylformamid zugetropft. Nach 20 Minuten wurde eine Lösung von 1,26 g 3-[N-(2-(3,4-Dimethoxyphenyl) -ethyl)-N-methylamino]-propanol in 10 ml Tetrahydrofuran zugetropft. Das Reaktionsgemisch wurde anschließend 48 Stunden bei 20 °C gerührt. Dann wurde das Reaktionsgemisch zur Trockne eingedampft. Das erhaltene Rohprodukt wurde durch Flash-Chromatographie nach der im Beispiel 1 E) beschriebenen Methode gereinigt. Es wurden 1,14 g der Titelverbindung als ölige Base erhalten. Diese wurde wie in Beispiel 1 E) beschrieben in das Hydrochlorid mit einem Schmelzpunkt von 189 bis 192 °C überführt.

### Beispiel 3:

### 3-{[N-(2-(3,4-Dimethoxyphenyl)-ethyl)-piperidin-3-yl]-methoxy}-5-(3,4-dimethoxyphenyl)-pyrazol.

A) Ein Gemisch aus 5,75 g 3-(Hydroxymethyl)-piperidin, 13,8 ml Triethylamin und 10 g 2-(3,4-Dimethoxyphenyl)-ethylchlorid wurde 4 Stunden unter Rückfluß erhitzt. Nach dem Abkühlen wurde das Reaktionsgemisch mit ca. 30 ml wäßriger 2n Salzsäurelösung angesäuert und nicht umgesetztes 2-(3,4-Dimethoxyphenyl)-ethylchlorid wurde durch Extraktion mit tert.-Butylmethylether abgetrennt. Dann wurde die wäßrige Phase durch Zugabe von Natriumhydroxidlösung alkalisch gestellt und mit Dichlormethan extrahiert. Die organische Phase wurde abgetrennt, getrocknet und eingeengt. Es wurden 5,3 g N-[2-(3,4-Dimethoxyphenyl)-ethyl]-3-(hydroxymethyl)-piperidin erhalten.
B) 5,3 g des vorstehend erhaltenenen Produktes wurden in 70 ml Dichlormethan, welchem 0,1 ml Dimethylformamid zugesetzt worden waren, gelöst und der Lösung wurden 5 ml Thionylchlorid zugesetzt. Das Reaktionsgemisch wurde 12 Stunden bei Raumtemperatur gerührt, eingeengt und 2-mal mit Toluol abgeraucht. Der verbleibende Rückstand wurde in einem Gemisch aus gesättigter Natriumcarbonatlö-sung und Essigsäureethylester gelöst. Die organische Phase wurde abgetrennt, mit Natriumsulfat getrocknet und eingedampft. Als Rückstand wurden 6,6 g N-[2-(3,4-Dimethoxyphenyl)-ethyl]-3-(chlormethyl)-piperidin erhalten.
C) 6,6 g des vorstehend erhaltenen Produktes wurden mit 4,4 g 5-(3,4 Dimethoxyphenyl)-pyrazolin-3-on nach der in Beispiel 1 E) beschriebenen Methode umgesetzt. Die erhaltene rohe Titelverbindung wurde wie in Beispiel 1 E) beschrieben gereinigt. Es wurden 4,1 g der Titelverbindung als ölige Base erhalten.

Zur Salzbildung wurden 4,1 g der Titelverbindung in Ethanol gelöst und die Lösung mit 1,07 g Oxalsäure in Ethanol versetzt. Es wurden 3,4 g 3-{[N-(2-(3,4-Dimethoxyphenyl)-ethyl)-piperidin-3-yl]-methoxy)-3,4-dimethoxyphenyl)-pyrazol-monohydrogenoxalat mit einem Schmelzpunkt von 147 bis 150 °C erhalten.

### Beispiel 4:

### 3-{3-[N-(2-(3-Methylphenyl)-ethyl)-N-methylamino]-propyloxy}-2-methyl-5-(2-fluorphenyl)-pyrazol.

A) Zu einer Lösung von 100 g 2-Fluorbenzoylessigsäureethylester in 400 ml Ethanol wurden unter Eiskühlung 32 g Methylhydrazin zugetropft. Zur Vervollständigung der Reaktion wurde das Reaktionsgemisch 2 Tage bei Raumtemperatur stehengelassen. Sodann wurde das kristallin ausgefallene 2-Methyl-5-(2-fluorphenyl)-pyrazolin-3-on abfiltriert.
B) 5 g des vorstehend erhaltenen Produktes wurden in 60 ml Dimethylformamid gelöst. Der Lösung wurden 0,86 Natriumhydrid (als 80 %-ige Lösung in Paraffin) zugesetzt und das Reaktionsgemisch wurde 1 Stunde bei 80 °C gerührt. Dann wurde auf Raumtemperatur abgekühlt, 4,1 g 1-Brom-3-chlorpropan zugesetzt und das Reaktionsgemisch 12 Stunden bei Raumtemperatur gerührt. Anschließend wurde das Reaktionsgemisch eingeengt, der Rückstand in Wasser aufgenommen und mit Diethylether extrahiert. Die etherische Lösung wurde abgetrennt und durch Flash-Chromatographie über Kieselgel gereinigt. Es wurden 4,8 g 3-(3-Chlorpropyloxy)-2-methyl-5-(2-fluorphenyl)-pyrazol erhalten.
C) 2,4 g des vorstehend erhaltenen Produktes und 1,5 g N-Methyl-2- (3-methylphenyl) -ethylamin wurden in 75 ml Dimethylformamid gelöst. Zu der Lösung wurden 2,5 g feingemahlenes, wasserfreies Kaliumcarbonat gegeben. Das Reaktionsgemisch wurde 11 Stunden bei einer Badtemperatur von 100 °C gerührt. Anschließend wurde das Gemisch eingeengt, der Rückstand mit Wasser aufgenommen und das Reaktionsprodukt mit Essigsäureethylester extrahiert. Die organische Phase wurde abgetrennt und 2-mal mit wäßriger 2m Zitronensäurelösung extrahiert. Anschließend wurde die wäßrige zitronensaure Lösung des Produktes durch Zugabe von Natriumcarbonat alkalisch gestellt und das Reaktionsprodukt mit Essigsäureethylester extrahiert. Die Essigsäureethylesterphase wurde mit Magnesiumsulfat getrocknet und eingeengt. Es wurden 1,0 g der Titelverbindung als ölige Base erhalten. IR-Spektrum der Base (als Film) : 1555 cm⁻¹, 1508 cm⁻¹, 1464 cm⁻¹.

Zur Salzbildung wurden 0,9 g der Titelverbindung in Ethanol gelöst, der Lösung eine molare Menge L-(+)-Weinsäure zugesetzt und eingeengt. Es wurden 1,25 g 3-{3-[N-(2-(3-Methyl-phenyl)-ethyl)-N-methylamino]-propyloxy}-2-methyl-5-(2-fluorphenyl)pyrazol x 1,1-hydrogentartrat als amorpher Feststoff erhalten.

### Beispiel 5:

### 3-{3-[N-(2-(4-Nitrophenyl)-ethyl)-N-methylamino]-propyloxy}-5-(2-fluorphenyl)-2-methyl-pyrazol

A) 260 ml 1-Brom-3-chlorpropan wurden in 400 ml Aceton gelöst. Zu der Lösung wurden 230 g Kaliumcarbonat gegeben. Zu dem Reaktionsgemisch wurde unter Rühren eine Lösung von 115 ml N-Methyl-N-benzylamin in 115 ml Aceton über einen Zeitraum von 30 Minuten verteilt bei einer Badtemperaratur von 40 °C zugetropft. Anschließend wurde das Reaktionsgemisch 4 Stunden bei einer Temperatur von 40 °C gerührt. Dann wurden die ausgefallenen Salze abfiltriert. Das Filtrat wurde bei einer Temperatur von 40 °C unter vermindertem Druck eingeengt. Der Rückstand wurde mit 16 %-iger wäßriger Salzsäurelösung bis zum Erreichen von pH 1 angesäuert und 3-mal mit tert.-Butylmethylether zum Entfernen von unumgesetzten 1-Brom-3-chlorpropan gewaschen. Anschließend wurde die wäßrige Phase durch Zugabe von 16 %-iger Natriumhydroxidlösung auf pH 8 gebracht und mehrfach mit Essigsäurethylester extrahiert. Die vereinigten Essigsäureethylester-Phasen wurden mit Magnesiumsulfat getrocknet und eingeengt. Es wurden 71 g 3-(N-Benzyl-N-methylamino)-propylchlorid erhalten.
B) 3,3 g 2-Methyl-5-(2-fluorphenyl)-pyrazolin-3-on wurden in 50 ml 1,3-Dimethyl-3,4,5,6-tetrahydro-2(1H)-pyrimidon gelöst. Der Lösung wurden 0,55 g Natriumhydrid (als 80 %-ige Lösung in Paraffin) zugesetzt und das Reaktionsgemisch wurde 30 Minuten bei 80 °C gerührt. Zu dem Reaktionsgemisch wurden 3,3 g 3-(N-benzyl-N-methylamino)-propylchlorid gegeben und weitere 2 Stunden bei 80 °C gerührt. Nach dem Abkühlen wurden dem Reaktionsgemisch 10 ml einer isopropanolischen 2n Salzsäurelösung zugesetzt und das Gemisch dann in 2 Liter eines Gemisches aus Aceton und tert.-Butylmethylether 1:3 eingetropft. Der gebildete Niederschlag wurde abfiltriert und in einem Gemisch aus gesättigter wäßriger Natriumcarbonatlösung und Essigsäureethylester gelöst. Die organische Phase wurde abgetrennt und die wäßrige Phase mehrfach mit Essigsäureethylester nachextrahiert. Die vereinigten Essigsäureethylesterextrakte wurden mit Natriumsulfat getrocknet und eingedampft. Die erhaltene rohe Base wurde durch Chromatographie an Kieselgel gereinigt. Es wurden 4,4 g 3-[3-(N-benzyl-N-methylamino)-propyloxy]-2-methyl-5-(2-fluorphenyl)-pyrazol erhalten.
C) 4,4 g des vorstehende erhaltenen Produktes wurden in wäßrigem Methanol gelöst. Die Lösung wurde mit Salzsäure angesäuert. Dann wurde eine katalytische Menge Palladium auf Kohle zugegeben und das Reaktionsgemisch mit Wasserstoff bei einem Druck von 6 bar 24 Stunden bei Raumtemperatur hydriert. Anschließend wurde der Katalysator abfiltriert, das Filtrat unter vermindertem Druck eingedampft. Der verbleibende Rückstand wurde in einem Gemisch aus gesättigter wäßriger Natriumcarbonatlösung und Essigsäureethylester gelöst. Die organische Phase wurde abgetrennt und die wäßrige Phase wurde mehrfach mit Essigsäureethylester nachextrahiert. Die vereinigten Essigsäureethylesterphasen wurden getrocknet und eingedampft. Es wurden 3,2 g 3-[3-(N-methylamino)-propyloxy]-2-methyl-5-(2-fluorphenyl)-pyrazol erhalten.
D) 3,1 g des vorstehend erhaltenen Produktes wurden in 30 ml 1,3-Dimethyl-3,4,5,6-tetrahydro-2(1H)-pyrimidon gelöst. Der Lösung wurden 3 g Kaliumcarbonat zugefügt und das Reaktionsgemisch wurde 30 Minuten bei einer Temperatur von 80 °C gerührt. Dann wurden 2,5 g 2-(4-Nitrophenyl)-ethylbromid zugesetzt, das Reaktionsgemisch 5 Stunden bei 50 °C gerührt, dann nochmals 0,25 g 2-(4-Nitrophenyl)-ethylbromid zugesetzt und weitere 13 Stunden bei 80 °C gerührt. Anschließend wurde das Reaktionsgemisch in der in Beispiel 5 B) beschriebenen Weise aufgearbeitet. Es wurden 2,6 g der Titelverbindung als ölige Base erhalten. IR-Spektrum (als Film): 1555 cm⁻¹, 1515 cm⁻¹, 1345 cm⁻¹.

Die Titelverbindung wurde anschließend wie in Beispiel 4 C) beschrieben in ihr Hydrogentartrat überführt. Es wurden 3,54 g des Monohydrogentartrates der Titelverbindung als amorpher Feststoff erhalten.

### Beispiel 6:

### 3-{3-[N-(3,4-Dimethylbenzyl)-N-methylamino]-2-hydroxypropyloxy}-5-(2-fluorphenyl)-2-methylpyrazol.

A) 50 g 3,4-Dimethylbenzoesäure wurden mit 78 g Thionylchlorid versetzt und das Reaktionsgemisch 3 Stunden am Rückfluß erhitzt. Anschließend wurde das Reaktionsgemisch eingeengt und der Rückstand noch zweimal in Toluol aufgenommen und wiederum eingeengt. Es wurden 53 g 3,4-Dimethylbenzoylchlorid erhalten.
B) 108 ml einer 40 %-igen wäßrigen Lösung von Methylamin wurden in 300 ml Tetrahydrofuran gelöst. Zu dieser Lösung wurden 53 g 3,4-Dimethylbenzoylchlorid unter Eiskühlung zugetropft. Anschließend wurde das Reaktionsgemisch 1 Stunde bei Raumtemperatur gerührt. Dann wurde das Reaktionsgemisch unter vermindertem Druck auf das halbe Volumen eingeengt, mit gesättigter Natriumchloridlösung versetzt und mit konzentrierter Salzsäure angesäuert. Das dabei ausgefallene Produkt wurde abgesaugt und im Trockenschrank bei 40 °C über Kaliumhydroxid getrocknet. Es wurden 47 g N-Methyl-3,4-dimethylbenzamid erhalten.
C) 6,4 g Lithiumaluminiumhydrid wurden in 200 ml Tetrahydrofuran gegeben und zum Sieden erhitzt. Innerhalb einer Stunde wurde eine Lösung von 25 g des unter B) hergestellten Amids in Tetrahydrofuran zugetropft und das Reaktionsgemisch weitere 3 Stunden am Rückfluß erhitzt. Anschließend wurde das Reaktionsgemisch zur Entfernung von überschüssigem Lithiumaluminiumhydrid mit wäßriger Natriumhydroxydlösung zersetzt und mit Wasser und Essigsäureethylester verdünnt. Die ausgefallenen Salze wurden abfiltriert, die organische Phase abgetrennt, über Magnesiumsulfat getrocknet und eingeengt. Es wurden 22,55 g N-Methyl-(3,4-dimethylbenzyl)-amin erhalten.
D) 10 g des vorstehend erhaltenen Produktes und 0,2 ml Wasser wurden bei einer Temperatur von 28 bis 30 °C tropfenweise zu 5,25 ml Epichlorhydrin gegeben. Das Reaktionsgemisch wurde 5 Stunden bei ca. 30 °C gerührt. Nach Abkühlung auf Raumtemperatur wurde dem Reaktionsgemisch tropfenweise 60 %-ige wäßrige Natriumhydroxidlösung zugegeben und anschließend weitere 40 Minuten gerührt. Zur Aufarbeitung wurde das Reaktionsgemisch in 26 ml Wasser gegossen. Das sich abscheidende Öl wurde abgetrennt und mit Kaliumhydroxyd getrocknet. Die wäßrige Phase wurde mit Ethylacetat extrahiert, der Ethylacetatextrakt getrocknet, eingeengt und das als Rückstand verbleibende Öl mit der als Öl abgeschiedenen Hauptmenge des Produktes vereinigt. Es wurden insgesamt 11,3 g 2-[-N-(3,4-Dimethylbenzyl)-N-methyl-aminomethyl]-oxiran erhalten.
E) 500 mg 5-(2-Fluorphenyl)-2-methylpyrazol-3-on wurden mit 360 mg wasserfreiem Kaliumcarbonat in 10 ml Dimethylformamid bei einer Temperatur von 100 °C 15 Minuten gerührt. Nach Abkühlung des Reaktionsgemisches auf 60 °C wurden 535 mg des unter D) erhaltenen Oxirans zugegeben. Das Reaktionsgemisch wurde anschließend 8 Stunden bei 100 °C gerührt. Zur Aufarbeitung wurde das Reaktionsgemisch eingeengt, der Rückstand in Wasser aufgenommen und mit Essigsäureethylester extrahiert. Die organische Phase wurde abgetrennt und eingeengt. Das als Rückstand verbliebene Rohprodukt wurde mittels Flash-Chromatographie gereinigt. Es wurden 0,11 g der Titelverbindung als ölige Base erhalten.
   IR-Spektrum (als Film): 1556 cm⁻¹, 1462 cm⁻¹, 754 cm⁻¹.
Zur Salzbildung wurden 320 mg der vorstehend erhaltenen Titelbase in einem Gemisch aus Ethanol/Essigsäureethylester 1:1 gelöst und der Lösung eine molare Menge Fumarsäure zugesetzt. Das Reaktionsgemisch wurde zur Trockne eingeengt und das erhaltene Salz bei 50 °C getrocknet. Es wurden 540 mg 3-{3-[N-(3,4-Dimethylbenzyl)-N-methylamino]-2-hydroxypropyloxy}-5-(2-fluorphenyl)-2-methylpyrazol-monohydrogenfumarat als amorpher Feststoff erhalten.

### Beispiel 7:

### 3-{2-[N-(2-(3,4-Dimethoxyphenyl)-ethyl)-N-methylamino]-1,1-dimethylethoxy}-5-(3,4-dimethoxyphenyl)-pyrazol

A) 8,8 g 5-(3,4-Dimethoxyphenyl)-pyrazolin-3-on wurden in 50 ml Dimethylformamid gelöst und mit 5,5 g Kaliumcarbonat versetzt. Das Reaktionsgemisch wurde unter Stickstoff 0,5 Stunden auf 100 °C erhitzt. Anschließend wurden bei 80 °C 8,9 g 2-Brom-2-methylpropionsäureethylester zugetropft. Es wurde 1 Stunde bei 80 °C gerührt, abgekühlt und die ausgefallenen Salze abfiltriert. Das Filtrat wurde im Vakuum eingeengt und der Rückstand in tert.-Butylmethylether gelöst. Nach dem Abfiltrieren unlöslicher Anteile wurde mit gesättigter Kochsalzlösung gewaschen und eingeengt. Es wurden 10 g 3-(2-Ethoxycarbonylpropyl-2-oxy)-5-(3,4-dimethoxyphenyl)-pyrazol als Öl erhalten.
B) 10 g des vorstehend erhaltenen Produktes wurden in einem Gemisch aus 20 ml 20-%iger Natronlauge und 40 ml Ethanol gelöst, und das Reaktionsgemisch wurde 2 Stunden unter Rückfluß erhitzt. Nach dem Abkühlen wurde mit konzentrierter Salzsäure auf pH 1 angesäuert und das Reaktionsgemisch mehrfach mit tert.-Butylmethylether extrahiert. Die vereinigten Extrakte wurden mit Magnesiumsulfat getrocknet und eingeengt. Es wurden 9 g 3-(2-Hydroxycarbonylpropyl-2-oxy)-5-(3,4-dimethoxyphenyl)-pyrazol erhalten.
C) 5 g des vorstehend erhaltenen Produktes wurden in Dichlormethan angeschlemmt, bei Raumtemperatur mit 4,5 ml Triethylamin versetzt, auf -30 °C abgekühlt und mit 1,28 ml Methansulfonsäurechlorid versetzt. Das Reaktionsgemisch wurde 1 h bei -30 °C gerührt, dann wurde eine Spatelspitze Pyrrolidinopyridin zugefügt und auf Raumtemperatur erwärmt. Nach 12 h wurde mit Dichlormethan verdünnt. Nach Entfernung der basischen Bestandteile durch Extraktion mit zitronensaurer Natriumchloridlösung wurde die organische Phase mit konzentrierter Sodalösung säurefrei gewaschen, mit Natriumsulfat getrocknet und eingeengt. Es wurden 3,7 g 2,2-Dimethyl-6-(3,4-dimethoxyphenyl) -pyrazolo(5,1)oxazol erhalten. Schmelzpunkt 120 bis 130 °C.
D) 3,5 g des vorstehend erhaltenen Produktes und 4,2 g N-Methyl-2-(3,4-dimethoxyphenyl)-ethylamin wurden in Diglyme gelöst und 2 h unter Stickstoff auf 150 °C erhitzt. Nach dem Abkühlen wurde mit Methyl-tert.-butyläther verdünnt, mit zitronensaurer Natriumchloridlösung basefrei und anschließend mit gesättigter Sodalösung säurefrei gewaschen. Die organische Phase wurde mit Magnesiumsulfat getrocknet und eingedampft. Es wurden 6 g 3-{2-[N-(2-(3,4-Dimethoxyphenyl)-ethyl)-N-methylaminocarbonyl]-propyl-2-oxy}-5-(3,4-dimethoxyphenyl)-pyrazol erhalten.
E) Eine Lösung von 6 g des vorstehend erhaltenen Produktes in trockenem Tetrahydrofuran wurde zu 20 ml einer siedenden 1-molaren Lösung von Lithiumaluminiumhydrid in Tetrahydrofuran getropft. Das Reaktionsgemisch wurde 2 Stunden am Rückfluß erhitzt. So dann wurden zur Aufarbeitung unter Eiskühlung nacheinander 0,57 ml Wasser, 0,75 ml 15 %-ige Natriumhydroxidlösung und nochmals 2,5 ml Wasser zugegeben. Das ausgefallene Aluminiumhydroxid wurde abfiltriert und das Filtrat eingeengt. Der Rückstand wurde in wäßriger 1m Zitronensäurelösung gelöst. Die Lösung wurde mit Essigsäureethylester gewaschen. Anschließend wurde die zitronensaure Lösung abgetrennt, durch Zugabe von wäßriger Natriumcarbonatlösung alkalisch gestellt und das Reaktionsprodukt mit Essigsäureethylester extrahiert. Die Essigsäureethylesterphase wurde getrocknet und eingedampft. Es wurden 4,1 g Rohprodukt erhalten, welches durch Chromatographie an Kieselgel gereinigt wurde. Es wurden 2,7 g der Titelverbindung als ölige Base erhalten.
   IR-Spektrum (als Film) : 1512 cm⁻¹, 1464 cm⁻¹, 1261 cm⁻¹. Die Titelverbindung wurde anschließend nach der in Beispiel 4 C beschriebenen Methode zur Salzbildung mit einer molaren Menge L-(+)-Weinsäure umgesetzt. Es wurden 3,56 g 3-{2-[N-(2-(3,4-Dimethoxyphenyl)-ethyl)-N-methylamino]-1,1-dimethylethoxy}-5-(3,4-dimethoxyphenyl)-pyrazol x 1,2-hydrogentartrat als amorpher Feststoff erhalten.

### Beispiel 8:

### 3-{6-[N-(2-(3,4-Dimethoxyphenyl)-ethyl)-N-methylamino]-hexyloxy}-5-(3,4-dimethoxyphenyl)-pyrazol

A) 10 ml 6-Bromhexanoylchlorid wurden in einem Gemisch aus 100 ml absolutem Dichlormethan und 100 ml absolutem Toluol gelöst. Zu der Lösung wurde unter Eiskühlung eine Lösung von 13, 6 g N-Methyl-N-[2-(3,4-dimethoxyphenyl) - ethyl] -amin und 9,5 ml Triethylamin in 25 ml absolutem Toluol zugetropft. Anschließend wurde das Reaktionsgemisch 12 Stunden bei Raumtemperatur gerührt. Zur Aufarbeitung wurden die ausgefallenen Salze abfiltriert und mit Toluol gewaschen. Das Filtrat wurde zunächst mit 1-molarer Zitronensäurelösung und dann mit Natriumbicarbonatlösung gewaschen, mit Magnesiumsulfat getrocknet und eingeengt. Es wurden 19,3 g 5-{N-[2-(3,4-Dimethoxyphenyl)-ethyl]-N-methylaminocarbonyl}-pentylbromid als Öl erhalten.
B) 3,85 g 5-(3,4-Dimethoxyphenyl)-pyrazolin-3-on wurden in 50 ml wasserfreiem Dimethylformamid gelöst. Zu der Lösung wurden 3,15 g fein gemahlenes Kaliumcarbonat gegeben und das Reaktionsgemisch unter Stickstoffatmosphäre unter Rühren auf 100 °C erhitzt. Zu dem Reaktionsgemisch wurde eine Lösung von 5,6 g 5-{N-[2-(3,4-Dimethoxyphenyl)-ethyl]-N-methylaminocarbonyl}-pentylbromid in 10 ml wasserfreiem Dimethylformamid langsam zugetropft und das Reaktionsgemisch weitere 2 Stunden bei 100 °C gerührt. Zur Aufarbeitung wurden nach dem Abkühlen die ausgefallenen Salze abfiltriert und das Filtrat eingedampft. Der verbleibende ölige Rückstand wurde in Essigsäureethylester gelöst und zur Entfernung von nichtumgesetztem 5-(3,4-Dimethoxyphenyl)-pyrazolin-3-on mit 7 %-iger Natriumhydroxydlösung gewaschen. Die organische Phase wurde abgetrennt mit Natriumsulfat getrocknet und eingedampft. Es wurden 6,5 g 3-{5-[N-(2-(3,4-Dimethyloxyphenyl)-ethyl)-N-methylaminocarbonyl]-pentyloxy}-5-(3,4-dimeth-oxyphenyl)-pyrazol als Öl erhalten.
C) 6,5 g des vorstehend erhaltenen Produktes wurden nach der in Beispiel 7 B) beschriebenen Methode mit Lithiumaluminiumhydrid reduziert. Das Reaktionsgemisch wurde anschließend nach der in Beispiel 7 E) beschriebenen Methode aufgearbeitet. Es wurden 2,5 g der Titelverbindung als ölige Base erhalten. IR-Spektrum (als Film) : 1509 cm⁻¹, 1465 cm⁻¹, 1261 cm⁻¹.
   Die Titelverbindung wurde anschließend nach der in Beispiel 4 C) beschriebenen Methode zur Salzbildung mit einer molaren Menge L-(+)-Weinsäure umgesetzt. Es wurden 3,25 g 3-{6-[N-(2-(3,4-Dimethoxyphenyl)-ethyl)-N-methylamino]-hexyloxy}-5-(3,4-dimethoxyphenyl)-pyrazol x 1,1-hydro-gen-t-artrat als amorpher Feststoff erhalten.

### Beispiel 9:

### 3-{2-[N-(2-(4-Chlorphenyl)-ethyl)-N-methylamino]-ethyloxy}-5-(4-hydroxyphenyl)-2-methylpyrazol

1,8 g 3-{2-[N-(2-(4-Chlorphenyl)-ethyl)-N-methylamino]-ethyloxy}-5(-4-methoxyphenyl)-2-methylpyrazol (siehe Beispiel 24) wurden in 100 ml Dichlormethan gelöst. Der Lösung wurden 10 ml einer 1-molaren Lösung von Bortribromid in Dichlormethan zugesetzt. Das Reaktionsgemisch wurde 12 Stunden stehengelassen, dann in Wasser gegeben und mit Dichlormethan extrahiert. Die wäßrige Phase wurde abgetrennt, alkalisch gestellt, dreimal mit einem Gemisch aus Dichlormethan und Methanol extrahiert und die vereinigten organischen Phasen wurden getrocknet und eingeengt. Die als Öl erhaltene rohe Titelverbindung wurde durch Chromatographie an Kieselgel unter Verwendung von Dichlormethan/Methanol gereinigt. Die gereinigte Titelverbindung wurde zur Salzbildung mit einer molaren Menge L-(+)-Weinsäure nach der in Beispiel 4 C) beschriebenen Methode umgesetzt und das erhaltene Salz wurde aus einem Gemisch aus Diethylether und Isopropanol kristallisiert. Es wurden 1,15 g 3-{2-[N-(2-(4-Chlorphenyl)-ethyl)-N-methylamino]-ethyloxy}-5-(4-hydroxyphenyl)-2-methylpyrazol x 1,3-hydrogentartrat mit einem Schmelzpunkt von 90 bis 91 °C erhalten.

### Beispiel 10:

### 3-{2-[N-(2-(4-Chlorphenyl)-ethyl)-N-methylamino]-ethyloxy}-5-(4-acetoxyphenyl)-2-methylpyrazol

100 mg 3-{2-[N-(2-(4-Chlorphenyl)-ethyl)-N-methylamino]-ethyloxy}-5-(4-hydroxyphenyl)-2-methylpyrazol (Herstellung siehe Beispiel 9) wurden in 2 ml Dichlormethan gelöst. Der Lösung wurden 0,15 ml Pyridin und 1 ml Acetanhydrid zugefügt und das Reaktionsgemisch wurde 24 Stunden bei Raumtemperatur gerührt. Anschließend wurde das Reaktionsgemisch unter vermindertem Druck eingedampft, der Rückstand in wäßriger Natriumbicarbonatlösung aufgenommen und mit Dichlormethan extrahiert. Die organische Phase wurde abgetrennt, getrocknet und eingeengt. Die als Rückstand verbleibende rohe Titelverbindung wurde in tert.-Butylmethylether aufgenommen. Zur Salzbildung wurde isopropanolische 2n Salzsäurelösung zugegeben. Das ausgefällte Salz wurde abgetrennt. Es wurden 80 mg des Hydrochlorids der Titelverbindung mit einem Schmelzpunkt von 160 bis 175 °C erhalten.

### Beispiel 11:

### 3-{3-[N-(2-(3,4-Dimethoxyphenyl)-ethyl)-amino]-propyloxy}-2-benzyl-5-(3,4-dimethoxyphenyl) -pyrazol

A) 6,6 g 3,4-Dimethoxybenzoylessigsäurethylester wurden in Ethanol angeschlämmt und unter Eiskühlung mit 3,5 g Benzylhydrazin tropfenweise während 1,5 Stunden versetzt. Das Reaktionsgemisch wurde 12 Stunden bei Raumtemperatur gerührt. Dann wurde das ausgefallene Produkt abfiltriert und zunächst mit Ethanol, dann mit tert.-Butylmethyläther gewaschen. Es wurden 4,36 g 5-(3,4-Dimethoxyphenyl)-2-benzylpyrazolin-3-on erhalten. Schmelzpunkt: 164 - 166 °C.
B) 5,5 g des vorstehenden Produktes wurden in 50 ml Dimethylformamid gelöst. Die Lösung wurde mit 0,5 g Natriumhydrid versetzt und 0,5 h bei 80 °C unter Stickstoff-Atmosphäre gerührt. Dann wurden bei Raumtemperatur 1,97 ml 1-Brom-3-chlorpropan zugetropft und das Reaktionsgemisch 14 Stunden gerührt. Dann wurde das Reaktionsgemisch unter vermindertem Druck eingedampft, mit tert.-Butylmethyläther aufgenommen und der unlösliche Rückstand abfiltriert. Das Filtrat wurde eingeengt und durch Chromatographie über feinkörniges Kieselgel unter leicht erhöhtem Druck (= Flash-Chromatographie) gereinigt. Man erhielt 7 g 3-(3-Chlor-propyloxy)-5-(3,4-dimethoxyphenyl)-2-benzyl-pyrazol.
C) 6 g des vorstehend erhaltenen Produktes und 7 g Homoveratrylamin wurden in 20 ml Dimethylformamid gelöst, mit 10 ml Triethylamin versetzt und 4 Stunden auf 120 °C erhitzt. Das Reaktionsgemisch wurde eingedampft, mit Essigsäureethylester aufgenommen und mit 2m Zitronensäur extrahiert. Anschließend wurde die wäßrige zitronensaure Lösung des Produktes durch Zugabe von Natriumhydrogencarbonat alkalisch gestellt und das Reaktionsprodukt mit Essigsäureethylester extrahiert. Der Extrakt wurde durch Chromatographie über feinkörniges Kieselgel unter leicht erhöhtem Druck (= Flash-Chromatographie) gereinigt. Man erhielt 4,9 g 3-(3-[N-(2-(3,4-Dimethoxyphenyl)-ethyl)-amino]-propyloxy}-2-benzyl-5-(3,4-dimethoxyphenyl)-pyrazol. IR-Spektrum der Base (als Film) : 1524 cm⁻¹, 1260 cm⁻¹, 1236 cm⁻¹.

### Beispiel 12:

### 3-{3-[N-(2-(3,4-Dimethoxyphenyl)-ethyl)-amino]-propyloxy}-5-(3,4-dimethoxyphenyl)-pyrazol

4,9 g 3-{3-[N-(2-(3,4-Dimethoxyphenyl)-ethyl)-amino]-propyloxy)-2-benzyl-5-(3,4-dimethoxyphenyl)-pyrazol wurden in Eisessig gelöst, mit 0,5 g Palladium auf Kohle versetzt und während 24 Stunden bei 80 bar Wasserstoffdruck und 80 °C hydriert. Nach Abfiltrieren des Katalysators wurde eingedampft, mit Natronlauge alkalisch gestellt (pH = 10) und das Produkt mit Essigsäureethylester extrahiert. Die organische Phase wurde abgetrennt, eingeengt und durch Flash-Chromatographie über Kieselgel gereinigt. Es wurden 1,1 g 3-{3-[N-(2-(3,4-Dimethoxyphenyl)-ethyl)-amino]-propyloxy}-5-(3,4-dimethoxyphenyl)-pyrazol erhalten.
IR-Spektrum der Base (als Film): 1511 cm⁻¹, 1465 cm⁻¹,
1262 cm⁻¹.
Zur Salzbildung wurden 1,1 g der Titelverbindung in Ethanol gelöst, der Lösung eine molare Menge L-(+)-Weinsäure zugesetzt und eingeengt. Es wurden 1,46 g 3-{3-[N-(2-(3,4-Dimethoxyphenyl) -ethyl)-amino]-propyloxy}-5-(3,4-dimethoxyphenyl)-pyrazolmonohydrogentartrat als amorpher Feststoff erhalten.

### Beispiel 13:

### 3-{3-[N-(2-(3,4-Dimethoxyphenyl)-ethyl)-N-methylamino]-propyloxy}-5-(3,4-dimethoxyphenyl)-2-benzyl-pyrazol

A) 5 g 5-(3,4-Dimethoxyphenyl)-pyrazolin-3-on (Herstellung siehe Beispiel 1C) wurden in 100 ml Toluol angeschlemmt. Zu der Anschlemmung wurden tropfenweise zunächst 3 ml Collidin und anschließend 3,6 ml Benzylbromid zugegeben. Nach 6-stündigem Erhitzen unter Rückfluß wurde abgekühlt, die ausgefallenen Kristalle abfiltriert und mit Isopropanol gewaschen. Die erhaltenen Kristalle wurden in Dichlormethan gelöst und mit 2 m Zitronensäure gewaschen. Die organische
   Phase wurde mit Magnesiumsulfat getrocknet und eingeengt. Zur Reinigung wurde aus Isopropanol umkristallisiert. Es wurden 4 g 5-(3,4-Dimethoxyphenyl) -2-benzyl-pyrazolin-3-on mit einem Schmelzpunkt von 165 °C erhalten.
B) 62 g des vorstehend erhaltenen Produktes wurden in 500 ml Dimethylformamid gelöst. Der Lösung wurden 31 g Kaliumcarbonat zugesetzt, und das Reaktionsgemisch wurde 0,5 Stunden bei 100 °C unter Stickstoff erhitzt. Nach dem Zutropfen einer Lösung von 56 g 3-[N-(2-(3,4-Dimethoxyphenyl)-ethyl)-N-methylamino]-propylchlorid in 70 ml Dimethylformamid wurde das Reaktionsgemisch weitere 4 Stunden bei 100 °C erhitzt. Die ausgefallenen Salze wurden abfiltriert und das Filtrat zur Trockene eingeengt. Der Rückstand wurde mit 350 ml 0,75 n Salzsäure aufgenommen und mit Essigsäureethylester extrahiert. Die wäßrige saure Phase wurde durch Zugabe von wäßriger Natronlauge alkalisiert und mit Essigsäureethylester extrahiert. Das nach dem Trocknen und Eindampfen der organischen Phase erhaltene Rohprodukt wurde durch Flash-Chromatographie eingeengt. Es wurden 68 g 3-{3-[N-(2-(3,4-Dimethoxyphenyl)-ethyl)-N-methylamino]-propyloxy}-5-(3,4-dimethoxyphenyl)-2-benzylpyrazol erhalten.
   IR-Spektrum der Base (als Film): 1524 cm⁻¹, 1260 cm⁻¹, 1236 cm⁻¹.
   Zu 7,1 g der in 100 ml Aceton gelösten Base wurden 1,63 g Oxalsäure-Dihydrat, gelöst in 20 ml Aceton zugetropft. Die ausgefallenen Kristalle wurden abfiltriert und getrocknet. Es wurden 7,7 g des Hydrogenoxalates des 3-{-3-[N-(2-(3,4-Dimethoxyphenyl)-ethyl)-N-methylamino]-propyloxy}-5-(3,4-dimethoxyphenyl)-2-benzyl-pyrazol mit einem Schmelzpunkt von 135 bis 137 °C erhalten.

### Beispiel 14:

### 3-{3-[N-(2-(3,4-Dimethoxyphenyl)-ethyl)-N-methylamino)-propyloxy}-5-(3,4-dimethoxyphenyl)-pyrazol

A) 2 g 3-{3-[N-(2-(3,4-dimethoxyphenyl)-ethyl)-N-methylamino]-propyloxy}-5-(3,4-dimethoxyphenyl)-2-benzyl-pyrazol (Herstellung siehe Beispiel 13) wurden in 10 ml Methanol gelöst und auf 2 g Palladium-Schwarz unter Stickstoff gegeben. Zu der unter Stickstoff gerührten Lösung wurden 5,5 ml 98 %-ige Ameisensäure hinzugefügt. Nach 3-stündigem Rühren unter Stickstoff wurde der Katalysator abfiltriert und das Filtrat eingedampft. Der ölige Rückstand wurde mit gesättigter Natriumcarbonatlösung aufgenommen und mit Essigsäureethylester mehrfach extrahiert. Die vereinigten organischen Phasen wurden mit Natriumsulfat getrocknet und eingedampft. Es wurden 1,6 g 3-{2-[N-(2-(3,4-Dimethoxyphenyl)-ethyl)-N-methylamino]-propyloxy}-5-(3,4-dimethoxyphenyl)-pyrazol als ölige Base erhalten. Diese wurde analog Beispiel 1 E) in das Hydrochlorid mit einem Schmelzpunkt von 189 bis 192 °C umgewandelt.

Nach den in den vorstehenden Beispielen beschriebenen Verfahren können auch die in der folgenden Tabelle aufgeführten Verbindungen der Formel I hergestellt werden. Die in der Tabelle in cm⁻¹ angeführten IR-Banden sind die charakteristischen Banden der IR-Spektren der jeweiligen freien Basen (sofern nicht anders angegeben als Film).

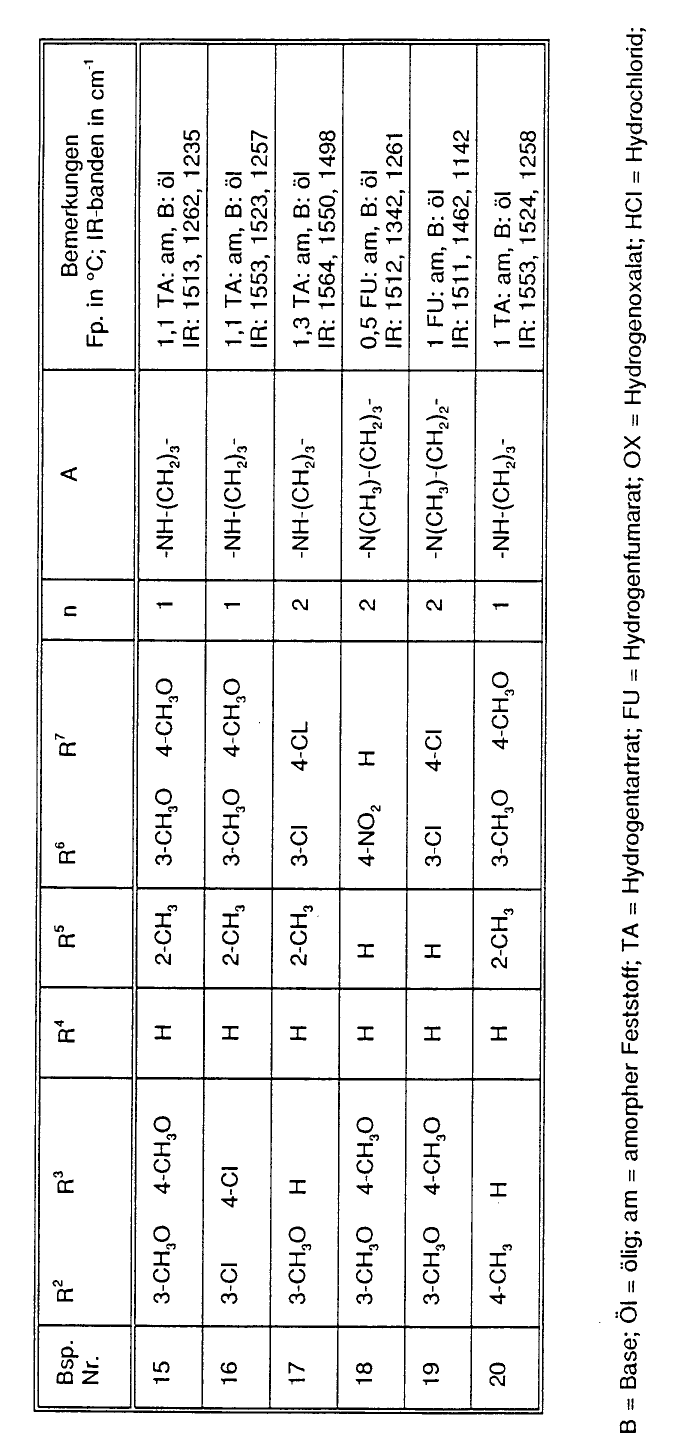

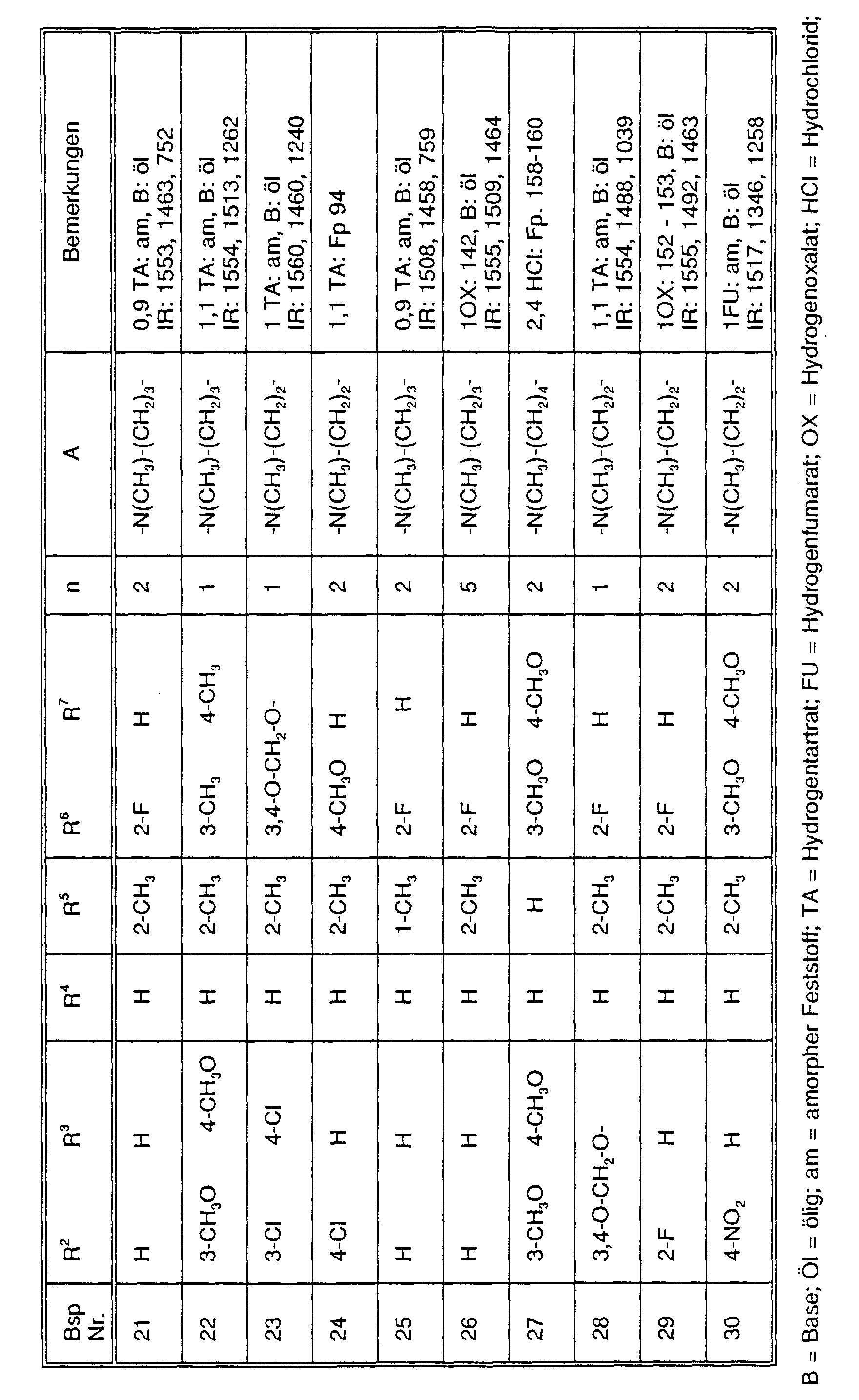

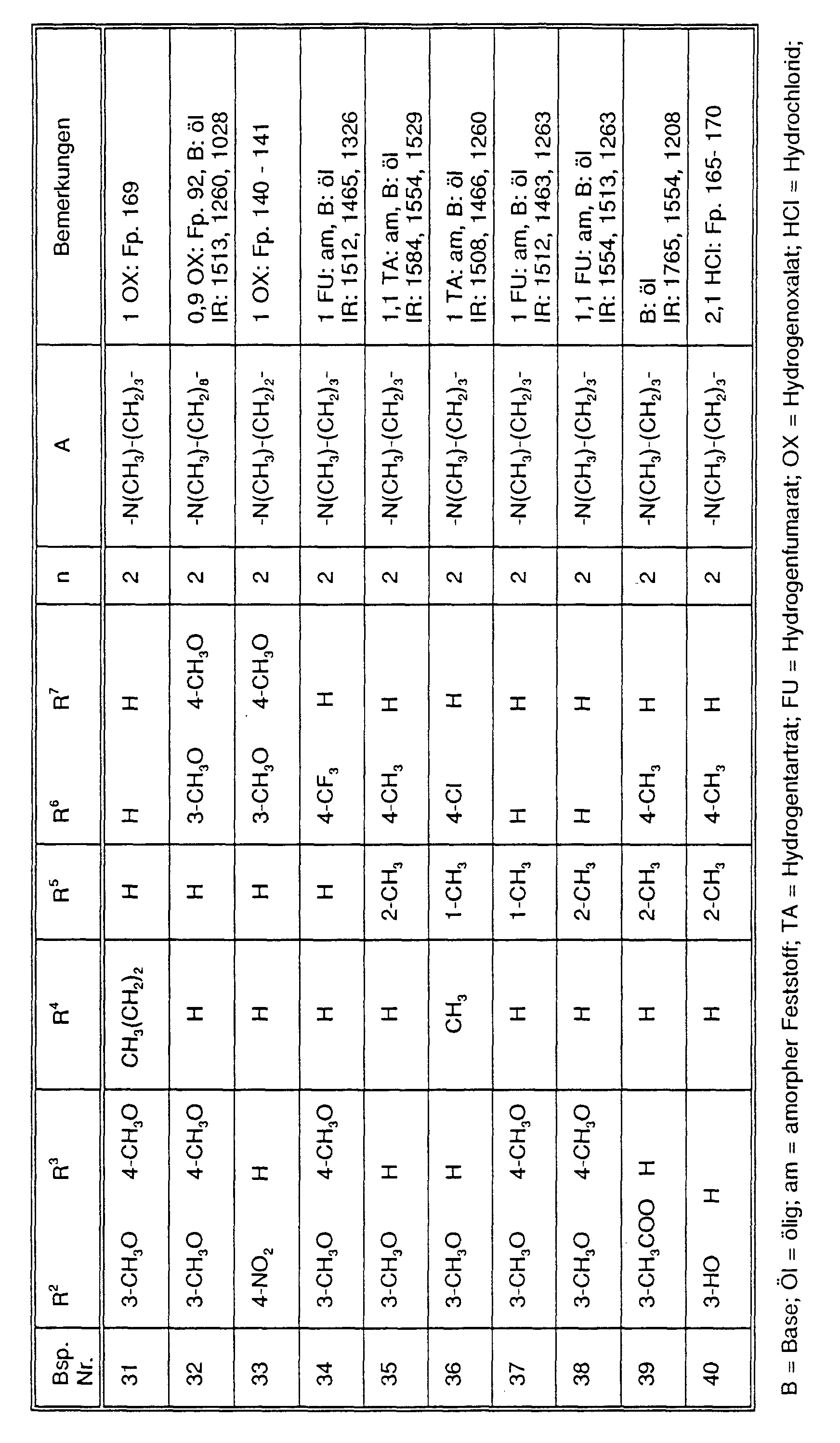

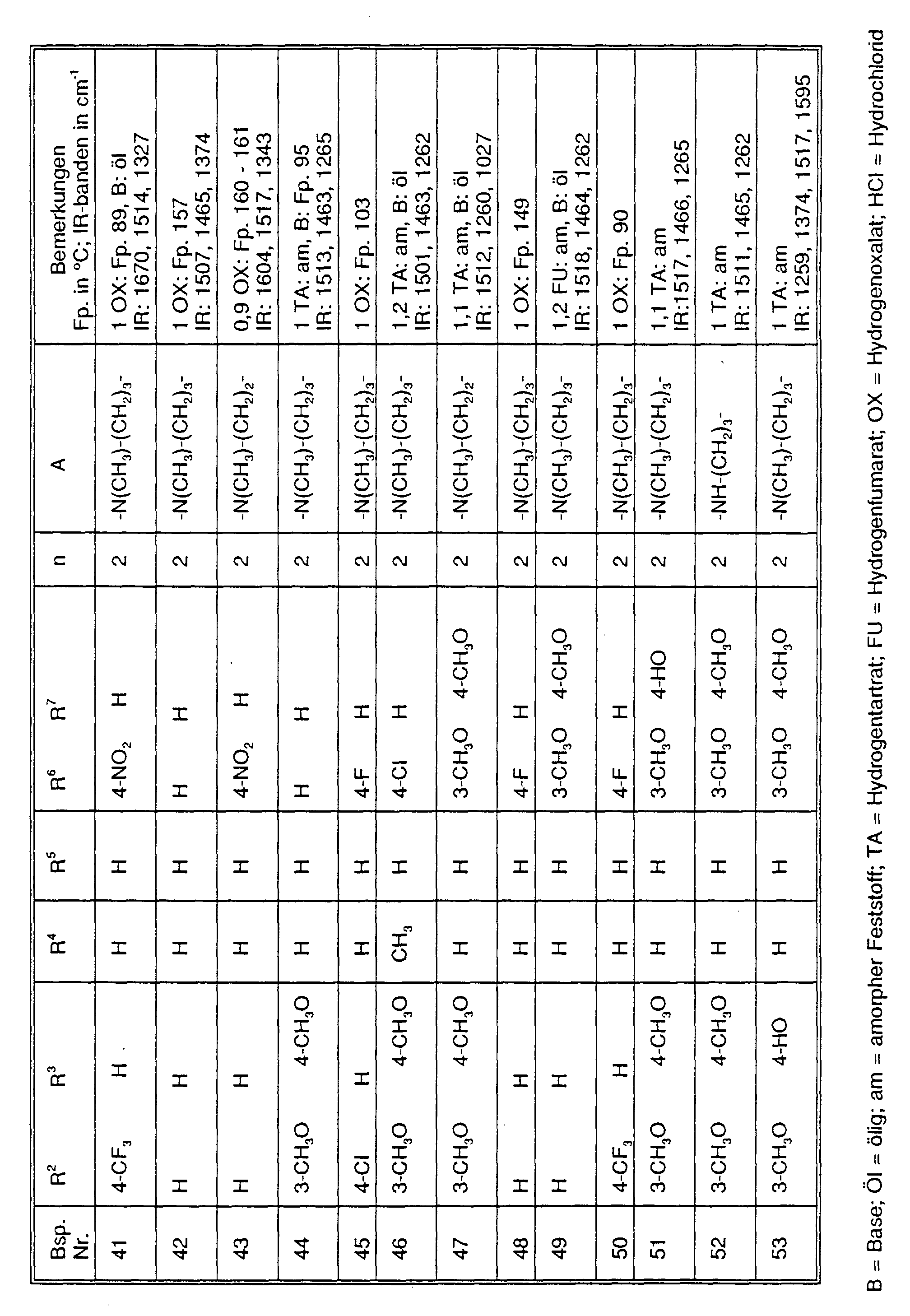

### Beispiel I:

### 3-{3-[N-(2-(3,4 Dimethoxyphenyl)-ethyl)-N-methylamino]-propyloxy}-5-(3,4-dimethoxyphenyl)-pyrazol-hydrochlorid enthaltende Tabletten

Man stellte Tabletten in folgender Zusammensetzung pro Tablette her:

| | |
|---|---|
| 3-{3-[N-(2-(3,4-Dimethoxyphenyl)-ethyl) -N-methylamino]-propyloxy}-5-(3,4-dimethoxyphenyl)-pyrazol-hydrochlorid | 20 mg |
| Maisstärke | 60 mg |
| Milchzucker | 135 mg |
| Gelatine (als 10 %-ige Lösung) | 6 mg |

Der Wirkstoff, die Maisstärke und der Milchzucker wurden mit der 10 %-igen Gelatine-Lösung eingedickt. Die Paste wurde zerkleinert, und das entstehende Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstereat | 5 mg |
| Maisstärke | 9 mg |

und sodann zu Tabletten von 240 mg verpreßt.

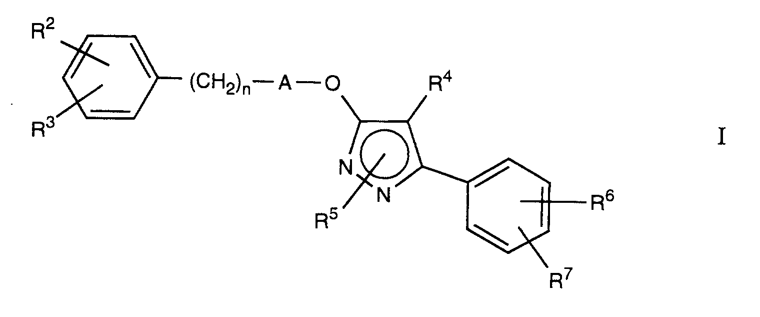

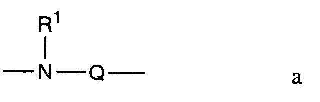

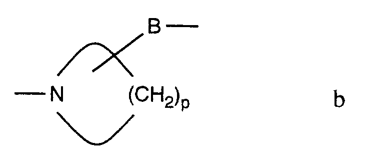

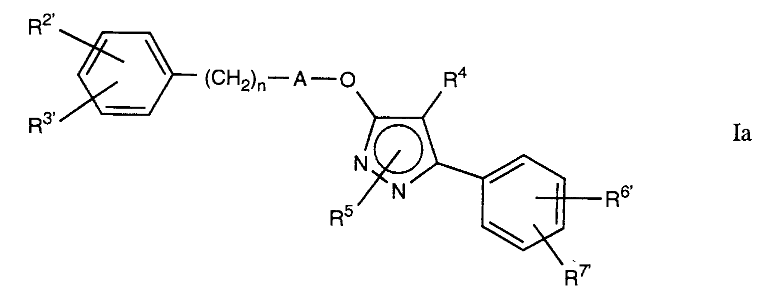

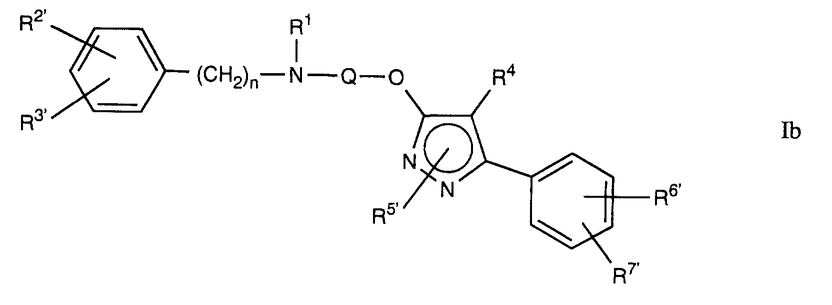

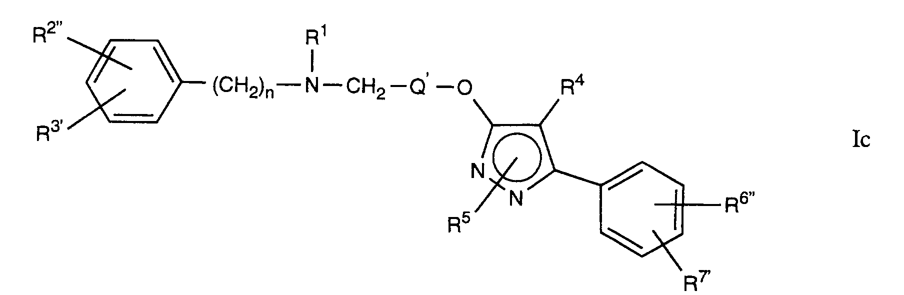

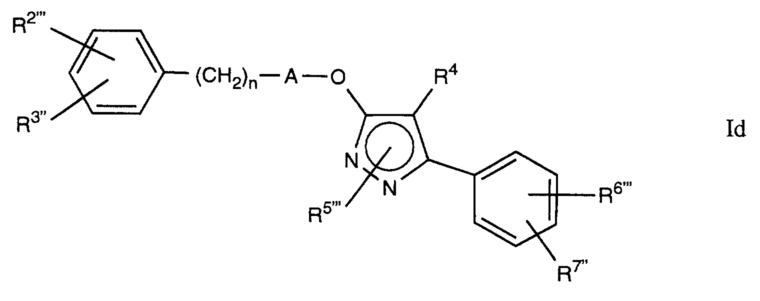

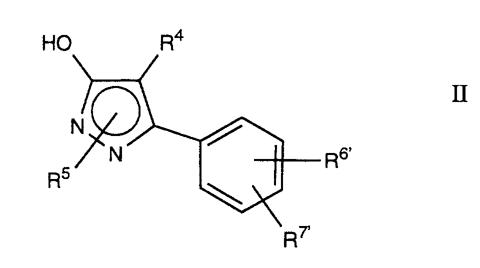

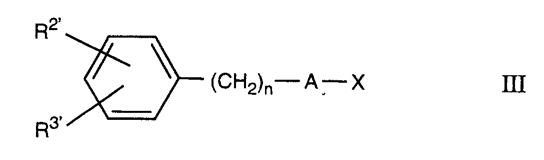

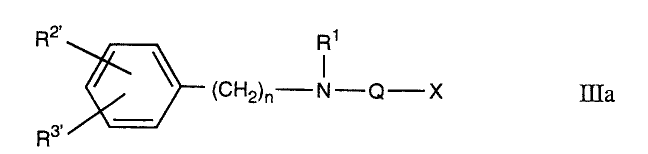

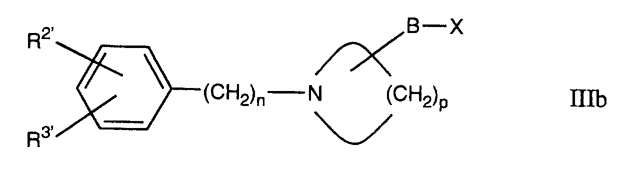

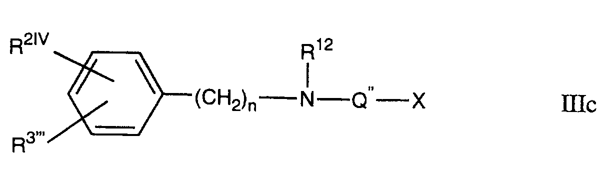

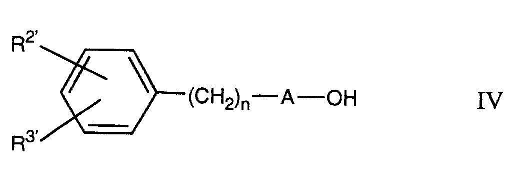

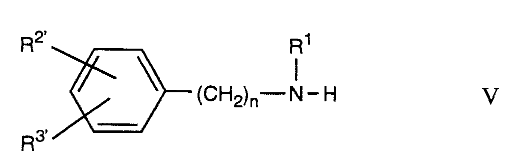

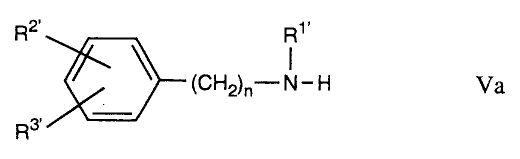

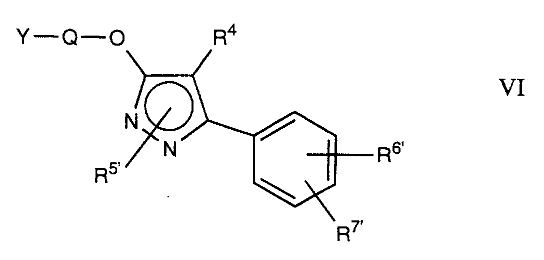

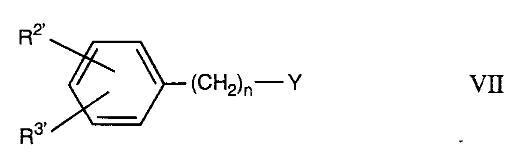

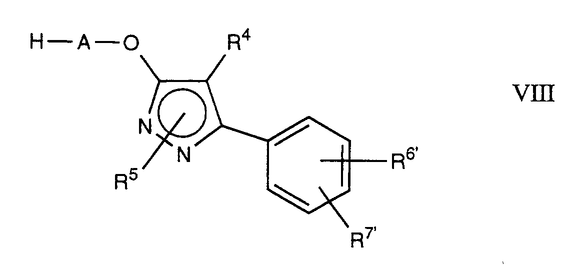

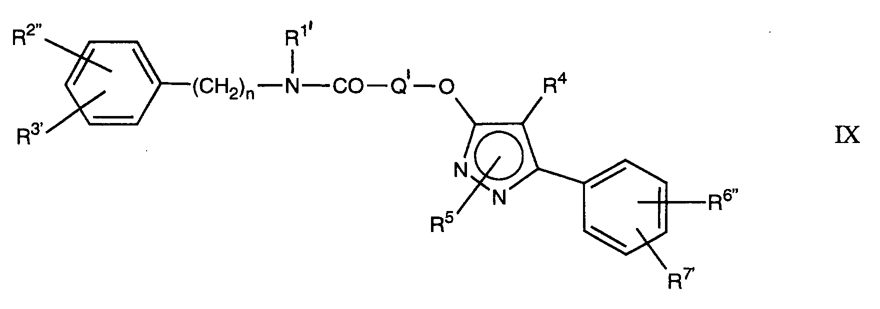

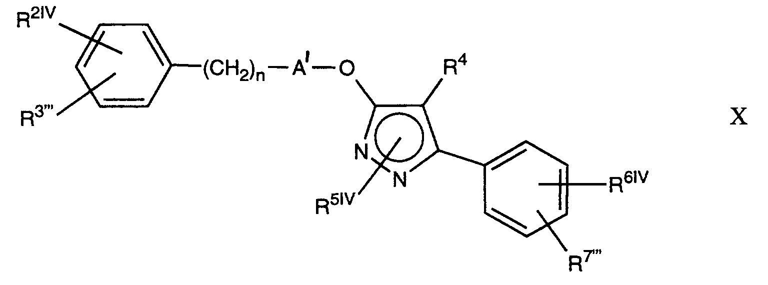

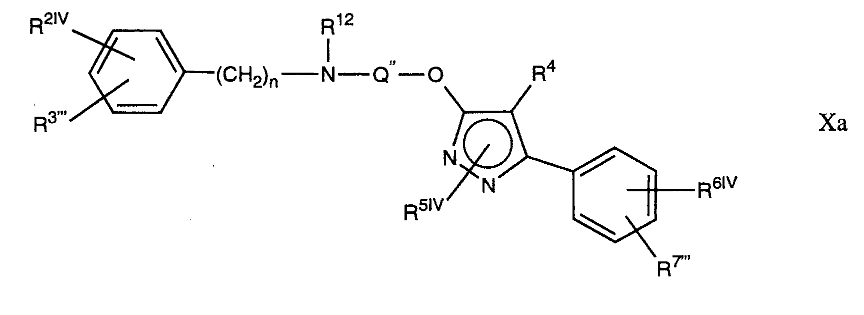

R¹―NH₂ xm XIII

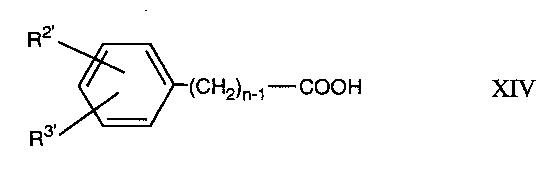

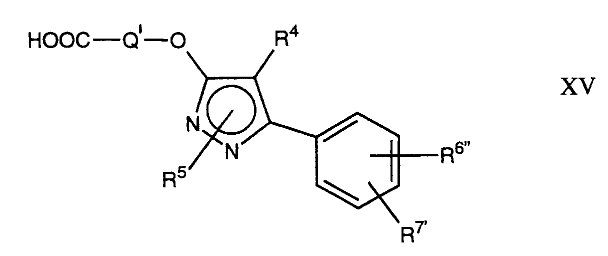

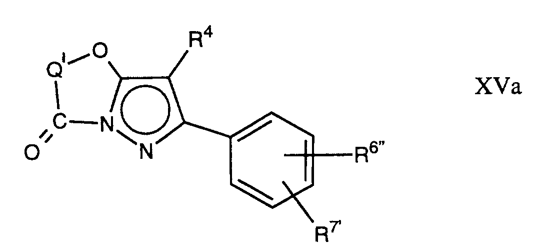

Hal―Q'―COOR¹² XVa

Hal―Q'―CN XVIb

X―Q'―COOH XVII

R¹⁰OOC―N=N―COOR¹¹ XVIII

H₂N―NHR⁵ XIX

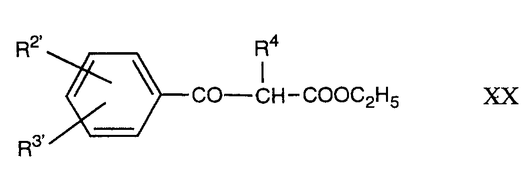

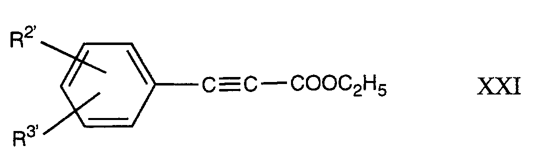

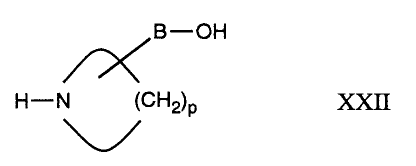

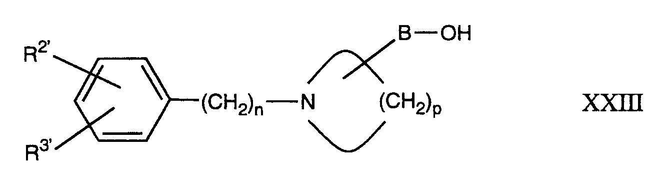

H―A―OH XXIV

D―A―X XXV

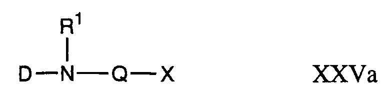

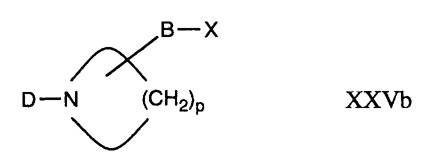

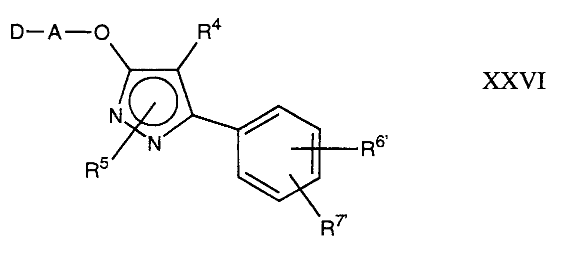

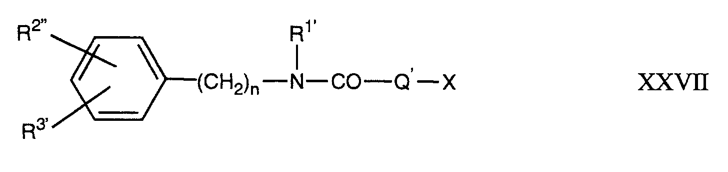

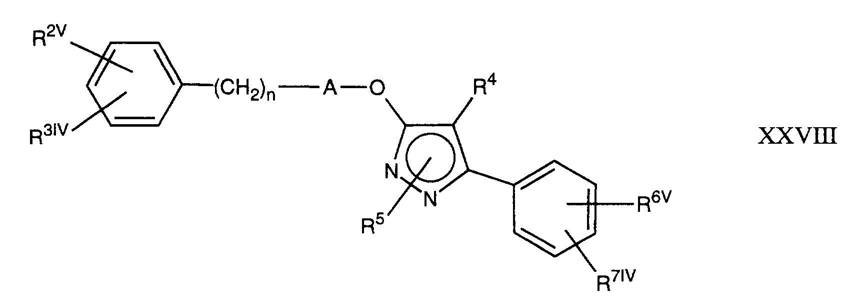

X―Q―T' XXIX

Y―Q―T XXX

## Patentansprüche

1. Verbindungen der allgemeinen Formel I 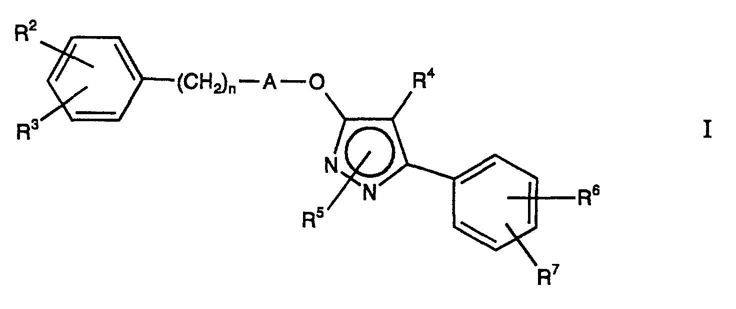 worin
n eine ganze Zahl von 1 bis 5 bedeutet,
A für eine Gruppe der allgemeinen Formel a 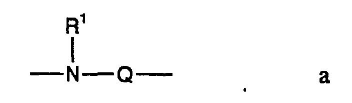 worin
R¹ Wasserstoff oder eine niedere Alkylgruppe bedeutet und Q eine (CH₂)ₘ-Gruppe, worin m 2 bis 8 bedeutet und welche gegebenenfalls in α-Stellung zu dem Sauerstoffatom durch 1 bis 2 niedere Alkylgruppen substituiert sein kann, steht, oder A für eine Gruppe der allgemeinen Formel b 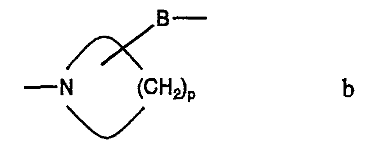
worin p 4 bis 6 bedeutet und B eine (CH₂)ᵣ-Gruppe, worin r 1 bis 3 bedeutet, darstellt, steht,
R² Wasserstoff, Halogen, niederes Alkyl, niederes Alkoxy, Hydroxy, Trifluormethyl oder Nitro oder, falls R³, R⁶ und R⁷ nicht Hydroxy sind, auch niederes Alkanoyloxy bedeutet und
R³ Wasserstoff, Halogen, niederes Alkyl, niederes Alkoxy, Hydroxy oder, falls R², R⁶ und R⁷ nicht Hydroxy sind, auch niederes Alkanoyloxy bedeutet oder
R² und R³ an zwei benachbarte Kohlenstoffatome gebunden sind und gemeinsam eine Alkylendioxygruppe mit 1 bis 2 Kohlenstoffatomen darstellen,
R⁴ Wasserstoff oder niederes Alkyl bedeutet,
R⁵ in 1- oder 2-Stellung angeordnet ist und Wasserstoff, niederes Alkyl oder eine Phenylniederalkylgruppe bedeutet,
R⁶ Wasserstoff, niederes Alkyl, niederes Alkoxy, Hydroxy, Halogen oder Nitro oder, falls R², R³ und R⁷ nicht Hydroxy sind, auch niederes Alkanoyloxy bedeutet und
R⁷ Wasserstoff, niederes Alkyl, niederes Alkoxy, Hydroxy oder Halogen oder, falls R², R³ und R⁶ nicht Hydroxy sind, auch niederes Alkanoyloxy bedeutet oder
R⁶ und R⁷ an zwei benachbarte Kohlenstoffatome gebunden sind und gemeinsam eine Alkylendioxygruppe mit 1 bis 2 Kohlenstoffatomen bilden,
sowie deren physiologisch verträgliche Säureadditionssalze.

2. Verbindungen gemäß Anspruch 1, worin A für eine Gruppe der Formel a) steht, worin R¹ niederes Alkyl oder Wasserstoff bedeutet und Q eine (CH₂)ₘ-Gruppe, worin m 2 bis 6 bedeutet, darstellt, oder A für eine Gruppe der Formel b) steht, worin p 5 ist.

3. Verbindungen gemäß einem der vorstehenden Ansprüche, worin R⁴ Wasserstoff und R⁵ Wasserstoff, Alkyl mit 1 bis 2 Kohlenstoffatomen oder Benzyl bedeuten.

4. Verbindungen gemäß einem der vorstehenden Ansprüche, worin n 2 oder 3 bedeutet.

5. Verbindungen gemäß einem der vorstehenden Ansprüche, dadurch gekennzeichnet, daß n 2 oder 3 bedeutet, A für eine Gruppe der Formel a), worin R¹ Wasserstoff oder niederes Alkyl bedeutet und Q eine (CH₂)ₘ-Gruppe, worin m 2 bis 4 bedeutet, darstellt, steht, oder A für eine Gruppe der Formel b), worin p 5 bedeutet, steht, R² Wasserstoff, niederes Alkoxy oder Hydroxy bedeutet, R³ Wasserstoff oder niederes Alkoxy bedeutet, R⁴ Wasserstoff bedeutet, R⁵ Wasserstoff oder Benzyl bedeutet, R⁶ Wasserstoff, niederes Alkoxy, Hydroxy oder Nitro bedeutet und R⁷ Wasserstoff oder niederes Alkoxy bedeutet.

6. Verbindungen gemäß Anspruch 5, dadurch gekennzeichnet, daß n 2 bedeutet, A für eine Gruppe der Formal a), worin R¹ Methyl oder Wasserstoff bedeutet und Q eine (CH₂)ₘ-Gruppe darstellt, worin m 3 oder 4 bedeutet, R² Methoxy oder Hydroxy bedeutet, R³ Methoxy bedeutet, R⁴ Wasserstoff bedeutet, R⁵ Wasserstoff oder Benzyl bedeutet, R⁶ Methoxy, Wasserstoff oder Hydroxy bedeutet und R⁷ Methoxy oder Wasserstoff bedeutet.

7. 3-{3-[N-(2-(3,4-Dimethoxyphenyl)-ethyl)-N-methylamino]-propyloxy}-5-(3,4-dimethoxyphenyl)-pyrazol, 3-{3-[N-(2-(3,4-Dimethoxyphenyl)-ethyl)-N-methylamino]-propyloxy}-5-(4-hydroxy-3-methoxyphenyl)-pyrazol und deren physiologisch verträgliche Säureadditionssalze gemäß Anspruch 6.

8. 3-{[N-(2-(3,4-Dimethoxphenyl)-ethyl)-piperidin-3-yl]-methoxy}-5-(3,4-dimethoxyphenyl)-pyrazol und dessen physiologisch verträgliche Säureadditionssalze gemäß Anspruch 5.

9. Arzneimittel enthaltend eine pharmakologisch wirksame Menge einer Verbindung gemäß Anspruch 1 und übliche pharmazeutische Hilfs- und/oder Trägerstoffe.

10. Verfahren zur Herstellung von Verbindungen der allgemeinen Formel I 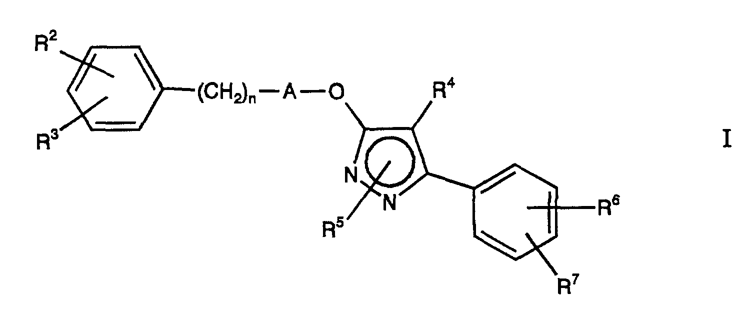 worin
n eine ganze Zahl von 1 bis 5 bedeutet,
A für eine Gruppe der allgemeinen Formel a 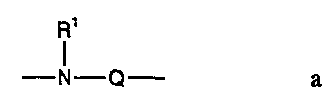 worin R¹ Wasserstoff oder eine niedere Alkylgruppe bedeutet und Q eine (CH₂)ₘ-Gruppe, worin m 2 bis 8 bedeutet und welche gegebenenfalls in α-Stellung zu dem Sauerstoffatom durch 1 bis 2 niedere Alkylgruppen substituiert sein kann, steht oder A für eine Gruppe der allgemeinen Formel b 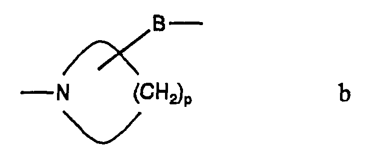 worin p 4 bis 6 bedeutet und B eine (CH₂)ᵣ-Gruppe, worin r 1 bis 3 bedeutet, darstellt, steht,
R² Wasserstoff, Halogen, niederes Alkyl, niederes Alkoxy, Hydroxy, Trifluormethyl oder Nitro oder, falls R³, R⁶ und R⁷ nicht Hydroxy sind, auch niederes Alkanoyloxy bedeutet und
R³ Wasserstoff, Halogen, niederes Alkyl, niederes Alkoxy, Hydroxy oder, falls R², R⁶ und R⁷ nicht Hydroxy sind, auch niederes Alkanoyloxy bedeutet oder
R² und R³ an zwei benachbarte Kohlenstoffatome gebunden sind und gemeinsam eine Alkylendioxygruppe mit 1 bis 2 Kohlenstoffatomen darstellen,
R⁴ Wasserstoff oder niederes Alkyl bedeutet.
R⁵ in 1- oder 2-Stellung angeordnet ist und Wasserstoff, niederes Alkyl oder eine Phenylniederalkylgruppe bedeutet,
R⁶ Wasserstoff, niederes Alkyl, niederes Alkoxy, Hydroxy, Halogen, oder Nitro oder, falls R², R³ und R⁷ nicht Hydroxy sind, auch niederes Alkanoyloxy bedeutet und
R⁷ Wasserstoff, niederes Alkyl, niederes Alkoxy, Hydroxy oder Halogen oder, falls R², R³ und R⁶ nicht Hydroxy sind, auch niederes Alkanoyloxy bedeutet oder
R⁶ und R⁷ an zwei benachbarte Kohlenstoffatome gebunden sind und gemeinsam eine Alkylendioxygruppe mit 1 bis 2 Kohlenstoffatomen bilden,
sowie deren physiologisch verträglichen Säureadditionssalzen,
dadurch gekennzeichnet, daß man
a) zur Herstellung von Verbindungen der allgemeinen Formel Ia 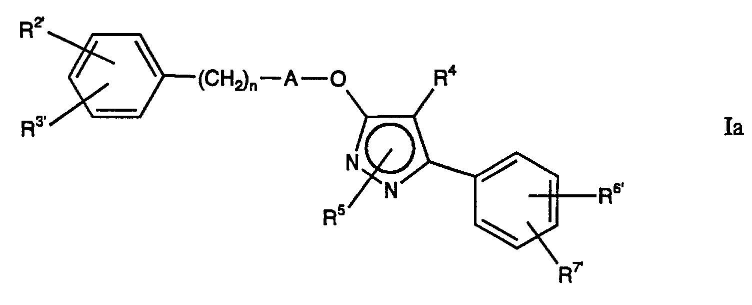 worin R⁴, R⁵, A und n obige Bedeutung besitzen und R^{2'}, R^{3'}, R^{6'} und R^{7'} die für R², R³, R⁶ und R⁷ angegebene Bedeutung mit Ausnahme von niederem Alkanoyloxy besitzen, Verbindungen der allgemeinen Formel II 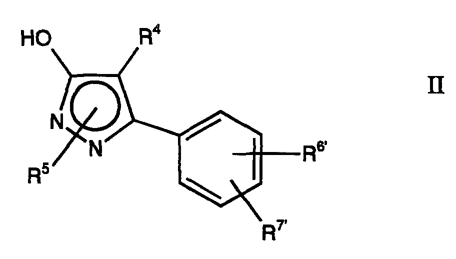 worin R⁴, R⁵, R^{6'} und R^{7'} obige Bedeutung besitzen, wobei jedoch allfällige freie Hydroxygruppen mit einer Hydroxy-Schutzgruppe versehen sind, mit Verbindungen der allgemeinen Formel III 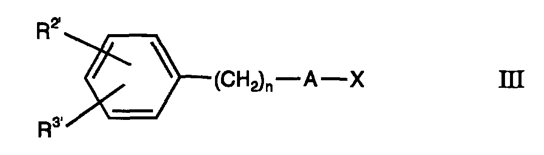 worin R²', R³', n und A obige Bedeutung besitzen, wobei jedoch allfällige freie Hydroxygruppen mit einer Hydroxy-Schutzgruppe versehen sind, und X für eine abspaltbare Fluchtgruppe steht, umsetzt und anschließend allfällige Hydroxy-Schutzgruppen wieder abspaltet, oder
b) zur Herstellung von Verbindungen der Formel Ia, Verbindungen der Formel II mit Triphenylphosphin in Gegenwart von Azodicarbonsäureestern der allgemeinen Formel XVIII
R¹⁰OOC―N=N―COOR¹¹ XVIII
worin R¹⁰ und R¹¹ je niederes Alkyl bedeuten, umsetzt und das Reaktionsprodukt mit Verbindungen der allgemeinen Formel IV 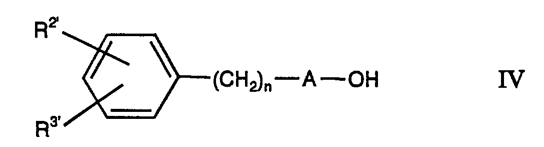 worin R^{2'}, R^{3'}, n und A obige Bedeutung besitzen, wobei jedoch eine allfällige freie NH-Gruppe in dem Rest A mit einer Aminoschutzgruppe und allfällige freie Hydroxygruppen mit Hydroxy-Schutzgruppen versehen sind, umsetzt und anschließend allfällige Amino- und/oder Hydroxy-Schutzgruppen wieder abspaltet, oder
c) zur Herstellung von Verbindungen der allgemeinen Formel Ib 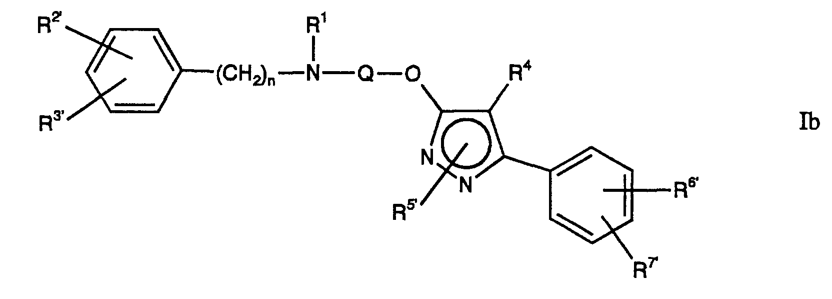 worin R¹, R^{2'}, R^{3'}, R⁴, R^{6'}, R^{7'}, Q und n obige Bedeutung besitzen und R^{5'} die für R⁵ angegebene Bedeutung besitzt, wobei jedoch, falls die Kettenlänge m des Restes Q 3 ist, R^{5'} eine an dem dem Sauerstoffatom benachbarten Stickstoffatom des Pyrazolringes angeordnete niedere Alkylgruppe oder Phenylniederalkylgruppe darstellt, Verbindungen der allgemeinen Formel V 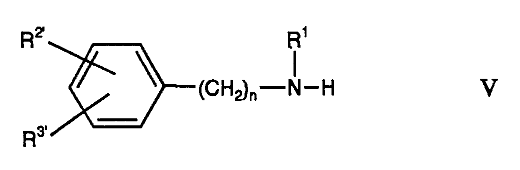 worin R¹, R^{2'}, R^{3'} und n obige Bedeutung besitzen, wobei jedoch allfällige freie Hydroxygruppen mit einer Hydroxy-Schutzgruppe versehen sind, mit Verbindungen der allgemeinen Formel VI 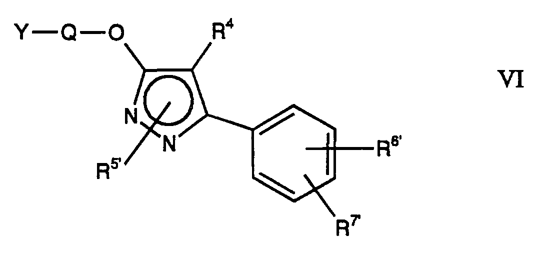 worin R⁴, R^{5'}, R^{6'}, R^{7'} und Q obige Bedeutung besitzen, wobei jedoch allfällige freie Hydroxygruppen mit einer Hydroxy-Schutzgruppe versehen sind, und Y eine aminolytisch abspaltbare Fluchtgruppe bedeutet, umsetzt und anschließend allfällige Hydroxy-Schutzgruppen wieder abspaltet, oder
d) zur Herstellung von Verbindungen der Formel Ia Verbindungen der allgemeinen Formel VII 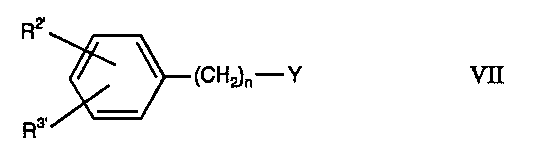 worin R^{2'}, R^{3'}, n und Y obige Bedeutung besitzen, wobei jedoch allfällige freie Hydroxygruppen mit einer Hydroxy-Schutzgruppe versehen sind,mit Verbindungen der allgemeinen Formel VIII 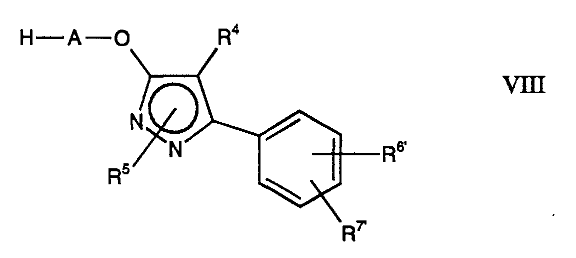 worin R⁴, R⁵, R^{6'}, R^{7'} und A obige Bedeutung besitzen, wobei jedoch allfällige freie Hydroxygruppen mit einer Hydroxy-Schutzgruppe versehen sind, umsetzt und anschließend allfällige Hydroxy-Schutzgruppen wieder abspaltet, oder
e) zur Herstellung von Verbindungen der allgemeinen Formel Ic 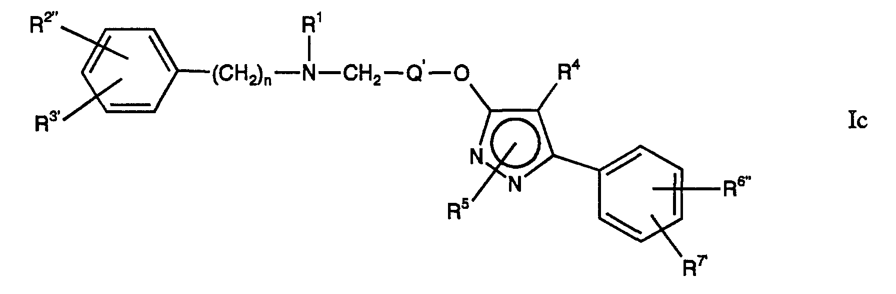 worin R¹, R^{3'}, R⁴, R⁵, R^{7'} und n obige Bedeutung besitzen, R^{2''} und R^{6''} die für R2 und R6 angegebene Bedeutung mit Ausnahme von Nitro und niederem Alkanoyloxy besitzen, und Q' eine (CH₂)ₘ,-Gruppe, worin m' 1 bis 7 bedeutet und welche gegebenenfalls in α-Stellung zu dem Sauerstoffatom durch 1 bis 2 niedere Alkylgruppen substituiert ist, darstellt, Verbindungen der allgemeinen Formel IX 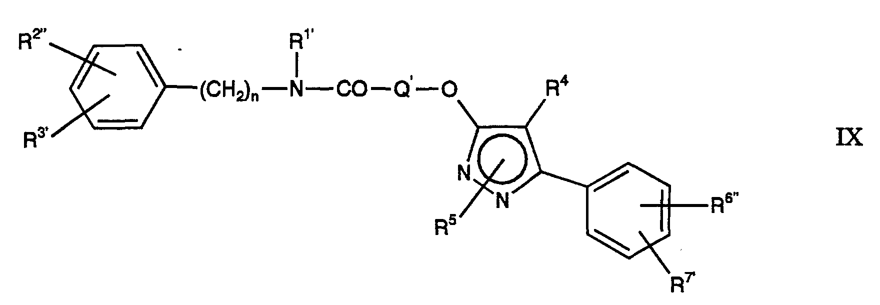 worin R^{2''}, R^{3'}, R⁴, R⁵, R^{6''}, R^{7'}, n und Q' obige Bedeutung besitzen, wobei jedoch allfällige freie Hydroxygruppen mit einer Hydroxy-Schutzgruppe versehen sind, und R¹' niederes Alkyl oder eine Aminoschutzgruppe bedeutet, reduziert und anschließend allfällige Amino- und/oder Hydroxy-Schutzgruppen wieder abspaltet, oder
f) zur Herstellung von Verbindungen der allgemeinen Formel Id 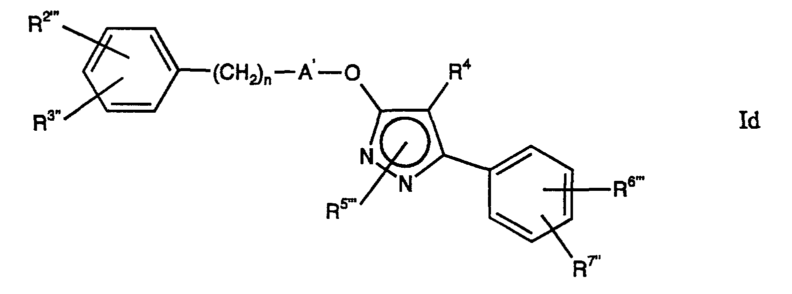 worin R⁴, n und A obige Bedeutung besitzen, R^{5'''} die für R⁵ angegebene Bedeutung besitzt, wobei jedoch R^{5'''} nicht Benzyl bedeutet, falls der Rest A eine NH-Gruppe enthält, und R^{2'''}, R^{3''}, R^{6'''} und R^{7''} die für R², R³, R⁶ und R⁷ angegebene Bedeutung mit Ausnahme von Hydroxy besitzen, wobei jedoch mindestens einer der Substituenten R^{2'''}, R^{3''}, R^{6'''} und R^{7''} niederes Alkanoyloxy bedeutet, in Verbindungen der allgemeinen Formel X 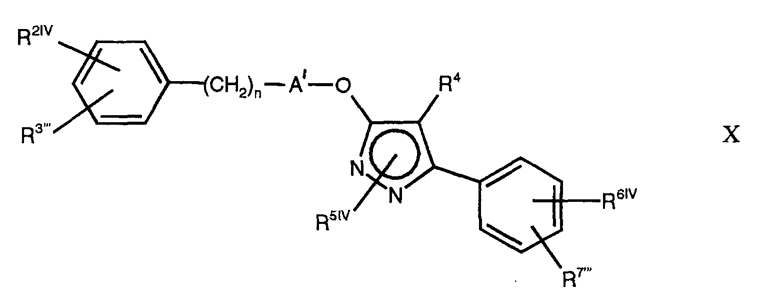 worin n und R4 obige Bedeutung besitzen, R^{2IV}, R^{3'''}, R^{6IV} und R^{7'''} die für R², R³, R⁶ und R⁷ angegebene Bedeutung mit Ausnahme von niederem Alkanoyloxy besitzen, worin jedoch mindestens einer der Substituenten R^{2IV}, R³''', R^{6IV} und R^{7'''} Hydroxy bedeutet, R^{5IV} niederes Alkyl oder eine Phenylniederalkylgruppe bedeutet und A' die für A angegebene Bedeutung besitzt, wobei jedoch eine allfällige in dem Rest A' enthaltene NH-Gruppe durch eine Benzylschutzgruppe geschützt ist, die freien Hydroxygruppen R^{2IV}, R^{3'''}, R^{6'''} und/oder R^{7'''} acyliert und anschließend eine allfällige Benzylschutzgruppe wieder abspaltet, und
gewünschtenfalls in erhaltenen Verbindungen der Formel I, worin R², R³, R⁶ und/oder R⁷ Methoxy bedeuten, aus diesen Gruppen eine Hydroxygruppe freisetzt und/oder gewünschtenfalls aus erhaltenen Verbindungen der Formel I, worin R⁵ Benzyl bedeutet, diese Benzylgruppe abspaltet, und gewünschtenfalls freie Verbindungen der Formel I in ihre Säureadditionssalze überführt oder die Säureadditonssalze in die freien Verbindungen der Formel I überführt.

11. Verbindungen der allgemeinen Formel IX 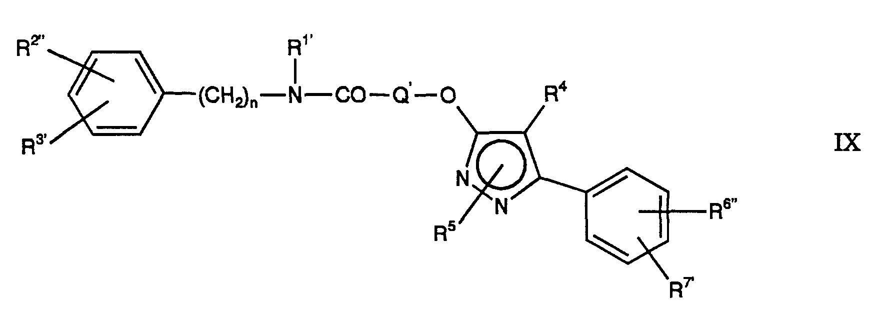 worin
R^{1'} eine niedere Alkylgruppe oder eine Aminoschutzgruppe bedeutet,
R^{2''} Wasserstoff, Halogen, niederes Alkyl, Trifluormethyl oder niederes Alkoxy bedeutet und
R^{3'} Wasserstoff, Halogen, niederes Alkyl oder niederes Alkoxy bedeutet oder
R^{2''} und R^{3'} an zwei benachbarte Kohlenstoffatome gebunden sind und gemeinsam eine Alkylendioxygruppe mit 1 bis 2 Kohlenstoffatomen darstellen,
n eine ganze Zahl von 1 bis 5 bedeutet,
Q' eine (CH₂)ₘ,-Gruppe, worin m' 1 bis 7 bedeutet und welche gegebenenfalls in α-Stellung zu dem Sauerstoffatom durch 1 bis 2 niedere Alkylgruppen substituiert ist, darstellt,
R⁴ Wasserstoff oder niederes Alkyl bedeutet,
R⁵ in 1- oder 2-Stellung angeordnet ist und Wasserstoff, niederes Alkyl oder eine Phenylniederalkylgruppe bedeutet,
R^{6''} Wasserstoff, niederes Alkyl, nieders Alkoxy oder Halogen bedeutet und
R^{7'} Wasserstoff, niederes Alkyl, niederes Alkoxy, oder Halogen bedeutet oder
R^{6''} und R^{7'} an zwei benachbarte Kohlenstoffatome gebunden sind und gemeinsam eine Alkylendioxygruppe mit 1 bis 2 Kohlenstoffatomen bilden.

## Claims

1. Compounds of the general formula I 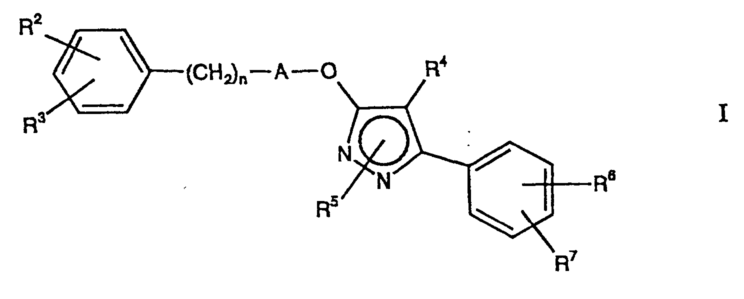 in which
n denotes an integer from 1 to 5,
A represents a group of the general formula a 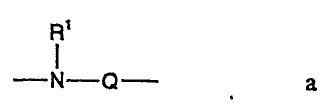 in which
R¹ denotes hydrogen or a lower alkyl group and Q denotes a (CH₂)ₘ group in which m denotes 2 to 8 and which may optionally be substituted in the α position to the oxygen atom by 1 to 2 lower alkyl groups,
or A represents a group of the general formula b 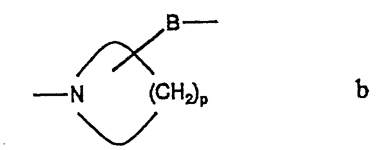 in which p denotes 4 to 6 and B represents a (CH₂)ᵣ group in which r denotes 1 to 3,
R² denotes hydrogen, halogen, lower alkyl, lower alkoxy, hydroxyl, trifluoromethyl or nitro or, if R³, R⁶ and R⁷ are not hydroxyl, alternatively denotes lower alkanoyloxy, and
R³ denotes hydrogen, halogen, lower alkyl, lower alkoxy, hydroxyl or, if R², R⁶ and R⁷ are not hydroxyl, alternatively denotes lower alkanoyloxy, or
R² and R³ are attached to two adjacent carbon atoms and together represent an alkylenedioxy group having 1 to 2 carbon atoms,
R⁴ denotes hydrogen or lower alkyl,
R⁵ is disposed in the 1 or 2 position and denotes hydrogen, lower alkyl or a phenyl-lower alkyl group,
R⁶ denotes hydrogen, lower alkyl, lower alkoxy, hydroxyl, halogen or nitro or, if R², R³ and R⁷ are not hydroxyl, alternatively denotes lower alkanoyloxy, and
R⁷ denotes hydrogen, lower alkyl, lower alkoxy, hydroxyl or halogen or, if R², R³ and R⁶ are not hydroxyl, altematively denotes lower alkanoyloxy, or
R⁶ and R⁷ are attached to two adjacent carbon atoms and together form an alkylenedioxy group having 1 to 2 carbon atoms,
and their physiologically tolerable acid addition salts.

2. Compounds according to Claim 1, in which A represents a group of the formula a) in which R¹ denotes lower alkyl or hydrogen and Q represents a (CH₂)ₘ group in which m denotes 2 to 6, or A represents a group of the formula b) in which p is 5.

3. Compounds according to one of the preceding claims, in which R⁴ denotes hydrogen and R⁵ denotes hydrogen, alkyl having 1 to 2 carbon atoms or benzyl.

4. Compounds according to one of the preceding claims, in which n denotes 2 or 3.

5. Compounds according to one of the preceding claims, characterised in that n denotes 2 or 3, A represents a group of the formula a) in which R¹ denotes hydrogen or lower alkyl and Q denotes a (CH₂)ₘ group in which m denotes 2 to 4, or A represents a group of the formula b) in which p denotes 5, and R² denotes hydrogen, lower alkoxy or hydroxyl, R³ denotes hydrogen or lower alkoxy, R⁴ denotes hydrogen, R⁵ denotes hydrogen or benzyl, R⁶ denotes hydrogen, lower alkoxy, hydroxyl or nitro and R⁷ denotes hydrogen or lower alkoxy.

6. Compounds according to Claim 5, characterised in that n denotes 2, A represents a group of the formula a) in which R¹ denotes methyl or hydrogen and Q represents a (CH₂)ₘ group in which m denotes 3 or 4, R² denotes methoxy or hydroxyl, R³ denotes methoxy, R⁴ denotes hydrogen, R⁵ denotes hydrogen or benzyl, R⁶ denotes methoxy, hydrogen or hydroxyl and R⁷ denotes methoxy or hydrogen.

7. 3-{3-[N-(2-(3,4-Dimethoxyphenyl)-ethyl)-N-methylamino]-propyloxy}-5-(3,4-dimethoxyphenyl)-pyrazole, 3-{3-[N-(2-(3,4-dimethoxyphenyl)-ethyl)-N-methylamino]-propyloxy}-5-(4-hydroxy-3-methoxyphenyl)-pyrazole and their physiologically tolerable acid addition salts according to Claim 6.

8. 3-{[N-(2-(3,4-Dimethoxyphenyl)-ethyl)-piperidin-3-yl]-methoxy}-5-(3,4-dimethoxyphenyl)-pyrazole and its physiologically tolerable acid addition salts according to Claim 5.

9. Medicaments containing a pharmacologically effective quantity of a compound according to Claim 1 and conventional pharmaceutical adjuvants and/or excipients.

10. Process for the preparation of compounds of the general formula I 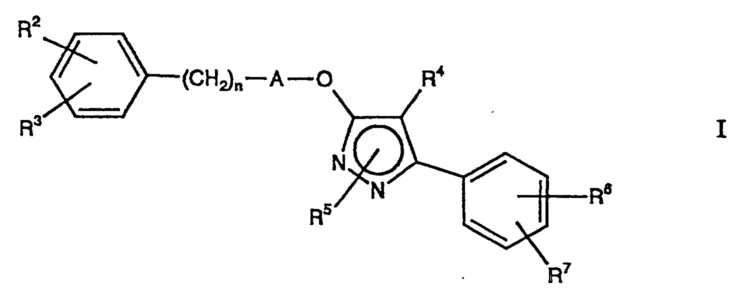 in which
n denotes an integer from 1 to 5,
A represents a group of the general formula a 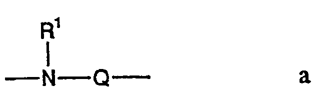 in which R¹ denotes hydrogen or a lower alkyl group and Q denotes a (CH₂)ₘ group in which m denotes 2 to 8 and which may optionally be substituted in the α position to the oxygen atom by 1 to 2 lower alkyl groups, or A represents a group of the general formula b 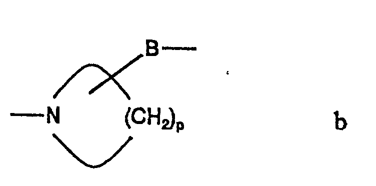 in which p denotes 4 to 6 and B represents a (CH₂)ᵣ group in which r denotes 1 to 3,
R² denotes hydrogen, halogen, lower alkyl, lower alkoxy, hydroxyl, trifluoromethyl or nitro or, if R³, R⁶ and R⁷ are not hydroxyl, alternatively denotes lower alkanoyloxy, and
R³ denotes hydrogen, halogen, lower alkyl, lower alkoxy, hydroxyl or, if R², R⁶ and R⁷ are not hydroxyl, alternatively denotes lower alkanovloxy, or
R² and R³ are attached to two adjacent carbon atoms and together represent an alkylenedioxy group having 1 to 2 carbon atoms,
R⁴ denotes hydrogen or lower alkyl,
R⁵ is disposed in the 1 or 2 position and denotes hydrogen, lower alkyl or a phenyl-lower alkyl group,
R⁶ denotes hydrogen, lower alkyl, lower alkoxy, hydroxyl, halogen or nitro or, if R², R³ and R⁷ are not hydroxyl, alternatively denotes lower alkanoyloxy, and
R⁷ denotes hydrogen, lower alkyl, lower alkoxy, hydroxyl or halogen or, if R², R³ and R⁶ are not hydroxyl, alternatively denotes lower alkanoyloxy, or
R⁶ and R⁷ are attached to two adjacent carbon atoms and together form an alkylenedioxy group having 1 to 2 carbon atoms,
and their physiologically tolerable acid addition salts,
characterised in that
a) for the preparation of compounds of the general formula la 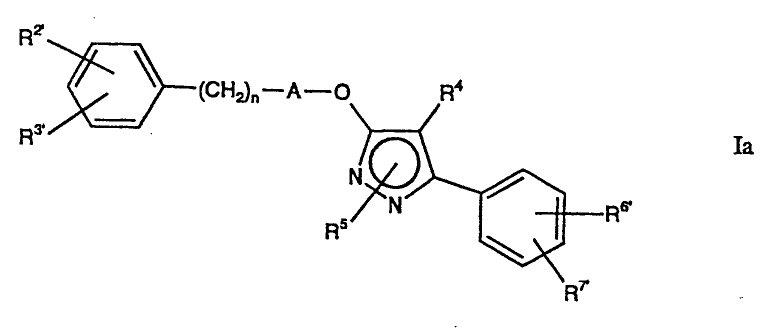 in which R⁴, R⁵, A and n possess the above meaning and R^{2'}, R^{3'}, R^{6'} and R^{7'} possess the meaning given for R², R³, R⁶ and R⁷ with the exception of lower alkanoyloxy,
compounds of the general formula II 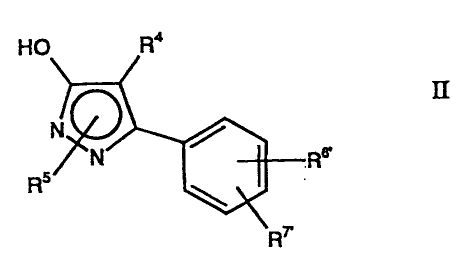 in which R⁴, R⁵, R^{6'} and R^{7'} possess the above meaning, but in which any free hydroxyl groups are provided with a hydroxyl-protecting group, are reacted with compounds of the general formula III 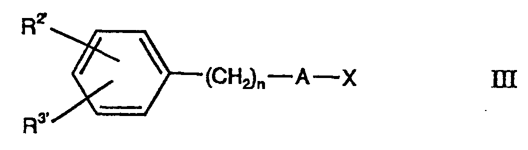 in which R^{2'}, R^{3'}, n and A possess the above meaning, but in which any free hydroxyl groups are provided with a hydroxyl-protecting group, and X represents a leaving group which can be eliminated, and subsequently any hydroxyl-protecting groups are eliminated again, or
b) for the preparation of compounds of the general formula la compounds of the formula II are reacted with triphenylphosphine in the presence of azodicarboxylic esters of the general formula XVIII
R¹⁰OOC-N=N-COOK¹¹ XVIII
in which R¹⁰ and R¹¹ each denotes lower alkyl, and the reaction product is reacted with compounds of the general formula IV 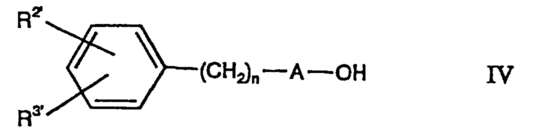 in which R^{2'}, R^{3'}, n and A possess the above meaning, but in which any free NH group in the radical A is provided with an amino-protecting group and any free hydroxyl groups are provided with hydroxyl-protecting groups, and subsequently any amino- and/or hydroxyl-protecting groups are eliminated again, or
c) for the preparation of compounds of the general formula Ib 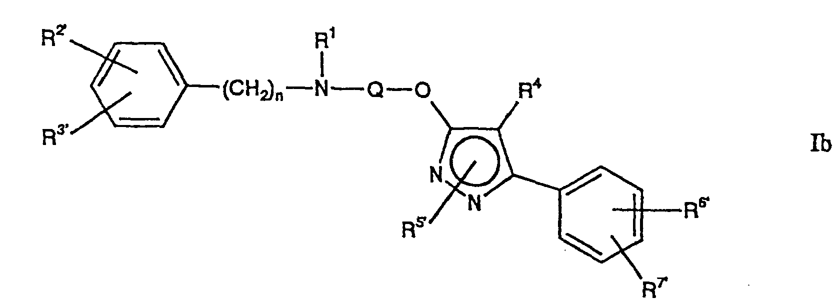 in which R¹, R², R³, R⁴, R^{6'}, R^{7'}, Q and n possess the above meaning and R^{5'} possesses the meaning given for R⁵, but in which, if the chain length m of the radical Q is 3, R^{5'} represents a phenyl-lower alkyl group or lower alkyl group which is arranged on that nitrogen atom of the pyrazole ring which is adjacent to the oxygen atom, compounds of the general formula V 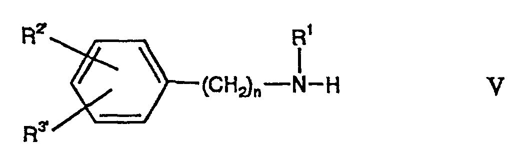 in which R¹, R^{2'}, R^{3'} and n possess the above meaning, but in which any free hydroxyl groups are provided with a hydroxyl-protecting group, are reacted with compounds of the general formula VI 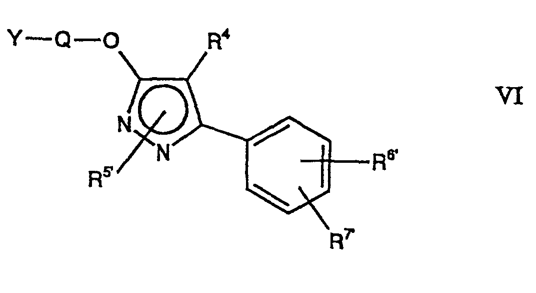 in which R⁴, R^{5'}, R^{6'}, R^{7'} and Q possess the above meaning, but in which any free hydroxyl groups are provided with a hydroxyl-protecting group, and Y denotes a leaving group which can be eliminated by aminolysis, and subsequently any hydroxyl-protecting groups are eliminated again, or
d) for the preparation of compounds of the formula la, compounds of the general formula VII 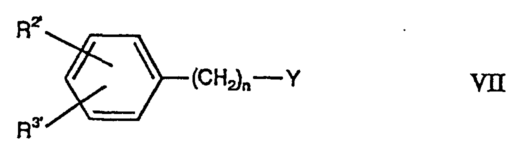 in which R^{2'}, R^{3'}, n and Y possess the above meaning, but in which any free hydroxyl groups are provided with a hydroxyl-protecting group, are reacted with compounds of the general formula VIII 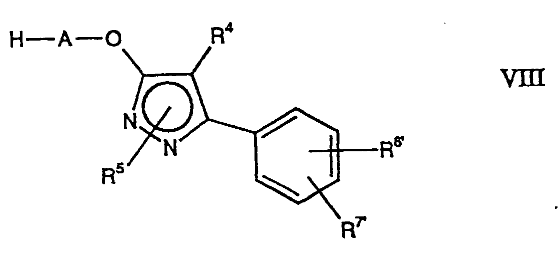 in which R⁴, R⁵, R^{6'}, R^{7'} and A possess the above meaning, but in which any free hydroxyl groups are provided with a hydroxyl-protecting group, and subsequently any hydroxyl-protecting groups are eliminated again, or
e) for the preparation of compounds of the general formula Ic 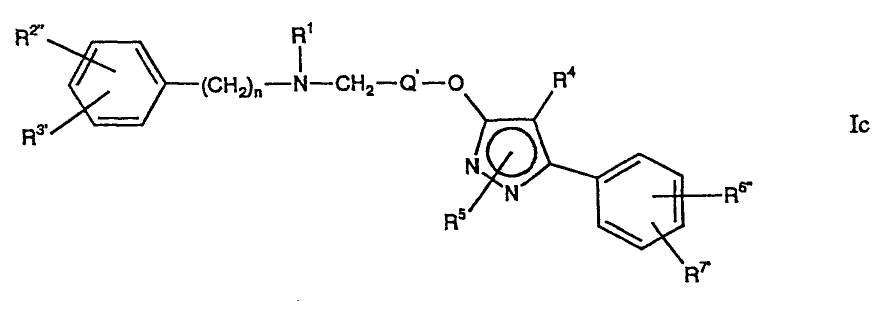 in which R¹, R^{3'}, R⁴, R⁵, R^{7'} and n possess the above meaning, R^{2''} and R^{6''} possess the meaning given for R² and R⁶ with the exception of nitro and lower alkanoyloxy, and Q' represents a (CH₂)_{m'} group in which m' denotes 1 to 7 and which is optionally substituted in the α position to the oxygen atom by 1 to 2 lower alkyl groups, compounds of the general formula IX 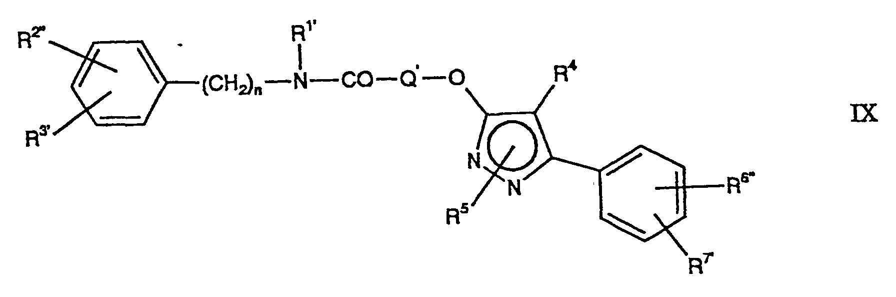 in which R^{2''}, R^{3'}, R⁴, R⁵, R^{6''}, R^{7'}, n and Q' possess the above meaning, but in which any free hydroxyl groups are provided with a hydroxyl-protecting group, and R^{1'} denotes lower alkyl or an amino-protecting group, are reduced and subsequently any amino- and/or hydroxyl-protecting groups are eliminated again, or
f) for the preparation of compounds of the general formula Id 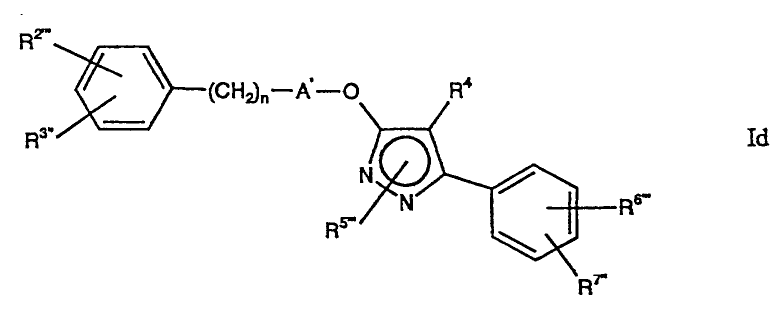 in which R⁴, n and A possess the above meaning, R^{5'''} possesses the meaning given for R⁵ but R^{5'''} does not denote benzyl if the radical A contains an NH group, and R^{2'''}, R^{3''}, R^{6'''} and R^{7''} possess the meaning given for R², R³, R⁶ and R⁷ with the exception of hydroxyl, but where at least one of the substituents R^{2'''}, R^{3''}, R^{6'''} and R^{7''} denotes lower alkanoyloxy, in compounds of the general formula X 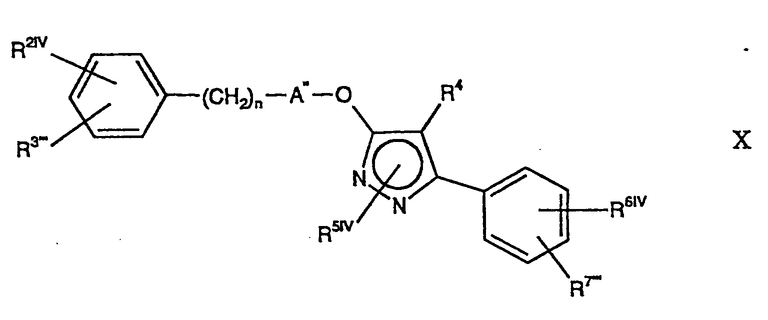 in which n and R⁴ possess the above meaning, R^{2IV}, R^{3'''}, R^{6IV} and R^{7'''} possess the meaning given for R², R³, R⁶ and R⁷ with the exception of lower alkanoyloxy, but in which at least one of the substituents R^{2IV}, R^{3'''}, R^{6IV} and R^{7'''} denotes hydroxyl, R^{5IV} denotes lower alkyl or a phenyl-lower alkyl group and A' possesses the meaning given for A, but in which any NH group present in the radical A' is protected by a benzyl protective group, the free hydroxyl groups R^{2IV}, R^{3'''}, R^{6'''} and/or R^{7'''} are acylated, and subsequently any benzyl protective group is eliminated again, and if desired, in resulting compounds of the formula I in which R², R³, R⁶ and/or R⁷ denote methoxy a hydroxyl group is liberated from these groups, and/or if desired the benzyl group is eliminated from resulting compounds of the formula I in which R⁵ denotes benzyl, and if desired free compounds of the formula I are converted to their acid addition salts or the acid addition salts are converted to the free compounds of the formula I.

11. Compounds of the general formula IX 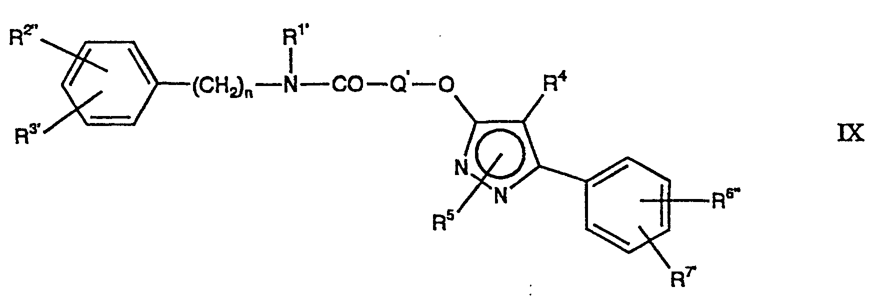 in which
R^{1'} denotes a lower alkyl group or an amino-protecting group,
R^{2''} denotes hydrogen, halogen, lower alkyl, trifluoromethyl or lower alkoxy, and
R^{3'} denotes hydrogen, halogen, lower alkyl or lower alkoxy, or
R^{2''} and R^{3'} are attached to two adjacent carbon atoms and together represent an alkylenedioxy group having 1 to 2 carbon atoms,
n denotes an integer from 1 to 5,
Q' represents a (CH₂)ₘ, group in which m' denotes 1 to 7 and which is optionally substituted in the α position to the oxygen atom by 1 to 2 lower alkyl groups,
R⁴ denotes hydrogen or lower alkyl,
R⁵ is disposed in the 1 or 2 position and denotes hydrogen, lower alkyl or a phenyl-lower alkyl group,
R^{6''} denotes hydrogen, lower alkyl, lower alkoxy or halogen, and
R^{7'} denotes hydrogen, lower alkyl, lower alkoxy or halogen, or
R^{6''} and R^{7'} are attached to two adjacent carbon atoms and together form an alkylenedioxy group having 1 to 2 carbon atoms.

## Revendications

1. Composés de formule générale I : 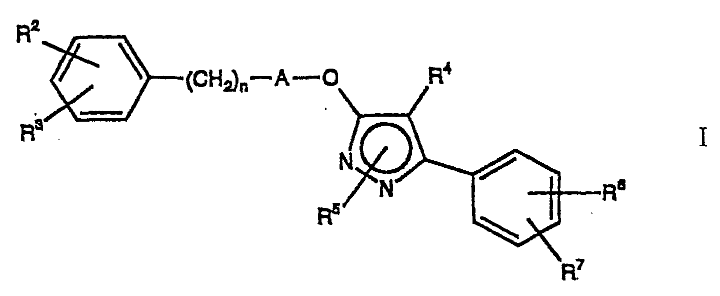 dans laquelle
n est un nombre entier valant 1 à 5,
A représente un groupe de formule générale (a) 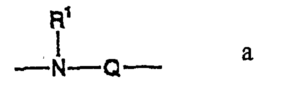 dans laquelle R¹ représente un atome d'hydrogène ou un groupe alkyle inférieur, et Q représente un groupe (CH₂)ₘ dans lequel m vaut 2 à 8 et qui peut éventuellement être substitué en position α par rapport à l'atome d'oxygène, par 1 à 2 groupes allyle inférieurs, ou bien A représente un groupe de formule générale (b) 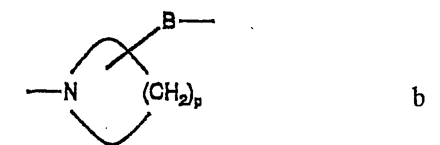 dans laquelle p vaut 4 à 6 et B représente un groupe (CH₂)ᵣ, dans lequel r vaut 1 à 3,
R² représente un atome d'hydrogène, un halogène, un groupe alkyle inférieur, alcoxy inférieur, hydroxy, trifluorométhyle ou nitro ou bien, si R³, R⁶ et R⁷ ne sont pas des groupes hydroxy, R² peut également représenter un groupe alcanoyloxy inférieur, et
R³ représente un atome d'hydrogène, un halogène, un groupe alkyle inférieur, alcoxy inférieur, hydroxy, ou, si R², R⁶ et R⁷ ne sont pas des groupes hydroxy, R³ peut également représenter un groupe alcanoyloxy inférieur, ou bien
R² et R³ sont fixés sur deux atomes de carbone voisins et forment ensemble un groupe alkylènedioxy ayant 1 ou 2 atomes de carbone,
R⁴ représente un atome d'hydrogène ou un groupe alkyle inférieur,
R⁵ est fixé en position 1 ou 2 et représente un atome d'hydrogène, un groupe alkyle inférieur ou un groupe phényl-alkyle inférieur,
R⁶ représente un atome d'hydrogène, un groupe alkyle inférieur, alcoxy inférieur, hydroxy, halogéno ou nitro, ou bien, si R², R³ et R⁷ ne sont pas des groupes hydroxy, R⁶ peut également représenter un groupe alcanoyloxy inférieur, et
R⁷ représente un atome d'hydrogène, un groupe alkyle inférieur, alcoxy inférieur, hydroxy ou halogéno, ou bien, si R², R³ et R⁶ ne sont pas des groupes hydroxy, R⁷ peut également représenter un groupe alcanoyloxy inférieur, ou bien
R⁶ et R⁷ sont fixés sur deux atomes de carbone voisins, et ils forment ensemble un groupe alkylènedioxy ayant 1 ou 2 atomes de carbone, ainsi que leurs sels d'addition d'acides physiologiquement acceptables.

2. Composés selon la revendication 1, où A représente un groupe de formule a), dans laquelle R¹ représente un groupe alkyle inférieur ou un atome d'hydrogène et Q représente un groupe (CH₂)ₘ, dans lequel m vaut 2 à 6, ou bien A représente un groupe de formule b), dans lequel p vaut 5.

3. Composés selon l'une des revendications précédentes, où R4 représente un atome d'hydrogène et R⁵ représente un atome d'hydrogène, un groupe alkyle ayant 1 ou 2 atomes de carbone ou un groupe benzyle.

4. Composés selon l'une quelconque des revendications précédentes, où n vaut 2 ou 3.

5. Composés selon l'une quelconque des revendications précédentes, caractérisés en ce que n vaut 2 ou 3, A représente un groupe de formule a) dans laquelle R¹ représente un atome d'hydrogène ou un groupe alkyle inférieur, et Q représente un groupe (CH₂)ₘ dans lequel m vaut 2 à 4, ou bien A représente un groupe de formule b), dans laquelle p vaut 5, R² représente un atome d'hydrogène, un groupe alcoxy inférieur ou hydroxy, R³ représente un atome d'hydrogène ou un groupe alcoxy inférieur, R⁴ représente un atome d'hydrogène, R⁵ représente un atome d'hydrogène ou un groupe benzyle, R⁶ représente un atome d'hydrogène, un groupe alcoxy inférieur, hydroxy ou nitro, et R⁷ représente un atome d'hydrogène ou un groupe alcoxy inférieur.

6. Composés selon la revendication 5, caractérisés en ce que n vaut 2, A représente un groupe de formule a), dans laquelle R¹ représente un groupe méthyle ou un atome d'hydrogène et Q représente un groupe (CH₂)ₘ, dans lequel m vaut 3 ou 4, R² représente un groupe méthoxy ou hydroxy, R³ représente un groupe méthoxy, R⁴ représente un atome d'hydrogène, R⁵ représente un atome d'hydrogène ou un groupe benzyle, R⁶ représente un groupe méthoxy, un atome d'hydrogène ou un groupe hydroxy, et R⁷ représente un groupe méthoxy ou un atome d'hydrogène.

7. Composés selon la revendication 6, caractérisés en ce qu'il s'agit du 3-{3-[N-(2-(3,4-diméthoxyphényl)-éthyl)-N-méthylamino]-propyloxy}-5-(3,4-diméthoxyphényl)-pyrazole, et du 3-{3-[N-2-(3,4-diméthoxyphényl)-éthyl)-N-méthylamino]-propyloxy}-5-(4-hydroxy-3-méthoxyphényl)-pyrazole et de leurs sels d'addition d'acides physiologiquement acceptables.

8. Composé selon la revendication 5, caractérisé en ce qu'il s'agit du 3-{[N-(2-(3,4-diméthoxyphényl)-éthyl)-pipéridine-3-yl]-méthoxy}-5-(3,4-diméthoxyphényl)-pyrazole et de ses sels d'addition d'acides physiologiquement acceptables.

9. Médicament caractérisé en ce qu'il contient une quantité pharmacologiquement active d'un composé selon la revendication 1 et des adjuvants, excipients et supports pharmaceutiques usuels.

10. Procédé de préparation de composés de formule générale I 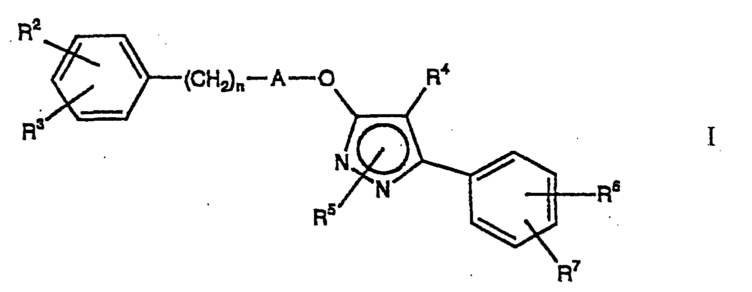 dans laquelle
n est un nombre entier valant 1 à 5,
A représente un groupe de formule générale a : 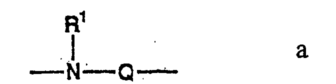 dans laquelle R¹ représente un atome d'hydrogène ou un groupe alkyle inférieur, et Q représente un groupe (CH₂)ₘ dans lequel m vaut 2 à 8 et qui peut éventuellement être substitué en position α par rapport à l'atome d'oxygène par 1 à 2 groupes alkyle inférieurs, ou bien A représente un groupe de formule générale b : 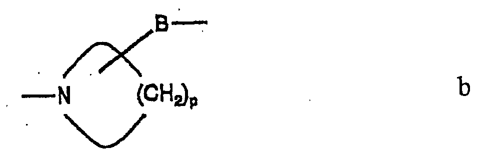 dans laquelle p vaut 4 à 6 et B représente un groupe (CH₂)ᵣ, dans lequel r vaut 1 à 3,
R² représente un atome d'hydrogène, un halogène, un groupe alkyle inférieur, alcoxy inférieur, hydroxy, trifluorométhyle ou nitro ou bien, si R³, R⁶ et R⁷ ne représentent pas chacun un groupe hydroxy, R² peut également représenter un groupe alcanoyloxy inférieur,
R³ représente un atome d'hydrogène, un halogène, un groupe alkyle inférieur, alcoxy inférieur, hydroxy, ou, si R², R⁶ et R⁷ ne représentent pas chacun un groupe hydroxy, R³ peut également représenter un groupe alcanoyloxy inférieur, ou
R² et R³ sont fixés sur deux atomes de carbone voisins et forment ensemble un groupe alkylènedioxy comportant 1 ou 2 atomes de carbone,
R⁴ représente un atome d'hydrogène ou un groupe alkyle inférieur,
R⁵ est placé en position 1 ou 2 et représente un atome d'hydrogène, un groupe alkyle inférieur ou un groupe phényl-alkyle inférieur,
R⁶ représente un atome d'hydrogène, un groupe alkyle inférieur, alcoxy inférieur, hydroxy, un atome d'halogène, ou un groupe nitro ou, si R², R³ et R⁷ ne représentent pas chacun un groupe hydroxy, R⁶ peut également représenter un groupe alcanoyloxy inférieur, et
R⁷ représente un atome d'hydrogène, un groupe alkyle inférieur, alcoxy inférieur, hydroxy ou un atome d'halogène, ou bien, si R², R3 et R6 ne représentent pas chacun un groupe hydroxy, R⁷ peut également représenter un groupe alcanoyloxy inférieur, ou bien
R⁶ et R⁷ sont fixés sur deux atomes de carbone voisins, et forment ensemble un groupe alkylènedioxy ayant 1 ou 2 atomes de carbone, ainsi que leurs sels d'addition d'acides pharmacologiquement acceptables,
caractérisé en ce que :
a) pour préparer des composés de formule générale Ia 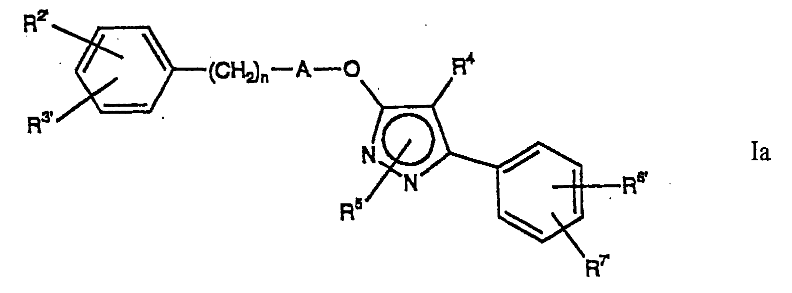 dans laquelle R⁴, R⁵, A et n ont le sens précité, et R^{2'}, R^{3'}, R^{6'} et R^{7'} ont le sens indiqué pour R², R³, R⁶ et R⁷, à l'exclusion du sens d'un groupe alcanoyloxy inférieur, on fait réagir des composés de formule générale II : 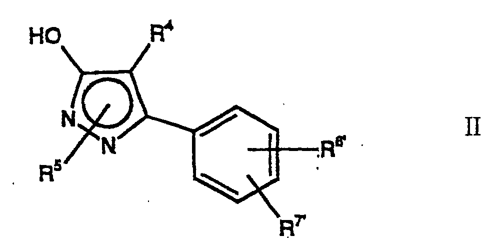 dans laquelle R⁴, R⁵, R^{6'} et R^{7'} ont le sens précité, les groupes hydroxy libres éventuels comportant cependant un groupe protecteur hydroxy, avec des composés de formule générale III : 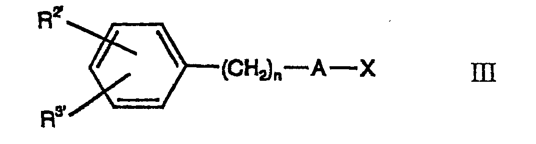 dans laquelle R^{2'}, R^{3'}, n et A ont le sens précité, les groupes hydroxy libres éventuels comportant cependant un groupe protecteur hydroxy et X représentant un groupe partant scindable, et l'on scinde ensuite à nouveau les groupes protecteurs hydroxy éventuellement présents ou bien,
b) pour préparer des composés de formule générale Ia, 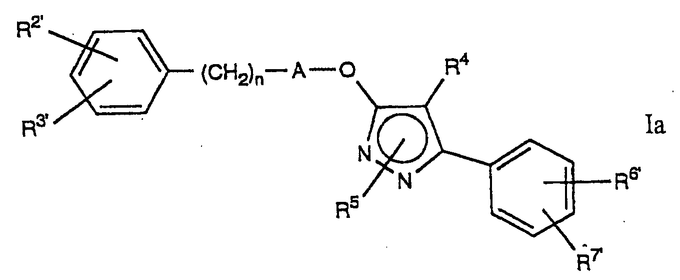 on fait réagir des composés de formule II avec de la triphénylphosphine en présence d'esters d'un acide azodicarboxylique de formule générale XVIII :
R¹⁰OOC―N=N―COOR¹¹ XVIII
dans laquelle R¹⁰ et R¹¹ représentent chacun un groupe alkyle inférieur, et l'on fait réagir le produit de cette réaction avec des composés de formule générale IV : 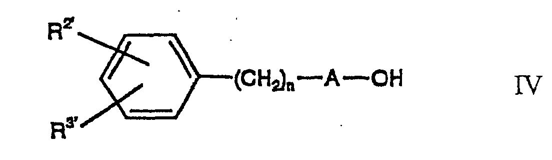 dans laquelle R^{2'}, R^{3'}, n et A ont le sens ci-dessus, tout groupe NH libre éventuellement présent dans le reste A comportant cependant un groupe amino protecteur et les groupes hydroxy libres éventuels comportant des groupes protecteurs hydroxy, et l'on scinde ensuite à nouveau les groupes amino protecteurs et/ou les groupes protecteurs hydroxy éventuellement présents, ou bien
c) pour préparer des composés de formule générale Ib 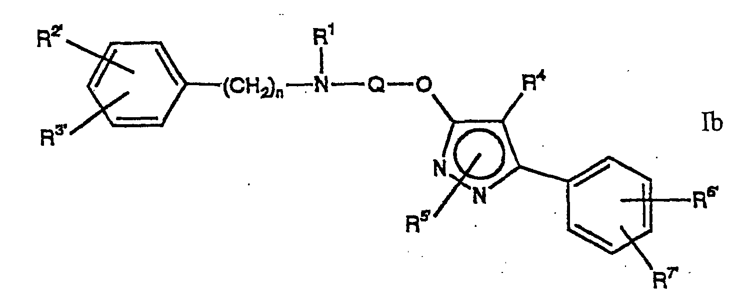 dans laquelle R¹, R^{2'}, R^{3'}, R⁴, R^{6'}, R^{7'}, Q et n ont le sens précité et R^{5'} a le sens indiqué pour R5 mais, si la longueur de chaîne m du reste Q vaut 3, R^{5'} représente un groupe alkyle inférieur ou un groupe phényl-alkyle inférieur placé sur un atome d'azote du noyau pyrazole voisin de l'atome d'oxygène, on fait réagir des composés de formule générale V : 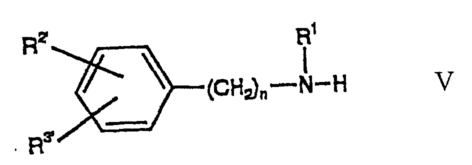 dans laquelle R¹, R^{2'}, R^{3'}, et n ont le sens précité, les groupes hydroxy libres éventuels comportant cependant un groupe protecteur hydroxy, avec des composés de formule générale VI : 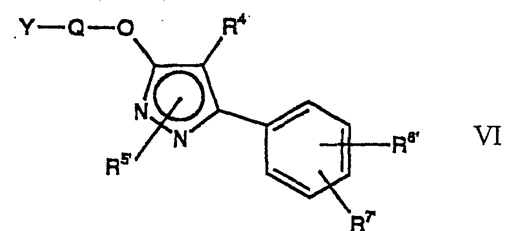 dans laquelle R⁴, R^{5'}, R^{6'}, R^{7'} et Q ont le sens précité, les groupes hydroxy libres éventuels comportant cependant un groupe protecteur hydroxy, et Y représentant un groupe partant scindable par aminolyse, et on scinde ensuite à nouveau les groupes protecteurs hydroxy éventuellement présents, ou bien
d) pour préparer des composés de formule Ia, on fait réagir des composés de formule générale VII : 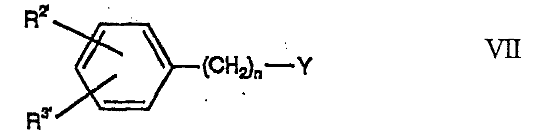 dans laquelle R^{2'}, R^{3'}, n et Y ont le sens précité, les groupes hydroxy libres éventuels comportant cependant un groupe protecteur hydroxy, avec des composés de formule générale VIII: 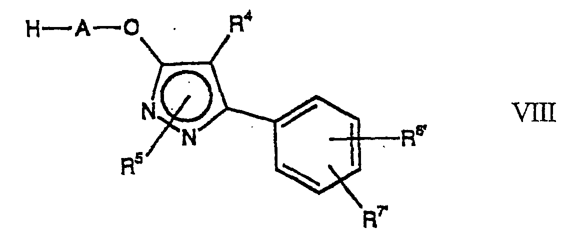 dans laquelle R⁴, R⁵, R^{6'}, R^{7'} et A ont le sens précité, les groupes hydroxy libres éventuellement présents comportant cependant un groupe protecteur hydroxy, et l'on scinde ensuite à nouveau les groupes protecteurs hydroxy éventuellement présents, ou
e) pour préparer des composés de formule générale Ic : 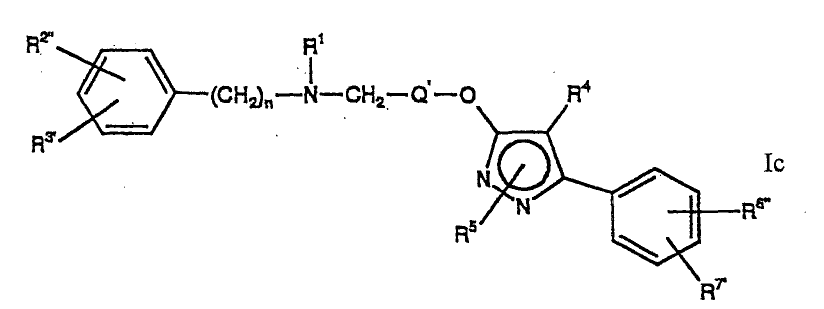 dans laquelle R¹, R^{3'}, R⁴, R⁵, R^{7'} et n ont le sens ci-dessus, R^{2''} et R^{6''} ont le sens donné pour R² et R⁶ à l'exception d'un groupe nitro et d'un groupe alcanoyloxy inférieur, Q' représente un groupe (CH₂)_{m'} où m' vaut 1 à 7 et peut être éventuellement substitué en position α par rapport à l'atome d'oxygène par 1 à 2 groupes alkyle inférieurs, on réduit des composés de formule générale IX : 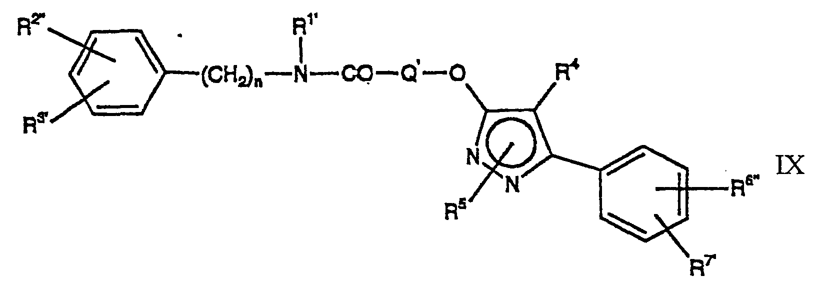 dans laquelle R^{2''}, R^{3'}, R⁴, R⁵, R^{6''}, R^{7'}, n et Q' ont le sens précité, les groupes hydroxy libres éventuellement présents comportant cependant un groupe protecteur hydroxy, et R^{1'} représente un groupe alkyle inférieur ou un groupe amino protecteur, puis l'on scinde ensuite à nouveau les groupes protecteurs amino et/ou hydroxy éventuellement présents, ou bien
f) pour préparer des composés de formule générale Id : 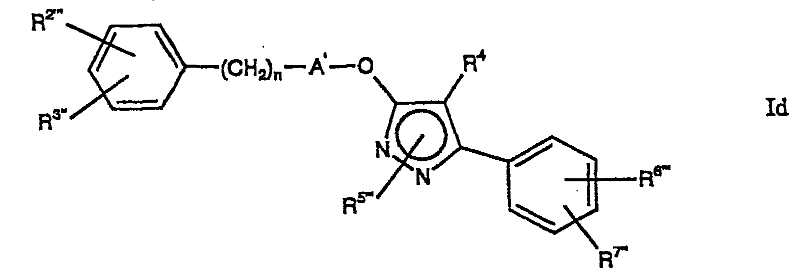 dans laquelle R⁴, n et A ont le sens précité, R^{5'''} a le sens indiqué pour R⁵, R^{5'''} ne représentant cependant pas un groupe benzyle, si le reste A contient un groupe NH, et R^{2'''}, R^{3''}, R^{6'''} et R^{7''} ont le sens indiqué pour R², R³, R⁶ et R⁷, sauf un groupe hydroxy, au moins l'un des substituants R^{2'''}, R^{3''}, R^{6'''} et R^{7''} représentant cependant un groupe alcanoyloxy inférieur, en effectuant dans les composés de formule générale X : 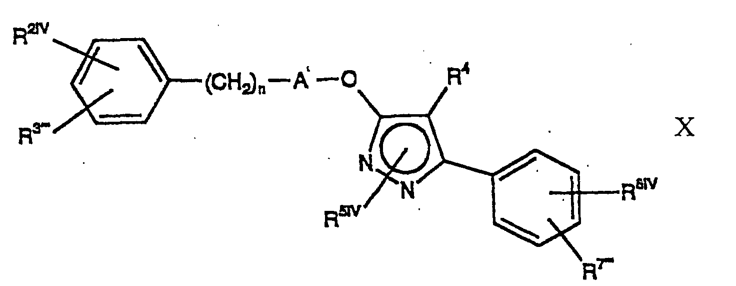 dans laquelle n et R⁴ ont le sens ci-dessus, R^{2IV}, R^{3'''}, R^{6IV} et R^{7'''} ont le sens indiqué pour R², R³, R⁶ et R⁷ sauf celui d'un groupe alcanoyloxy inférieur, au moins l'un des substituants R^{2IV}, R^{3'''}, R^{6IV} et R^{7'''} représentant cependant un groupe hydroxy, R^{5IV} représente un groupe alkyle inférieur ou un groupe phényl-alkyle inférieur et A' a le sens indiqué pour A, tout groupe NH éventuellement contenu dans le reste A' étant cependant protégé par un groupe benzyle protecteur, l'acylation des groupes hydroxy libres R^{2IV}, R^{3'''}, R^{6'''} et/ou R^{7'''}, et en scindant à nouveau éventuellement ensuite tout groupe benzyle protecteur éventuellement présent, et, éventuellement dans les composés obtenus de formule I,
dans laquelle R², R³, R⁶ et/ou R⁷ représentent un groupe méthoxy, en libérant de ces groupes un groupe hydroxy et/ou éventuellement dans les composés obtenus de formule I, dans laquelle R⁵ représente un groupe benzyle, en scindant ce groupe benzyle et en transformant éventuellement les composés de formule I libres en leurs sels d'addition d'acides, ou bien en transformant les sels d'addition d'acides en les composés de formule I libres.

11. Composés de formule générale IX : 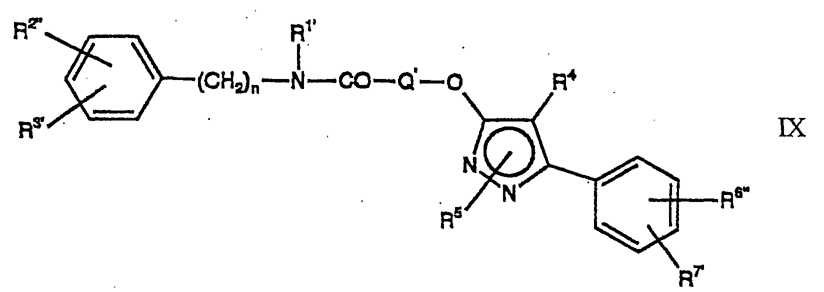 dans laquelle
R^{1'} représente un groupe alkyle inférieur ou un groupe amino protecteur,
R^{2''} représente un atome d'hydrogène, un halogène, un groupe alkyle inférieur, trifluorométhyle ou alcoxy inférieur, et
R^{3'} représente un atome d'hydrogène, un halogène, un groupe alkyle inférieur ou alcoxy inférieur, ou bien,
R^{2''} et R^{3'} sont fixés sur deux atomes de carbone voisins et forment ensemble un groupe alkylènedioxy ayant de 1 à 2 atomes de carbone,
n est un nombre entier valant 1 à 5,
Q' représente un groupe (CH₂)ₘ, dans lequel m' vaut 1 à 7 et qui est éventuellement substitué, en position α par rapport à l'atome d'oxygène, par 1 à 2 groupes alkyle inférieurs,
R⁴ représente un atome d'hydrogène ou un groupe alkyle inférieur,
R⁵ est fixé en position 1 ou 2 et représente un atome d'hydrogène, un groupe alkyle inférieur ou un groupe phényl-alkyle inférieur,
R^{6''} représente un atome d'hydrogène, un groupe alkyle inférieur, alcoxy inférieur, ou un halogène et,
R^{7'} représente un atome d'hydrogène, un groupe alkyle inférieur, alcoxy inférieur, ou un halogène, ou bien
R^{6''} et R^{7'} sont fixés sur deux atomes de carbone voisins et forment ensemble un groupe alkylènedioxy ayant 1 à 2 atomes de carbone.
